(19) Europäisches Patentamt
European Patent Office
Office européen des brevets

(11) EP 2 812 320 B1

(12) **EUROPEAN PATENT SPECIFICATION**

(45) Date of publication and mention of the grant of the patent:
**07.03.2018 Bulletin 2018/10**

(21) Application number: **13702741.3**

(22) Date of filing: **24.01.2013**

(51) Int Cl.:
***A61B 17/435*** *(2006.01)*

(86) International application number:
**PCT/EP2013/000215**

(87) International publication number:
**WO 2013/117299 (15.08.2013 Gazette 2013/33)**

# (54) DEUTERATED THIAZOLIDINONE ANALOGUES AS AGONISTS FOR FOLLICLE STIMULATING HORMONE RECEPTOR

DEUTERIERTE THIAZOLIDINON-ANALOGA ALS AGONISTEN FÜR DEN REZEPTOR DES FOLLIKEL-STIMULIERENDEN HORMONS

ANALOGUES DE THIAZOLIDINONE DEUTÉRÉS UTILISÉS COMME AGONISTES DU RÉCEPTEUR DE L'HORMONE FOLLICULO-STIMULANTE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.02.2012 US 201261596417 P**

(43) Date of publication of application:
**17.12.2014 Bulletin 2014/51**

(73) Proprietor: **Merck Patent GmbH**
**64293 Darmstadt (DE)**

(72) Inventors:
- **YU, Henry**
  **Wellesley, Massachusetts 02481 (US)**
- **DONNELLY, Marianne**
  **Reading, Massachusetts 01867 (US)**

(56) References cited:
**WO-A1-02/09706**
**WO-A1-2009/045476**
**WO-A1-2010/136438**

- **KUSHNER DJ ET AL: "Pharmacological uses and perspectives of heavy water and deuterated compounds", CANADIAN JOURNAL OF PHYSIOLOGY AND PHARMACOLOGY, OTTAWA, ONT, CA, vol. 77, no. 2, February 1999 (1999-02), pages 79-88, XP009086918,**
- **FOSTER A B: "Deuterium isotope effects in the metabolism of drugs and xenobiotics: implications for drug design", ADVANCES IN DRUG RESEARCH, ACADEMIC PRESS, LONDON, GB, vol. 14, 1985, pages 1-40, XP009086953, ISSN: 0065-2490**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 812 320 B1

## Description

Technical field

The present invention relates to deuterated thiazolidinone analogues as agonists for follicle stimulating hormone receptor and their use for treating fertility disorders.

Prior art

Gonadotropins serve important functions in a variety of bodily functions including metabolism, temperature regulation and the reproductive process. Gonadotropins act on specific gonadal cell types to initiate ovarian and testicular differentiation and steroidogenesis. The gonadotropin FSH (follicle stimulating hormone) is released from the anterior pituitary under the influence of gonadotropin-releasing hormone and estrogens, and from the placenta during pregnancy. FSH is a heterodimeric glycoprotein hormone that shares structural similarities with luteinizing hormone (LH) and thyroid stimulating hormone (TSH), both of which are also produced in the pituitary gland, and chorionic gonadotropin (CG), which is produced in the placenta. In the female, FSH plays a pivotal role in the stimulation of follicle development and maturation and in addition, it is the major hormone regulating secretion of estrogens, whereas LH induces ovulation. In the male, FSH is responsible for the integrity of the seminiferous tubules and acts on Sertoli cells to support gametogenesis.

The hormones are relatively large (28-38 kDa) and are composed of a common α-subunit non-covalently bound to a distinct β-subunit that confers receptor binding specificity. The cellular receptor for these hormones is expressed on testicular Sertoli cells and ovarian granulosa cells. The FSH receptor is known to be members of the G protein-coupled class of membrane-bound receptors, which when activated stimulate an increase in the activity of adenylyl cyclase. This results in an increase in the level of the intracellular second messenger adenosine 3', 5'-monophosphate (cAMP), which in turn causes increased steroid synthesis and secretion. Hydropathicity plots of the amino acid sequences of these receptors reveal three general domains: a hydrophilic amino-terminal region, considered to be the amino-terminal extracellular domain; seven hydrophobic segments of membrane-spanning length, considered to be the transmembrane domain; and a carboxy-terminal region that contains potential phosphorylation sites (serine, threonine, and tyrosine residues), considered to be the carboxy-terminal intracellular or cytoplasmic domain. The glycoprotein hormone receptor family is distinguished from other G protein-coupled receptors, such as the β-2-adrenergic, rhodopsin, and substance K receptors, by the large size of the hydrophilic amino-terminal domain, which is involved in hormone binding.

Annually in the U.S. there are 2.4 million couples experiencing infertility that are potential candidates for treatment. FSH, either extracted from urine or produced by recombinant DNA technology, is a parenterally-administered protein product used by specialists for ovulation induction and for controlled ovarial hyperstimulation. Whereas ovulation induction is directed at achieving a single follicle to ovulate, controlled ovarial hyperstimulation is directed at harvesting multiple oocytes for use in various in-vitro assisted reproductive technologies, e.g. in-vitro fertilization (IVF). FSH is also used clinically to treat male hypogonadism and male infertility, e.g. some types of failure of spermatogenesis.

Follicle stimulating hormone receptor (FSHR) is a highly specific target in the ovarian follicle growth process and is exclusively expressed in the ovary. However, the use of FSH is limited by its high cost, lack of oral dosing, and need of extensive monitoring by specialist physicians. Hence, identification of a non-peptidic small molecule substitute for FSH that could potentially be developed for oral administration is desirable. Low molecular weight FSH mimetics with agonistic properties are disclosed in the international applications WO 2002/09706 and WO 2010/136438 as well as the patent US 6,653,338. There is still a need for low molecular weight hormone mimetics that selectively activate FSHR.

Further prior art is as follows:

Pelletier JC et al. disclose the preparation of highly substituted gamma-lactam follicle stimulating hormone receptor agonists (Bioorg. Med. Chem. 2005, 13: 5986-5995).

Wrobel J et al. describe 5-alkylated thiazolidinones as follicle-stimulating hormone receptor agonists (Bioorg. Med. Chem. 2006, 14: 5729-5741).

WO 02/09705 is directed to thiazolidinones as agonists of follicle stimulating hormone activity.

WO 02/09706 relates to novel thiazolidinones as agonists of follicle stimulating hormone activity and discloses compound

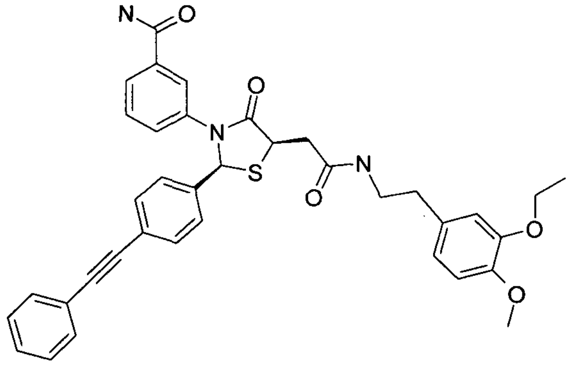

(hereinafter referred to as reference compound) in table 1 on page 20.

It has been known that the replacement of one or more hydrogen atoms in a drug molecule by deuterium is generally possible, as the sterical consequences or the influence on physicochemical properties are small (Foster, A.B.: Advances in Drug Research 14 (1985) 1-40).

It has also been known that most deuterated drugs are more resistant to metabolic changes, which potentially leads to increased duration of action and lower toxicity (Kushner et al., Canadian Journal of Physiology and Pharmacology, 1999, Vol. 77, No. 2 : pp. 79-88).

WO2009045476 discloses the use of deuterated DPP4 inhibitors for, i.a., the treatment of fertility disorders.

However, the general prior art, and also WO 02/09706 is silent about the substitution of hydrogen (H) atoms of the reference compound of WO 02/09706 by deuterium (D) as disclosed hereinbelow.

Description of the invention

The present invention has the object to provide deuterated thiazolidinone analogues as agonists for FSHR.

The object of the present invention has surprisingly been solved in one aspect by providing a deuterated thiazolidinone derivative according to formula (I)

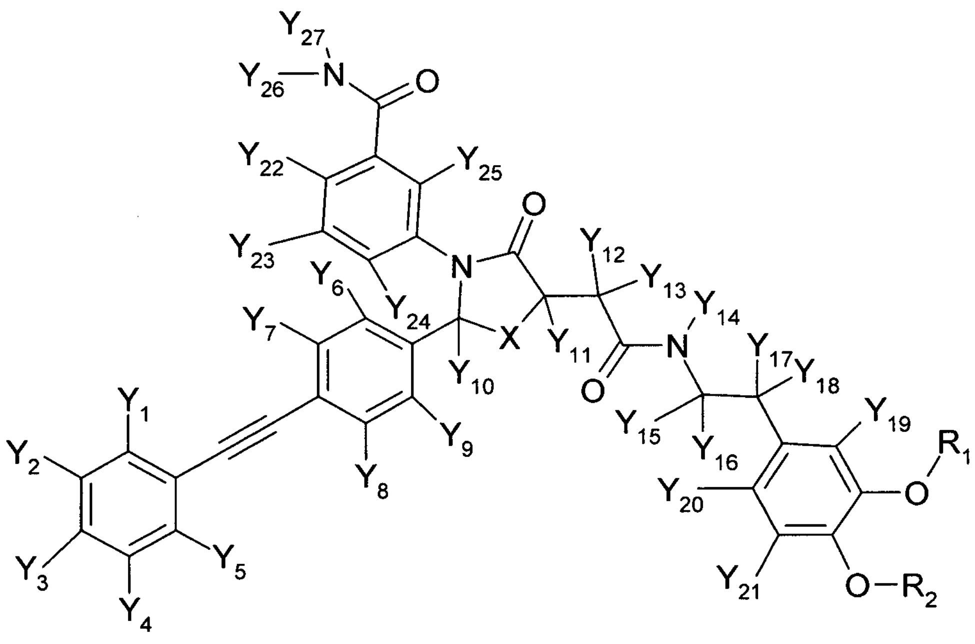

**(I)**

wherein:

each Y is independently from each other selected from the group consisting of: "hydrogen (H), deuterium (D)";

X is selected from the group consisting of: "S, sulfoxide";

R1, R2 are independently from each other selected from the group consisting of: "hydrogen (H), deuterium (D), methyl, $CH_2D$, $CHD_2$, $CD_3$, ethyl, $CHDCH_3$, $CHDCH_2D$, $CHDCHD_2$, $CHDCD_3$, $CD_2CH_3$, $CD_2CH_2D$, $CD_2CHD_2$, $CD_2CD_3$";

with the proviso that at least

(i) $Y_{15}$, $Y_{16}$, $Y_{17}$, $Y_{18}$ are deuterium (D), or
(ii) $Y_1$, $Y_2$, $Y_3$, $Y_4$, $Y_5$ are deuterium (D), or
(iii) one of R1, R2 comprises at least one deuterium (D);

optionally, at least one carbon atom is independently from each other replaced by $^{13}C$;

and the physiologically acceptable salts, solvates, tautomers and stereoisomers thereof, including mixtures thereof in all ratios.

In a preferred embodiment, a compound according to formula (I) supra and above embodiments is provided, wherein:

$R_1$ and/or $R_2$ are $CD_3$;

and the physiologically acceptable salts, solvates, tautomers and stereoisomers thereof, including mixtures thereof in all ratios.

In a preferred embodiment, a compound according to formula (I) supra and above embodiments is provided, wherein:

(a) $Y_{15}$, $Y_{16}$, $Y_{17}$, $Y_{18}$ are deuterium (D) and the other Y are hydrogen (H) and none of $R_1$, $R_2$ comprises one or more deuterium (D); or

(b) $Y_{15}$, $Y_{16}$, $Y_{17}$, $Y_{18}$ are deuterium (D) and the other Y are hydrogen (H) and one of $R_1$, $R_2$ is $CD_3$ and the other one of $R_1$, $R_2$ does not comprise one or more deuterium (D); or

(c) all Y are hydrogen (H) and one of $R_1$, $R_2$ is $CD_3$ and the other one of $R_1$, $R_2$ does not comprise one or more deuterium (D); or

(d) $Y_1$, $Y_2$, $Y_3$, $Y_4$, $Y_5$ are deuterium (D) and the other Y are hydrogen (H) and none of $R_1$, $R_2$ comprises one or more deuterium (D); or

(e) all Y are hydrogen (H) and both of $R_1$, $R_2$ are $CD_3$; or

(f) $Y_1$, $Y_2$, $Y_3$, $Y_4$, $Y_5$, $Y_{15}$, $Y_{16}$, $Y_{17}$, $Y_{18}$ are deuterium (D) and the other Y are hydrogen (H) and none of $R_1$, $R_2$ comprises one or more deuterium (D); or

(g) $Y_1$, $Y_2$, $Y_3$, Y4, $Y_5$, $Y_{15}$, $Y_{16}$, $Y_{17}$, $Y_{18}$ are deuterium (D) and the other Y are hydrogen (H) and one of $R_1$, $R_2$ is $CD_3$ and the other one of $R_1$, $R_2$ does not comprise one or more deuterium (D); or

(h) $Y_6$, $Y_7$, $Y_8$, $Y_9$, $Y_{15}$, $Y_{16}$, $Y_{17}$, $Y_{18}$ are deuterium (D) and the other Y are hydrogen (H) and one of $R_1$, $R_2$ is $CD_3$ and the other one of $R_1$, $R_2$ does not comprise one or more deuterium (D);

and the physiologically acceptable salts, solvates, tautomers and stereoisomers thereof, including mixtures thereof in all ratios.

In another aspect, the object of the present invention has surprisingly been solved by providing a compound selected from the group consisting of:

| Compound | Structure |
|---|---|
| **1** | 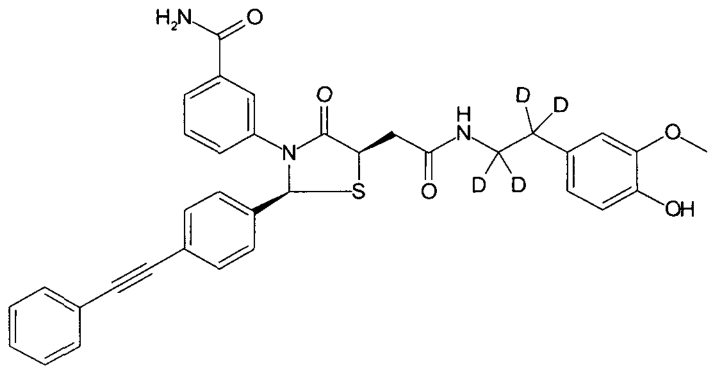 |
| **2** | 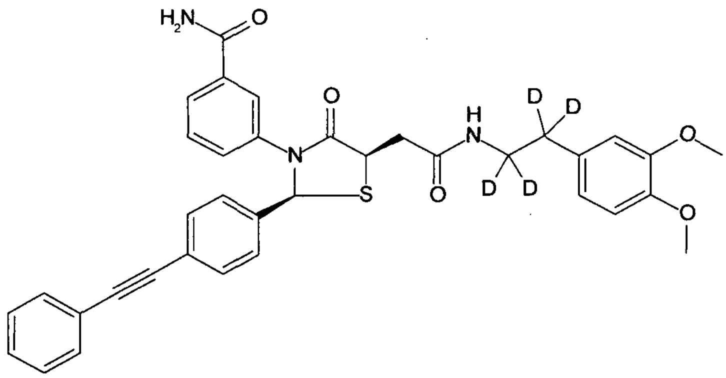 |
| **3** | 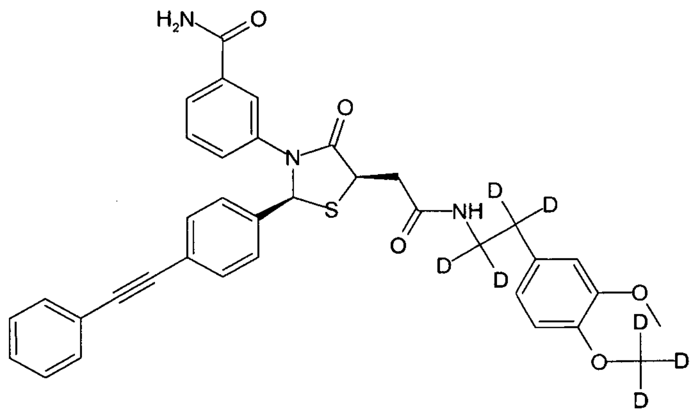 |
| **4** | 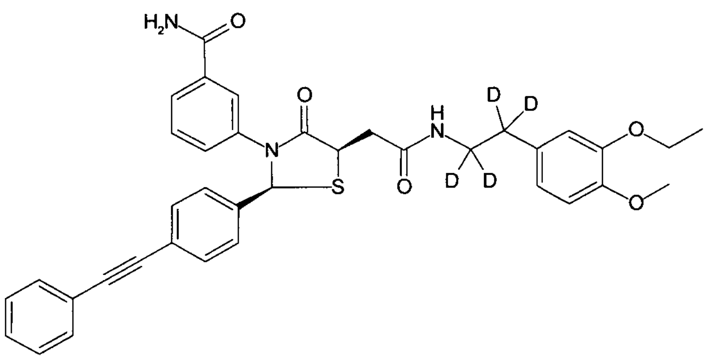 |

(continued)

| Compound | Structure |
|---|---|
| **5** | 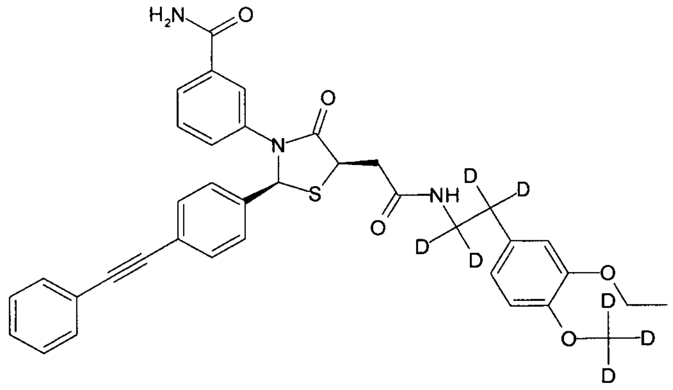 |
| **6** | 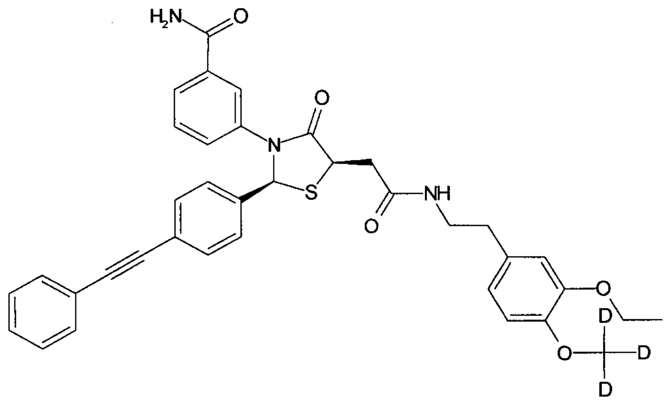 |
| **7** | 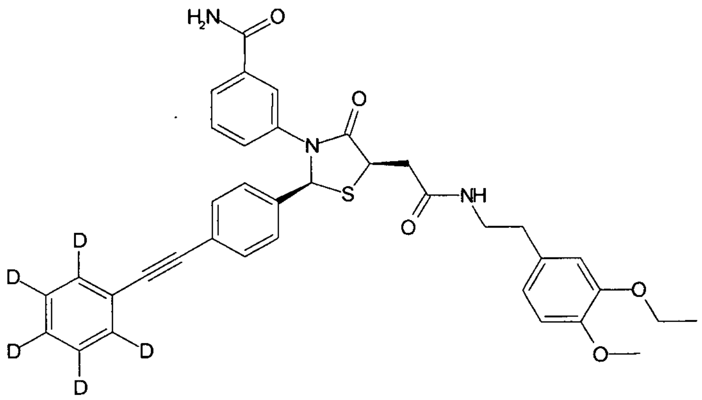 |
| **8** | 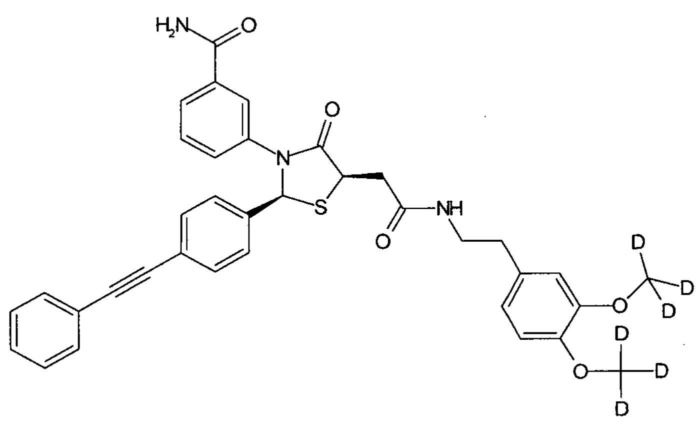 |

(continued)

| Compound | Structure |
|---|---|
| 9 | 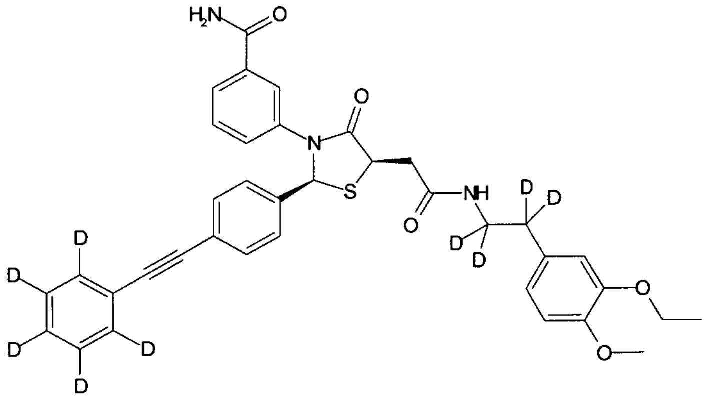 |
| 10 | 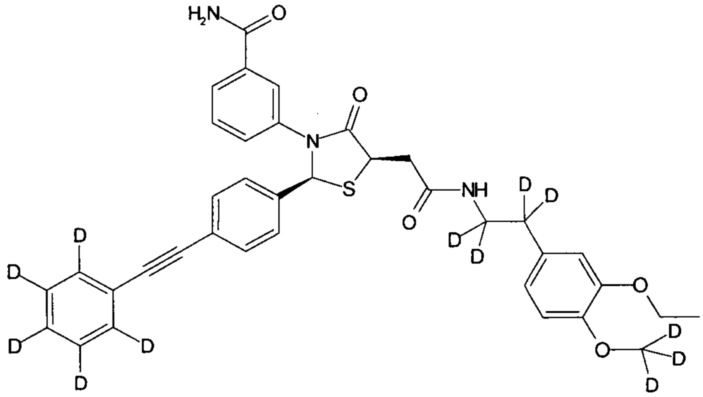 |
| 11 | 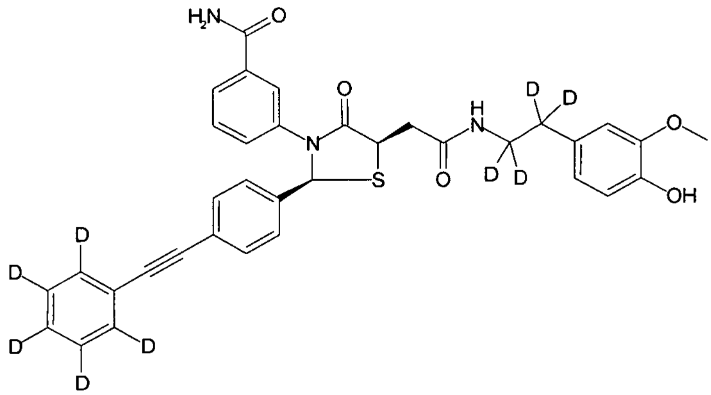 |
| 12 | 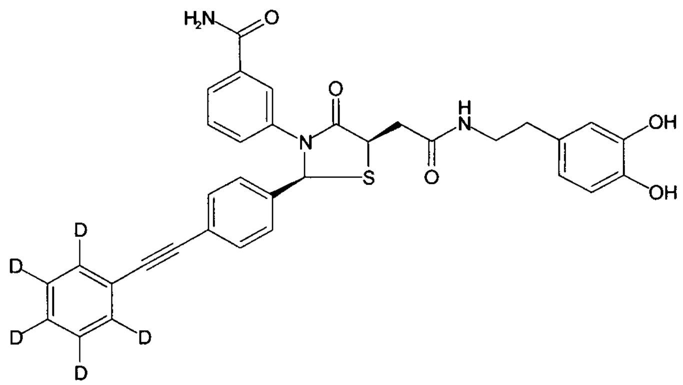 |

(continued)

| Compound | Structure |
|---|---|
| **13** | 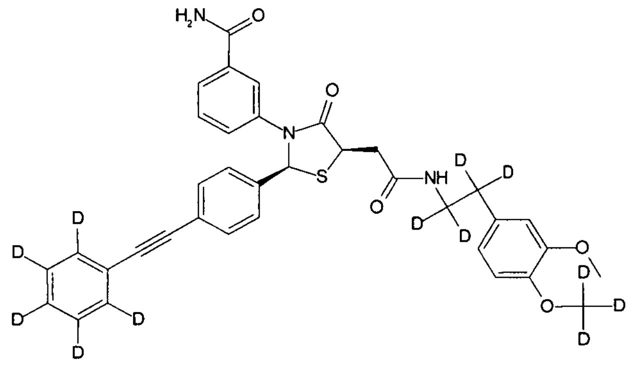 |
| **14** | 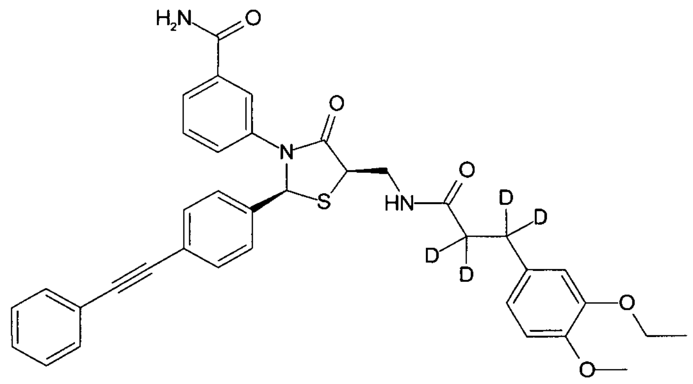 |
| **15** | 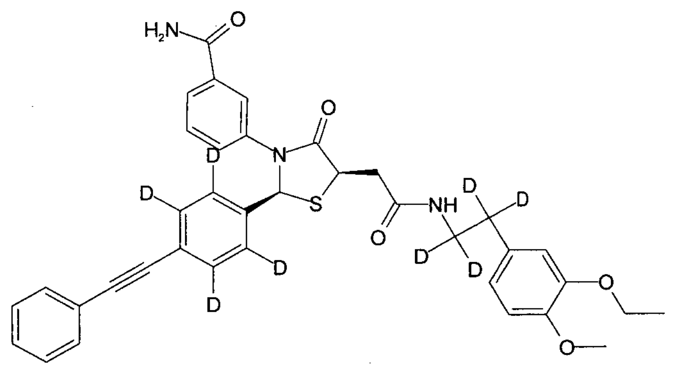 |
| **16** | 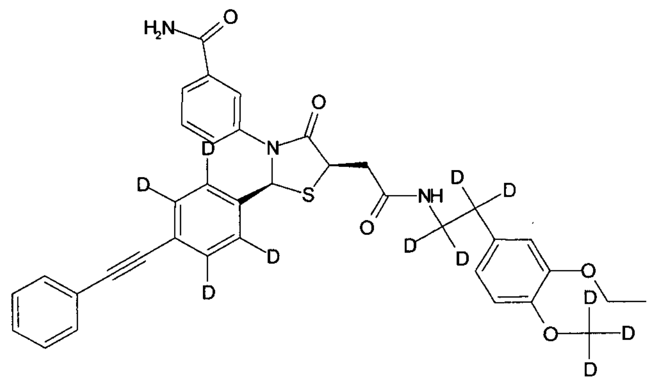 |

(continued)

| Compound | Structure |
|---|---|
| **17** | 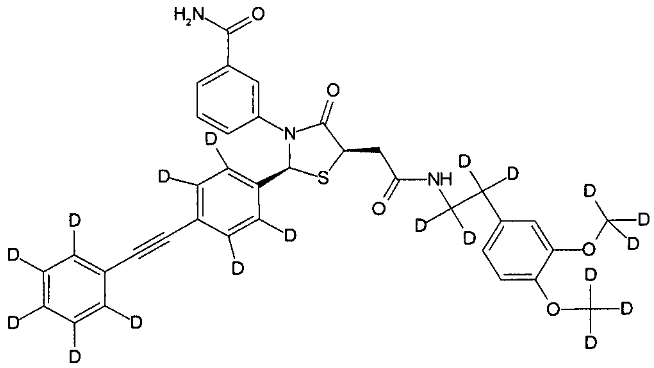 |
| **18** | 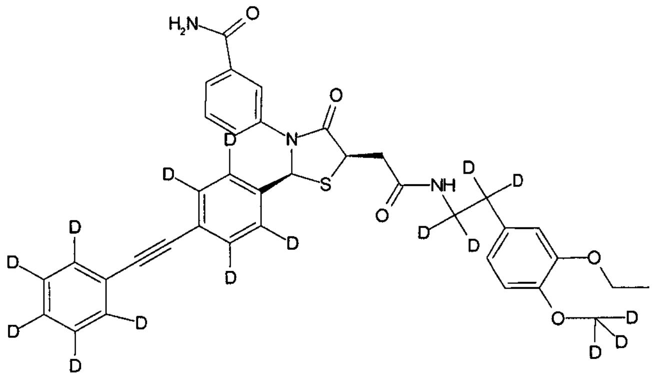 |
| **19** | 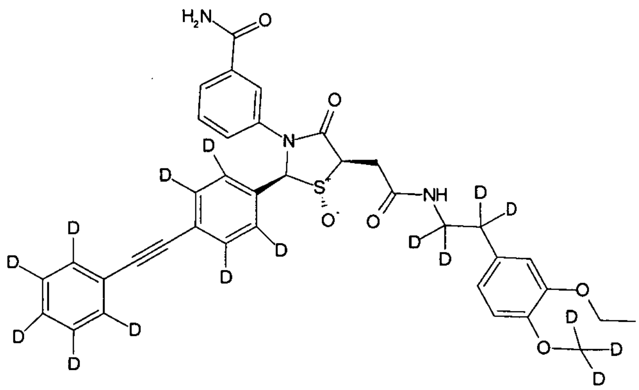 |
| **20** | 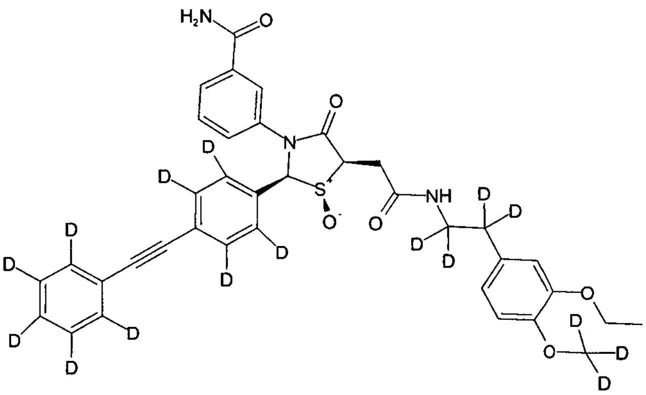 |

(continued)

| Compound | Structure |
|---|---|
| 21 | 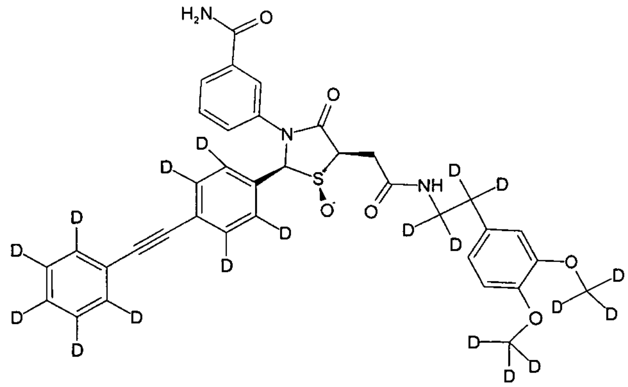 |
| 22 | 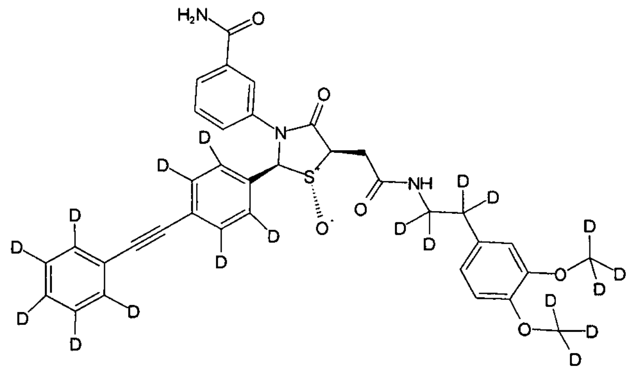 |

and the physiologically acceptable salts, solvates, tautomers and stereoisomers thereof, including mixtures thereof in all ratios.

For the avoidance of doubt, if chemical name and chemical structure of the above illustrated compounds do not correspond by mistake, the chemical structure is regarded to unambigously define the compound.

All the above generically or explicitly disclosed compounds, including preferred embodiments of the herein disclosed formula (I) and Compounds 1 to 22, are hereinafter referred to as compounds of the (present) invention.

The nomenclature as used herein for defining compounds, especially the compounds according to the invention, is in general based on the rules of the IUPAC organisation for chemical compounds and especially organic compounds.

The terms indicated for explanation of the above compounds of the invention always, unless indicated otherwise in the description or in the claims, have the following meanings:

The term "composition", as in pharmaceutical composition, for the purposes of this invention is intended to encompass a product comprising the active ingredient(s), and the inert ingredient(s) that make up the carrier, as well as any product which results, directly or indirectly, from combination, complexation or aggregation of any two or more of the ingredients, or from dissociation of one or more of the ingredients, or from other types of reactions or interactions of one or more of the ingredients. Accordingly, the pharmaceutical compositions of the present invention encompass any composition made by admixing a compound of the present invention and a pharmaceutically acceptable carrier.

The terms "administration of" and "administering a" compound should be understood to mean providing a compound of the invention to the individualist need.

As used herein, the term "effective amount" refers to any amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function.

All stereoisomers of the compounds of the invention are contemplated, either in a mixture or in pure or substantially pure form. The compounds of the invention can have asymmetric centers at any of the carbon atoms. Consequently, they can exist in the form of their racemates, in the form of the pure enantiomers and/or diastereomers or in the form of mixtures of these enantiomers and/or diastereomers. The mixtures may have any desired mixing ratio of the

stereoisomers.

Thus, for example, the compounds of the invention which have one or more centers of chirality and which occur as racemates or as diastereomer mixtures can be fractionated by methods known per se into their optical pure isomers, i.e. enantiomers or diastereomers. The separation of the compounds of the invention can take place by column separation on chiral or nonchiral phases or by recrystallization from an optionally optically active solvent or with use of an optically active acid or base or by derivatization with an optically active reagent such as, for example, an optically active alcohol, and subsequent elimination of the radical.

The compounds of the invention may be present in the form of their double bond isomers as "pure" E or Z isomers, or in the form of mixtures of these double bond isomers.

Where possible, the compounds of the invention may be in the form of the tautomers, such as keto-enol tautomers.

The compounds of the invention can, if they have a sufficiently basic group such as, for example, a secondary or tertiary amine, be converted with inorganic and organic acids into salts. The pharmaceutically acceptable salts of the compounds of the invention are preferably formed with hydrochloric acid, hydrobromic acid, iodic acid, sulfuric acid, phosphoric acid, methanesulfonic acid, p-toluenesulfonic acid, carbonic acid, formic acid, acetic acid, sulfoacetic acid, trifluoroacetic acid, oxalic acid, malonic acid, maleic acid, succinic acid, tartaric acid, racemic acid, malic acid, embonic acid, mandelic acid, fumaric acid, lactic acid, citric acid, taurocholic acid, glutaric acid, stearic acid, glutamic acid or aspartic acid. The salts which are formed are, inter alia, hydrochlorides, chlorides, hydrobromides, bromides, iodides, sulfates, phosphates, methanesulfonates, tosylates, carbonates, bicarbonates, formates, acetates, sulfoacetates, triflates, oxalates, malonates, maleates, succinates, tartrates, malates, embonates, mandelates, fumarates, lactates, citrates, glutarates, stearates, aspartates and glutamates. The stoichiometry of the salts formed from the compounds of the invention may moreover be an integral or non-integral multiple of one.

The compounds of the invention can, if they contain a sufficiently acidic group such as, for example, the carboxy, sulfonic acid, phosphoric acid or a phenolic group, be converted with inorganic and organic bases into their physiologically tolerated salts. Examples of suitable inorganic bases are ammonium, sodium hydroxide, potassium hydroxide, calcium hydroxide, and of organic bases are ethanolamine, diethanolamine, triethanolamine, ethylenediamine, t-butylamine, t-octylamine, dehydroabietylamine, cyclohexylamine, dibenzylethylene-diamine and lysine. The stoichiometry of the salts formed from the compounds of the invention can moreover be an integral or non-integral multiple of one.

It is likewise possible for the compounds of the invention to be in the form of their solvates and, in particular, hydrates which can be obtained for example by crystallization from a solvent or from aqueous solution. It is moreover possible for one, two, three or any number of solvate or water molecules to combine with the compounds of the invention to give solvates and hydrates.

By the term "solvate" is meant a hydrate, an alcoholate, or other solvate of crystallization.

It is known that chemical substances form solids which exist in different order states which are referred to as polymorphic forms or modifications. The various modifications of a polymorphic substance may differ greatly in their physical properties. The compounds of the invention can exist in various polymorphic forms and certain modifications may moreover be metastable. All these polymorphic forms of the compounds are to be regarded as belonging to the invention.

Following the incubation of compounds of the invention in human and rat hepatic microsomes intrinsic clearance (Clint) was reduced. As shown in Table 1, specifically compound 1 to 20 and 22 demonstrated a decreased Clint in rat hepatic microsome compared to reference compound. Compound 1 to 3, 4, 10, 19 and 22 demonstrated a decreased Clint in human hepatic microsome compared to reference compound. This is accompanied by a corresponding reduction in pharmacokinetic clearance and decrease in volume distribution. Such changes might reduce dosage and dosing frequency, which results in a better safety profile. Moreover, the further reduced pharmacokinetic interactions between the compounds of the invention and other drugs metabolized by the same metabolic enzymes (e.g. CYP) provide an increased safety as compared to reference compound.

The compounds of the invention preferably exhibit an advantageous biological activity, which is easily demonstrated in cell culture-based assays, for example assays as described herein or in prior art (cf. e.g. WO 2002/009706). In such assays, the compounds of the invention preferably exhibit and cause an agonistic effect. It is preferred that the compounds of the invention have an FSHR agonist activity, as expressed by an $EC_{50}$ standard, of less than 1000 nM, more preferably less than 500 nM. "$EC_{50}$" is the effective concentration of a compound at which 50 % of the maximal response of that obtained with FSH would be obtained.

As discussed herein, these signaling pathways are relevant for various diseases, preferably fertility disorders. Accordingly, the compounds of the invention are useful in the prophylaxis and/or treatment of diseases that are dependent on the said signaling pathways by interaction with one or more of the said signaling pathways. The present invention therefore relates to compounds of the invention for use as modulators, preferably agonists, more preferably positive allosteric modulators, of the signaling pathways described herein, preferably of the FSHR-mediated signaling pathway. The compounds of the invention are supposed to bind to the intracellular receptor domain without a competitive interaction

with FSH, but they act as an allosteric enhancer of FSH on its receptor. The non-competitive interaction refers to the nature of the agonist activity exhibited by the compounds of the invention, wherein the compounds activate FSHR without substantially reducing the magnitude of binding of FSH to FSHR.

Some crude products were subjected to standard chromatography using solvent mixtures containing methanol, ethanol, isopropanol, n-hexane, cyclohexane, dichloromethane, n-heptane or petrol ether, respectively.

For a further detailed description of the manufacturing processes, please refer also to the examples and the following general description of the preferred conditions.

A physiologically acceptable salt of a compound of the invention can also be obtained by isolating and/or treating the compound of the invention obtained by the described reaction with an acid or a base.

The compounds of the invention and also the starting materials for their preparation are, are prepared by methods as described in the examples or by methods known per se, as described in the literature (for example in standard works, such as Houben-Weyl, Methoden der Organischen Chemie [Methods of Organic Chemistry], Georg Thieme Verlag, Stuttgart; Organic Reactions, John Wiley & Sons, Inc., New York), to be precise under reaction conditions which are known and suitable for the said reactions. Use can also be made here of variants which are known per se, but are not mentioned here in greater detail.

The starting materials for the claimed process may, if desired, also be formed in situ by not isolating them from the reaction mixture, but instead immediately converting them further into the compounds of the invention. On the other hand, it is possible to carry out the reaction stepwise.

Preferably, the reaction of the compounds is carried out in the presence of a suitable solvent, which is preferably inert under the respective reaction conditions. Examples of suitable solvents are hydrocarbons, such as hexane, petroleum ether, benzene, toluene or xylene; chlorinated hydrocarbons, such as trichlorethylene, 1,2-dichloroethane, tetrachloromethane, chloroform or dichloromethane; alcohols, such as methanol, ethanol, isopropanol, n-propanol, n-butanol or tert-butanol; ethers, such as diethyl ether, diisopropyl ether, tetrahydrofuran (THF) or dioxane; glycol ethers, such as ethylene glycol monomethyl or monoethyl ether or ethylene glycol dimethyl ether (diglyme); ketones, such as acetone or butanone; amides, such as acetamide, dimethylacetamide, dimethylformamide (DMF) or N-methyl pyrrolidinone (NMP); nitriles, such as acetonitrile; sulfoxides, such as dimethyl sulfoxide (DMSO); nitro compounds, such as nitromethane or nitrobenzene; esters, such as ethyl acetate, or mixtures of the said solvents or mixtures with water. Polar solvents are in general preferred. Examples for suitable polar solvents are chlorinated hydrocarbons, alcohols, glycol ethers, nitriles, amides and sulfoxides or mixtures thereof. More preferred are amides, especially dimethylformamide (DMF).

As stated above, the reaction temperature is between about -100° C and 300° C, depending on the reaction step and the conditions used.

Reaction times are generally in the range between some minutes and several days, depending on the reactivity of the respective compounds and the respective reaction conditions. Suitable reaction times are readily determinable by methods known in the art, for example reaction monitoring. Based on the reaction temperatures given above, suitable reaction times generally lie in the range between 10 min and 48 hrs.

A base of a compound of the invention can be converted into the associated acid-addition salt using an acid, for example by reaction of equivalent amounts of the base and the acid in a preferably inert solvent, such as ethanol, followed by evaporation. Suitable acids for this reaction are, in particular, those which give physiologically acceptable salts. Thus, it is possible to use inorganic acids, for example sulfuric acid, sulfurous acid, dithionic acid, nitric acid, hydrohalic acids, such as hydrochloric acid or hydrobromic acid, phosphoric acids, such as, for example, orthophosphoric acid, sulfamic acid, furthermore organic acids, in particular aliphatic, alicyclic, araliphatic, aromatic or heterocyclic monobasic or polybasic carboxylic, sulfonic or sulfuric acids, for example formic acid, acetic acid, propionic acid, hexanoic acid, octanoic acid, decanoic acid, hexadecanoic acid, octadecanoic acid, pivalic acid, diethylacetic acid, malonic acid, succinic acid, pimelic acid, fumaric acid, maleic acid, lactic acid, tartaric acid, malic acid, citric acid, gluconic acid, ascorbic acid, nicotinic acid, isonicotinic acid, methane- or ethanesulfonic acid, ethanedisulfonic acid, 2-hydroxyethanesulfonic acid, benzenesulfonic acid, trimethoxybenzoic acid, adamantanecarboxylic acid, p-toluenesulfonic acid, glycolic acid, embonic acid, chlorophenoxyacetic acid, aspartic acid, glutamic acid, proline, glyoxylic acid, palmitic acid, parachlorophenoxyisobutyric acid, cyclohexanecarboxylic acid, glucose 1-phosphate, naphthalenemono- and -disulfonic acids or laurylsulfuric acid.

Salts with physiologically unacceptable acids, for example picrates, can be used to isolate and/or purify the compounds of the invention.

On the other hand, compounds of the invention can be converted into the corresponding metal salts, in particular alkali metal salts or alkaline earth metal salts, or into the corresponding ammonium salts, using bases (for example sodium hydroxide, potassium hydroxide, sodium carbonate or potassium carbonate). Suitable salts are furthermore substituted ammonium salts, for example the dimethyl-, diethyl- and diisopropylammonium salts, monoethanol-, diethanol- and diisopropanolammonium salts, cyclohexyl- and dicyclohexylammonium salts, dibenzylethylenediammonium salts, furthermore, for example, salts with arginine or lysine.

If desired, the free bases of the compounds of the invention can be liberated from their salts by treatment with

strong bases, such as sodium hydroxide, potassium hydroxide, sodium carbonate or potassium carbonate, so long as no further acidic groups are present in the molecule. In the cases where the compounds of the invention have free acid groups, salt formation can likewise be achieved by treatment with bases. Suitable bases are alkali metal hydroxides, alkaline earth metal hydroxides or organic bases in the form of primary, secondary or tertiary amines.

Every reaction step described herein can optionally be followed by one or more working up procedures and/or isolating procedures. Suitable such procedures are known in the art, for example from standard works, such as Houben-Weyl, Methoden der organischen Chemie [Methods of Organic Chemistry], Georg-Thieme-Verlag, Stuttgart). Examples for such procedures include evaporating a solvent, distilling, crystallization, fractionised crystallization, extraction procedures, washing procedures, digesting procedures, filtration procedures, chromatography, chromatography by HPLC and drying procedures, especially drying procedures in vacuo and/or elevated temperature.

The object of the present invention has surprisingly been solved in another aspect by providing a medicament comprising at least one compound of the invention.

The object of the present invention has surprisingly been solved in another aspect by providing a medicament comprising at least one compound of the invention for use in the prophylactic or therapeutic treatment and/or monitoring of fertility disorders.

Object of the present invention is also the compounds according to formula (I) and/or physiologically acceptable salts thereof for use in modulating a FSH receptor, particularly in the presence of FSH. The term "modulation" denotes any change in FSHR-mediated signal transduction, which is based on the action of the specific inventive compounds capable to interact with the FSHR target in such a manner that makes recognition, binding and activating possible. The compounds are characterized by such a high affinity to FSHR, which ensures a reliable binding and preferably a positive allosteric modulation of FSHR. More preferably, the substances are mono-specific in order to guarantee an exclusive and directed recognition with the single FSHR target. In the context of the present invention, the term "recognition" relates to any type of interaction between the specific compounds and the target, particularly covalent or non-covalent binding or association, such as a covalent bond, hydrophobic/ hydrophilic interactions, van der Waals forces, ion pairs, hydrogen bonds, ligand-receptor interactions, and the like. Such association may also encompass the presence of other molecules such as peptides, proteins or nucleotide sequences. The present receptor/ligand-interaction is characterized by high affinity, high selectivity and minimal or even lacking cross-reactivity to other target molecules to exclude unhealthy and harmful impacts to the treated subject.

Compounds of the invention may be used in combination with one or more other active substances (ingredients, drugs) in the treatment, prevention, suppression or amelioration of diseases or conditions for which compounds of the invention or the other substances have utility. Typically the combination of the drugs is safer or more effective than either drug alone, or the combination is safer or more effective than would it be expected based on the additive properties of the individual drugs. Such other drug(s) may be administered, by a route and in an amount commonly used contemporaneously or sequentially with a compound of the invention. When a compound of the invention is used contemporaneously with one or more other drugs, a combination product containing such other drug(s) and the compound of the invention is preferred. However, combination therapy also includes therapies in which the compound of the invention and one or more other drugs are administered on different overlapping schedules. It is contemplated that when used in combination with other active ingredients, the compound of the present invention or the other active ingredient or both may be used effectively in lower doses than when each is used alone. Accordingly, the pharmaceutical compositions of the present invention include those that contain one or more other active ingredients, in addition to a compound of the invention.

The present compounds are suitable for combination with known fertility-inducing agents. Preferably, the other active (pharmaceutical) ingredient is selected from the group of FSH, α-FSH (Gonal F), β-FSH, LH, hMG and 2-(4-(2-chloro-1,2-diphenylethenyl)-phenoxy)-N,N-diethyl-ethanamine citrate (Chlomifene citrate). Further ovulation adjuncts are known to those of skill in the art (cf. e.g. WO 2002/09706) and are useful with the compounds of the present invention.

In another aspect of the invention, a medicament according to above aspects and embodiments is provided, wherein in such medicament comprises at least one additional pharmacologically active substance (drug, ingredient).

In a preferred embodiment the at least one pharmacologically active substance is a substance as described herein.

In another aspect of the invention, a medicament according to above aspects and embodiments is provided, wherein the medicament is applied before and/or during and/or after treatment with at least one additional pharmacologically active substance.

In a preferred embodiment the at least one pharmacologically active substance is a substance as described herein.

In another aspect of the invention, a pharmaceutical composition comprising a therapeutically effective amount of at least one compound of the invention is provided.

In a preferred embodiment, the pharmaceutical composition contains at least one additional compound selected from the group consisting of physiologically acceptable excipients, auxiliaries, adjuvants, diluents, carriers and/or additional pharmaceutically active substance other than the compounds of the invention.

In another aspect of the invention, a pharmaceutical composition is disclosed which comprises at least one compound of the invention, at least one pharmacologically active substance other than the compounds of the invention as described herein; and a pharmaceutically acceptable carrier.

A further embodiment of the present invention is a process for the manufacture of said pharmaceutical compositions, characterized in that one or more compounds according to the invention and one or more compounds selected from the group consisting of solid, liquid or semiliquid excipients, auxiliaries, adjuvants, diluents, carriers and pharmaceutically active agents other than the compounds according to the invention, are converted in a suitable dosage form.

In another aspect of the invention, a kit is provided comprising a therapeutically effective amount of at least one compound of the invention and/or at least one pharmaceutical composition as described herein and a therapeutically effective amount of at least one further pharmacologically active substance other than the compounds of the invention.

In another aspect of the invention, a compound of the invention , or at least one pharmaceutical composition as described supra, is provided for use in modulating a FSH receptor in a positive allosteric manner, wherein a cell capable of expressing the FSH receptor is contacted in the presence of FSH with the said compound of the invention or the said at least one pharmaceutical composition.

In another aspect of the invention, a at least one compound of the invention, or at least one pharmaceutical composition as described supra, is provided for use in treating fertility disorders, wherein a therapeutically effective amount of the said at least one compound of the invention or the said at least one pharmaceutical composition as described supra is administered to a mammal in need of such treatment.

The pharmaceutical compositions of the present invention may be administered by any means that achieve their intended purpose. For example, administration may be by oral, parenteral, topical, enteral, intravenous, intramuscular, inhalant, nasal, intraarticular, intraspinal, transtracheal, transocular, subcutaneous, intraperitoneal, transdermal, or buccal routes. Alternatively, or concurrently, administration may be by the oral route. The dosage administered will be dependent upon the age, health, and weight of the recipient, kind of concurrent treatment, if any, frequency of treatment, and the nature of the effect desired. Parenteral administration is preferred. Oral administration is especially preferred.

Suitable dosage forms include capsules, tablets, pellets, dragees, semi-solids, powders, granules, suppositories, ointments, creams, lotions, inhalants, injections, cataplasms, gels, tapes, eye drops, solution, syrups, aerosols, suspension, emulsion, which can be produced according to methods known in the art, for example as described below:

tablets: mixing of active ingredient/s and auxiliaries, compression of said mixture into tablets (direct compression), optionally granulation of part of mixture before compression.

capsules: mixing of active ingredient/s and auxiliaries to obtain a flowable powder, optionally granulating powder, filling powders/granulate into opened capsules, capping of capsules.

semi-solids (ointments, gels, creams): dissolving/dispersing active ingredient/s in an aqueous or fatty carrier; subsequent mixing of aqueous/fatty phase with complementary fatty/ aqueous phase, homogenization (creams only).

suppositories (rectal and vaginal): dissolving/dispersing active ingredient/s in carrier material liquified by heat (rectal: carrier material normally a wax; vaginal: carrier normally a heated solution of a gelling agent), casting said mixture into suppository forms, annealing and withdrawal suppositories from the forms.

aerosols: dispersing/dissolving active agent/s in a propellant, bottling said mixture into an atomizer.

In general, non-chemical routes for the production of pharmaceutical compositions and/or pharmaceutical preparations comprise processing steps on suitable mechanical means known in the art that transfer one or more compounds of the invenion into a dosage form suitable for administration to a patient in need of such a treatment. Usually, the transfer of one or more compounds of the invention into such a dosage form comprises the addition of one or more compounds, selected from the group consisting of carriers, excipients, auxiliaries and pharmaceutical active ingredients other than the compounds of the invention. Suitable processing steps include combining, milling, mixing, granulating, dissolving, dispersing, homogenizing, casting and/or compressing the respective active and non-active ingredients. Mechanical means for performing said processing steps are known in the art, for example from Ullmann's Encyclopedia of Industrial Chemistry, 5th Edition. In this respect, active ingredients are preferably at least one compound of the invention and one or more additional compounds other than the compounds of the invention, which show valuable pharmaceutical properties, preferably those pharmaceutical active agents other than the compounds of the invention, which are disclosed herein.

Particularly suitable for oral use are tablets, pills, coated tablets, capsules, powders, granules, syrups, juices or drops, suitable for rectal use are suppositories, suitable for parenteral use are solutions, preferably oil-based or aqueous solutions, furthermore suspensions, emulsions or implants, and suitable for topical use are ointments, creams or powders. The compounds of the invention may also be lyophilised and the resultant lyophilisates used, for example, for the preparation of injection preparations. The preparations indicated may be sterilised and/or comprise assistants,

such as lubricants, preservatives, stabilisers and/or wetting agents, emulsifiers, salts for modifying the osmotic pressure, buffer substances, dyes, flavours and/or a plurality of further active ingredients, for example one or more vitamins.

Suitable excipients are organic or inorganic substances, which are suitable for enteral (for example oral), parenteral or topical administration and do not react with the compounds of the invention, for example water, vegetable oils, benzyl alcohols, alkylene glycols, polyethylene glycols, glycerol triacetate, gelatine, carbohydrates, such as lactose, sucrose, mannitol, sorbitol or starch (maize starch, wheat starch, rice starch, potato starch), cellulose preparations and/or calcium phosphates, for example tricalcium phosphate or calcium hydrogen phosphate, magnesium stearate, talc, gelatine, tragacanth, methyl cellulose, hydroxypropylmethylcellulose, sodium carboxymethylcellulose, polyvinyl pyrrolidone and/or vaseline.

If desired, disintegrating agents may be added such as the above-mentioned starches and also carboxymethylstarch, cross-linked polyvinyl pyrrolidone, agar, or alginic acid or a salt thereof, such as sodium alginate. Auxiliaries include flow-regulating agents and lubricants, for example, silica, talc, stearic acid or salts thereof, such as magnesium stearate or calcium stearate, and/or polyethylene glycol. Dragee cores are provided with suitable coatings, which, if desired, are resistant to gastric juices. For this purpose, concentrated saccharide solutions may be used, which may optionally contain gum arabic, talc, polyvinyl pyrrolidone, polyethylene glycol and/or titanium dioxide, lacquer solutions and suitable organic solvents or solvent mixtures. In order to produce coatings resistant to gastric juices or to provide a dosage form affording the advantage of prolonged action, the tablet, dragee or pill can comprise an inner dosage and an outer dosage component me latter being in the form of an envelope over the former. The two components can be separated by an enteric layer, which serves to resist disintegration in the stomach and permits the inner component to pass intact into the duodenum or to be delayed in release. A variety of materials can be used for such enteric layers or coatings, such materials including a number of polymeric acids and mixtures of polymeric acids with such materials as shellac, acetyl alcohol, solutions of suitable cellulose preparations such as acetyl-cellulose phthalate, cellulose acetate or hydroxypropylmethyl-cellulose phthalate, are used. Dye stuffs or pigments may be added to the tablets or dragee coatings, for example, for identification or in order to characterize combinations of active compound doses.

Suitable carrier substances are organic or inorganic substances which are suitable for enteral (e.g. oral) or parenteral administration or topical application and do not react with the novel compounds, for example water, vegetable oils, benzyl alcohols, polyethylene glycols, gelatin, carbohydrates such as lactose or starch, magnesium stearate, talc and petroleum jelly. In particular, tablets, coated tablets, capsules, syrups, suspensions, drops or suppositories are used for enteral administration, solutions, preferably oily or aqueous solutions, furthermore suspensions, emulsions or implants, are used for parenteral administration, and ointments, creams or powders are used for topical application. The compounds of the invention can also be lyophilized and the lyophilizates obtained can be used, for example, for the production of injection preparations.

The preparations indicated can be sterilized and/or can contain excipients such as lubricants, preservatives, stabilizers and/or wetting agents, emulsifiers, salts for affecting the osmotic pressure, buffer substances, colorants, flavourings and/or aromatizers. They can, if desired, also contain one or more further active compounds, e.g. one or more vitamins.

Other pharmaceutical preparations, which can be used orally include push-fit capsules made of gelatine, as well as soft, sealed capsules made of gelatine and a plasticizer such as glycerol or sorbitol. The push-fit capsules can contain the active compounds in the form of granules, which may be mixed with fillers such as lactose, binders such as starches, and/or lubricants such as talc or magnesium stearate and, optionally, stabilizers. In soft capsules, the active compounds are preferably dissolved or suspended in suitable liquids, such as fatty oils, or liquid paraffin. In addition, stabilizers may be added.

The liquid forms in which the novel compositions of the present invention may be incorporated for administration orally include aqueous solutions, suitably flavoured syrups, aqueous or oil suspensions, and flavoured emulsions with edible oils such as cottonseed oil, sesame oil, coconut oil or peanut oil, as well as elixirs and similar pharmaceutical vehicles. Suitable dispersing or suspending agents for aqueous suspensions include synthetic and natural gums such as tragacanth, acacia, alginate, dextran, sodium carboxymethylcellulose, methylcellulose, polyvinylpyrrolidone or gelatine.

Suitable formulations for parenteral administration include aqueous solutions of the active compounds in water-soluble form, for example, water-soluble salts and alkaline solutions. In addition, suspensions of the active compounds as appropriate oily injection suspensions may be administered. Suitable lipophilic solvents or vehicles include fatty oils, for example, sesame oil, or synthetic fatty acid esters, for example, ethyl oleate or triglycerides or polyethylene glycol-400 (the compounds are soluble in PEG-400).

Aqueous injection suspensions may contain substances, which increase the viscosity of the suspension, including, for example, sodium carboxymethyl cellulose, sorbitol, and/or dextran, optionally, the suspension may also contain stabilizers.

For administration as an inhalation spray, it is possible to use sprays in which the active ingredient is either dissolved or suspended in a propellant gas or propellant gas mixture (for example $CO_2$ or chlorofluorocarbons). The

active ingredient is advantageously used here in micronized form, in which case one or more additional physiologically acceptable solvents may be present, for example ethanol. Inhalation solutions can be administered with the aid of conventional inhalers.

Possible pharmaceutical preparations, which can be used rectally include, for example, suppositories, which consist of a combination of one or more of the active compounds with a suppository base. Suitable suppository bases are, for example, natural or synthetic triglycerides, or paraffin hydrocarbons. In addition, it is also possible to use gelatine rectal capsules, which consist of a combination of the active compounds with a base. Possible base materials include, for example, liquid triglycerides, polyethylene glycols, or paraffin hydrocarbons.

For use in medicine, the compounds of the present invention will be in the form of pharmaceutically acceptable salts. Other salts may, however, be useful in the preparation of the compounds of the invention or of their pharmaceutically acceptable salts. Suitable pharmaceutically acceptable salts of the compounds of this invention include acid addition salts which may, for example be formed by mixing a solution of the compound according to the invention with a solution of a pharmaceutically acceptable acid such as hydrochloric acid, sulphuric acid, methanesulphonic acid, fumaric acid, maleic acid, succinic acid, acetic acid, benzoic acid, oxalic acid, citric acid, tartaric acid, carbonic acid or phosphoric acid. Furthermore, where the compounds of the invention carry an acidic moiety, suitable pharmaceutically acceptable salts thereof may include alkali metal salts, e.g. sodium or potassium salts; alkaline earth metal salts, e.g. calcium or magnesium salts; and salts formed with suitable organic bases, e.g. quaternary ammonium salts.

The pharmaceutical preparations can be employed as medicaments in human and veterinary medicine. As used herein, the term "effective amount" means that amount of a drug or pharmaceutical agent that will elicit the biological or medical response of a tissue, system, animal or human that is being sought, for instance, by a researcher or clinician. Furthermore, the term "therapeutically effective amount" means any amount which, as compared to a corresponding subject who has not received such amount, results in improved treatment, healing, prevention, or amelioration of a disease, disorder, or side effect, or a decrease in the rate of advancement of a disease or disorder. The term also includes within its scope amounts effective to enhance normal physiological function. Said therapeutic effective amount of one or more of the compounds of the invention is known to the skilled artisan or can be easily determined by standard methods known in the art.

The compounds of the invention and the additional active substances are generally administered analogously to commercial preparations. Usually, suitable doses that are therapeutically effective lie in the range between 0.0005 mg and 1000 mg, preferably between 0.005 mg and 500 mg and especially between 0.5 mg and 100 mg per dose unit. The daily dose is preferably between about 0.001 mg/kg and 10 mg/kg of body weight.

Those of skill will readily appreciate that dose levels can vary as a function of the specific compound, the severity of the symptoms and the susceptibility of the subject to side effects. Some of the specific compounds are more potent than others. Preferred dosages for a given compound are readily determinable by those of skill in the art by a variety of means. A preferred means is to measure the physiological potency of a given compound.

For the purpose of the present invention, all mammalian species are regarded as being comprised. In a preferred embodiment, such mammals are selected from the group consisting of "primate, human, rodent, equine, bovine, canine, feline, domestic animals, cattle, livestock, pets, cow, sheep, pig, goat, horse, pony, donkey, hinny, mule, hare, rabbit, cat, dog, guinea pig, hamster, rat, mouse". More preferably, such mammals are humans. Animal models are of interest for experimental investigations, providing a model for treatment of human diseases.

The specific dose for the individual patient depends, however, on the multitude of factors, for example on the efficacy of the specific compounds employed, on the age, body weight, general state of health, the sex, the kind of diet, on the time and route of administration, on the excretion rate, the kind of administration and the dosage form to be administered, the pharmaceutical combination and severity of the particular disorder to which the therapy relates. The specific therapeutic effective dose for the individual patient can readily be determined by routine experimentation, for example by the doctor or physician, which advises or attends the therapeutic treatment.

In the case of many disorders, the susceptibility of a particular cell to treatment with the subject compounds may be determined by in vitro testing. Typically a culture of the cell is combined with a subject compound at varying concentrations for a period of time sufficient to allow the active agents to show a relevant reaction, usually between about one hour and one week. For in vitro testing, cultured cells from a biopsy sample may be used.

Even without further details, it is assumed that a person skilled in the art will be able to utilise the above description in the broadest scope. The preferred embodiments should therefore merely be regarded as descriptive disclosure.

Above and below, all temperatures are indicated in °C. In the following examples, "conventional work-up" means that, if necessary, the solvent is removed, water is added if necessary, the pH is adjusted, if necessary, to between 2 and 10, depending on the constitution of the end product, the mixture is extracted with ethyl acetate or dichloromethane, the phases are separated, the organic phase is washed with saturated $NaHCO_3$ solution, if desired with water and saturated NaCl solution, is dried over sodium sulfate, filtered and evaporated, and the product is purified by chromatography on silica gel, by preparative HPLC and/or by crystallisation. The purified compounds are, if desired, freeze-dried.

The invention is explained in more detail by means of the following examples.

Examples

**Standard description of analytical equipment**

$^1$H NMR was recorded on a Joel 400 MHz spectrometer, using residual signal of deuterated solvent as internal reference. Chemical shifts (δ) are reported in ppm relative to the residual solvent signal (δ = 2.49 ppm for 1H NMR in DMSO-$d_6$). $^1$H NMR data are reported as follows: chemical shift (multiplicity, coupling constants, and number of hydrogens). Multiplicity is abbreviated as follows: s (singlet), d (doublet), t (triplet), q (quartet), m (multiplet), br (broad).

LCMS method:

Column: Xbridge C8, 4.6x50mm 5um
Mobile Phase A: Water +0.1%TFA
Mobile Phase B: ACN +0.1%TFA
Gradient: 5-95%B in 3.5 minutes
Flow: 0.8 mL/min
Wavelength 254nm
Mass Scan: 100-900 Da

Example 1: 3-((2S,5R)-4-oxo-5-(2-oxo-2-((1,1,2,2-$d_4$-2-(4-hydroxy-3-methoxyphenyl)ethyl)amino)ethyl)-2-(4-(phenylethynyl)phenyl)thiazolidin-3-yl)benzamide

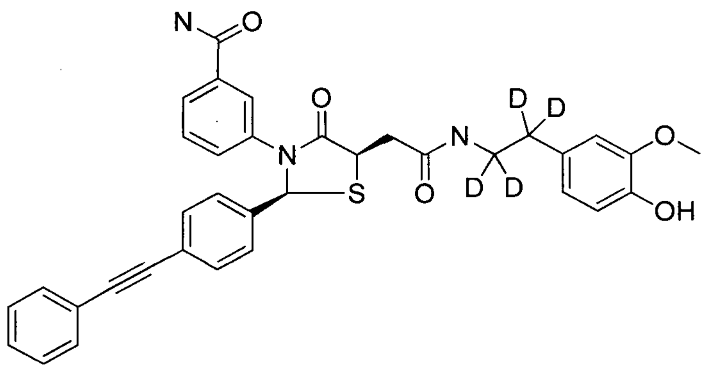

Step 1:

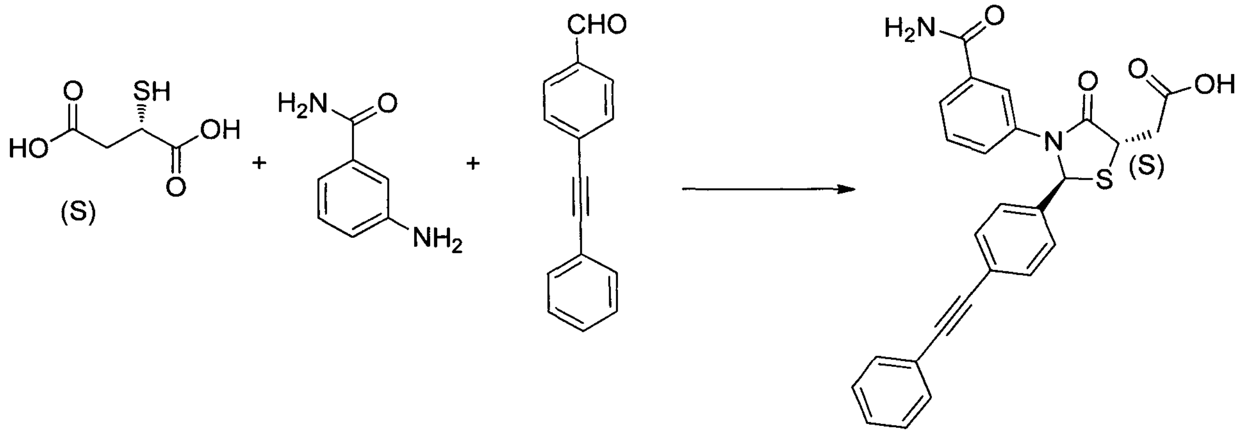

Similar procedures described in WO 02/09705 & WO 02/09706 To a solution of 4-(phenylethynyl)benzaldehyde (2.8 g, 13.6 mmol, 1 eq) in MeCN (240 ml) was added (S)-2-mercaptosuccinic acid (6.12 g, 40.8 mmol, 3 eq) and 3-aminobenzamide (1.85 g, 13.6 mmol, 1 eq). The reaction was stirred at 83°C for 3 days. The reaction was cooled and filtered to give solid. The solid was adjusted PH= 8~9 with saturated $Na_2CO_3$ and extracted with ethyl acetate (50 ml* 3). The water phase was adjusted PH=3~4 with 4N HCl and filtered to give solid. The solid was washed with EtOH (10 ml) and MeCN (10 ml) to afford title compound as a light yellow solid (3 g, 51.7%).

Step 2:

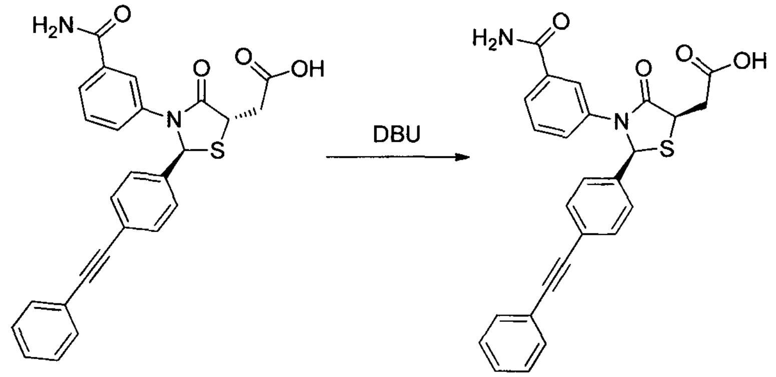


Similar procedures described in WO 02/09705 & WO 02/09706 To a solution of 2-((2S,5S)-3-(3-carbamoylphenyl)-4-oxo-2-(4-(phenylethynyl)phenyl)thiazolidin-5-yl)acetic acid (2.5 g, 5.58 mmol, 1 eq) in THF (127.5 ml) was added DBU (8.5 g, 55.8 mmol, 10eq) and MeOH (12.75 ml). The reaction was stirred at 70°C overnight. The reaction was cooled and evaporated. The residue was taken in ethyl acetate and 1N HCl and filtered. The filtrate was washed with 1N HCl, brine, dried over $Na_2SO_4$, filtrated and evaporated to give a solid (1.7 g). The solid was re-crystallized from MeCN to obtain 2-((2S,5R)-3-(3-carbamoylphenyl)-4-oxo-2-(4-(phenylethynyl)phenyl)thiazolidin-5-yl)acetic acid (0.6 g, 24 %).

Step 3: To [(2S,5R)-3-(3-Carbamoyl-phenyl)-4-oxo-2-(4-phenylethynyl-phenyl)-thiazolidin-5-yl]-acetic acid (50.00 mg; 0.11 mmol; 1.00 eq.) in dichloromethane(2.00 ml; 31.20 mmol; 284.87 eq.) was added N,N-Diisopropylethylamine (0.02 ml; 0.13 mmol; 1.20 eq.), 2-(4-hydroxy-3-methoxyphenyl)ethyl-1,1,2,2- $d_4$-amine HCl (25.02mg, 0.12mmol, 1.1 eq.) and 2,4,6-tripropyl-1,3,5,2,4,6-trioxatriphosphinane 2,4,6-trioxide (0.05 ml; 0.16 mmol; 1.50 eq.). The reaction was stirred at RT for 5h. The desired product was isolated by flash chromatography (10g, silica gel, 0 to 20% MeOH/DCM over 7CV) to afford product as white solid (35.2mg, 53%).

$^1$H NMR (500 MHz, $cd_3od$) δ 7.81 (s, 1H), 7.67 (d, *J* = 7.5, 1H), 7.51 - 7.25 (m, 11H), 6.77 (s, 1H), 6.73 - 6.57 (m, 2H), 6.42 (s, 1H), 4.50 (dd, *J* = 8.4, 3.9, 1H), 3.77 (s, 3H), 3.10 (dd, *J* = 15.2, 4.0, 1H), 2.89 (dd, *J* = 15.2, 8.5, 1H). m/z: 609 ; 610 $[M+H]^+$

Example 2: 3-((2S,5R)-4-oxo-5-(2-oxo-2-((1,1,2,2-$d_4$-2-(3,4-dimethoxyphenyl)ethyl)amino)ethyl)-2-(4-(phenylethynyl)phenyl)thiazolidin-3-yl)benzamide

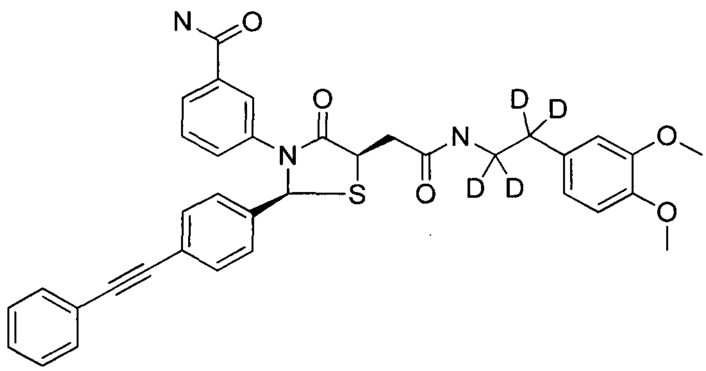

To 3-((2S,5R)-4-oxo-5-(2-oxo-2-((1,1,2,2-$d_4$-2-(4-hydroxy-3-methoxyphenyl)ethyl)amino)ethyl)-2-(4-(phenylethynyl)phenyl)thiazolidin-3-yl)benzamide (example 1) (25.00 mg; 0.04 mmol; 1.00 eq.) in acetone (1.00 ml; 13.62 mmol; 332.16 eq.) was added potassium carbonate (0.04 g; 0.27 mmol; 6.50 eq.), and dimethyl sulfate (0.18 μl; 0.008 mmol; 0.04 eq.). The reaction was stirred at RT overnight. The desired product was isolated by flash chromatography (10g, KPNH, 0 to 20% MeOH/DCM) to afford product as white solid (8.9mg, 35%).
$^1$H NMR (500 MHz, cd$_3$od) δ 7.83 - 7.78 (m, 1H), 7.69 - 7.64 (m, 1H), 7.50 - 7.30 (m, 11H), 6.81 (dd, *J* = 5.0, 3.1, 2H), 6.74 (dd, *J* = 8.2, 2.0, 1H), 6.42 (s, 1H), 4.53 - 4.47 (m, 1H), 3.76 (t, *J* = 4.6, 6H), 3.10 (dd, *J* = 15.3, 4.1, 1H), 2.89 (dd, *J* = 15.3, 8.5, 1H).
m/z: 624 ; 625 $[M+H]^+$

Example 3: 3-((2S,5R)-4-oxo-5-(2-oxo-2-((1,1,2,2-$d_4$-2-(3-methoxy-4-(methoxy-$d_3$)phenyl)ethyl)amino)ethyl)-2-(4-(phenylethynyl)phenyl)thiazolidin-3-yl)benzamide

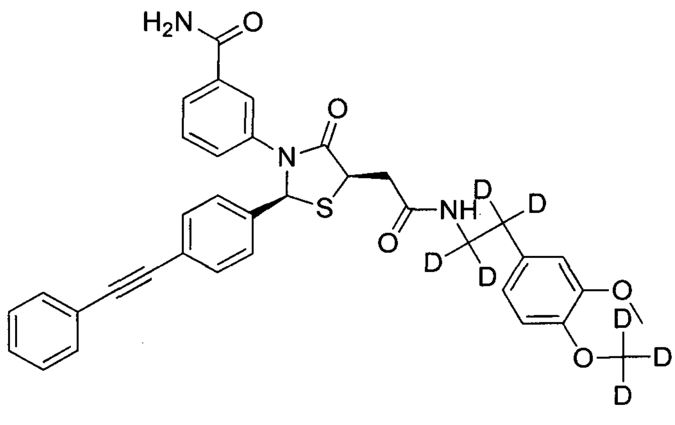

In a similar manner to example 2, product was obtained from 3-((2S,5R)-4-oxo-5-(2-oxo-2-((1,1,2,2-$d_4$-2-(4-hydroxy-3-methoxyphenyl)ethyl)amino)ethyl)-2-(4-(phenylethynyl)phenyl)thiazolidin-3-yl)benzamide (35.00 mg; 0.06 mmol; 1.00 eq.) and dimethyl sulfate-$d_6$ (2.92 μl; 0.03 mmol; 0.50 eq.). The reaction was stirred at RT overnight. The desired product was isolated by flash chromatography (10g, KPNH, 0 to 20% MeOH/DCM) to afford product as white solid (22.3mg, 63%).
$^1$H NMR (500 MHz, cd$_3$od) δ 7.83 - 7.79 (m, 1H), 7.70 - 7.65 (m, 1H), 7.48 - 7.30 (m, 11H), 6.83 - 6.77 (m, 2H), 6.74 (dd, *J* = 8.2, 2.0, 1H), 6.42 (s, 1H), 4.53 - 4.47 (m, 1H), 3.75 (s, 3H), 3.10 (dd, *J* = 15.2, 4.1, 1H), 2.89 (dd, *J* = 15.3, 8.5, 1H).
m/z: 626 ; 627 $[M+H]^+$

Example 4: 3-((2S,5R)-4-oxo-5-(2-oxo-2-((1,1,2,2-$d_4$-2-(3-ethoxy-4-methoxyphenyl)ethyl)amino)ethyl)-2-(4-(phenylethynyl)phenyl)thiazolidin-3-yl)benzamide

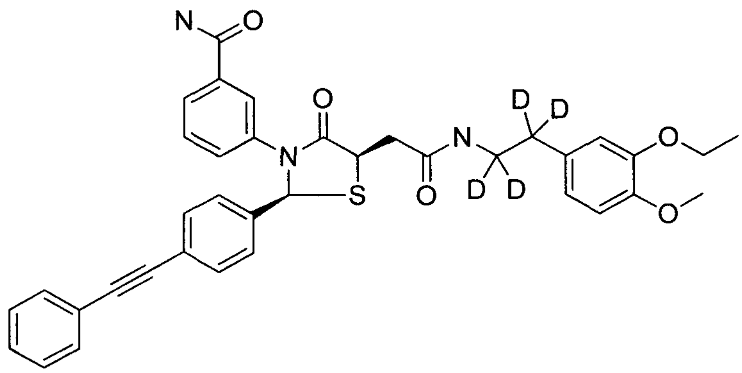

In a similar manner to example 1, product was obtained from [(2S,5R)-3-(3-Carbamoyl-phenyl)-4-oxo-2-(4-phenylethynyl-phenyl)-thiazolidin-5-yl]-acetic acid (50.00 mg; 0.11 mmol; 1.00 eq.) and 2-(3-ethoxy-4-methoxyphenyl)ethyl-1,1,2,2-$d_4$-amine HCl (28.40 mg; 0.12 mmol; 1.10 eq.). Isolated 44.2mg (63%) of the title compound as white solid.
$^1$H NMR (500 MHz, $cd_3od$) $\delta$ 7.81 (s, 1H), 7.67 (d, $J$ = 7.6, 1H), 7.50 - 7.29 (m, 11H), 6.87 - 6.69 (m, 3H), 6.42 (s, 1H), 4.50 (dd, $J$ = 8.4, 3.9, 1H), 3.99 (dt, $J$ = 12.3, 6.2, 2H), 3.80 - 3.71 (m, 3H), 3.09 (dd, $J$ = 15.2, 3.8, 1H), 2.90 (dd, $J$ = 15.3, 8.4, 1H), 1.38 - 1.25 (m, 3H).
m/z: 637 ; 638 $[M+H]^+$

Example 5: 3-((2S,5R)-4-oxo-5-(2-oxo-2-((1,1,2,2-$d_4$-2-(3-ethoxy-4-(methoxy-$d_3$)phenyl)ethyl)amino)ethyl)-2-(4-(phenylethynyl)phenyl)thiazolidin-3-yl)benzamide

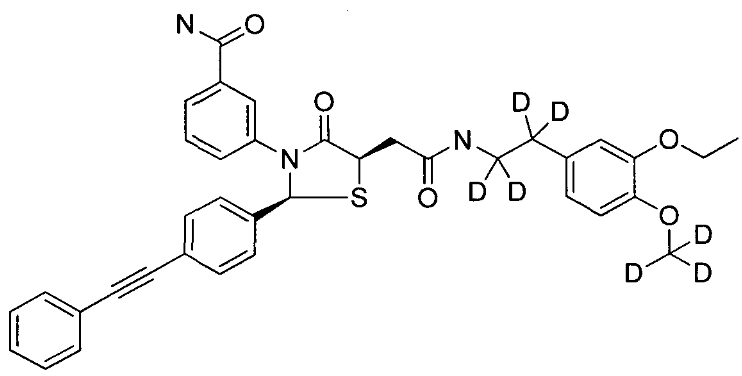

In a similar manner to example 1, product was obtained from [(2S,5R)-3-(3-Carbamoyl-phenyl)-4-oxo-2-(4-phenylethynyl-phenyl)-thiazolidin-5-yl]-acetic acid (50.00 mg; 0.11 mmol; 1.00 eq.) and 2-(3-ethoxy-4-methoxy-$d_3$-phenyl)ethyl-1,1,2,2-$d_4$-amine HCl ((28.77 mg; 0.12 mmol; 1.10 eq.). 54.2 mg (77%) of the title compound was obtained as white solid.
$^1$H NMR (500 MHz, $cd_3od$) $\delta$ 7.83 - 7.79 (m, 1H), 7.69 - 7.65 (m, 1H), 7.50 - 7.26 (m, 11H), 6.80 (dd, $J$ = 8.4, 5.0, 2H), 6.74 (dd, $J$ = 8.1, 2.0, 1H), 6.41 (s, 1H), 4.52 - 4.46 (m, 1H), 4.02 - 3.94 (m, 2H), 3.09 (dd, $J$ = 15.3, 4.0, 1H), 2.90 (dd, $J$ = 15.3, 8.4, 1H), 1.33 (t, $J$ = 7.0, 3H).
m/z: 640 ; 641 $[M+H]^+$

Example 6: 3-((2S,5R)-4-oxo-5-(2-oxo-2-((2-(3-ethoxy-4-(methoxy-$d_3$)phenyl)ethyl)amino)ethyl)-2-(4-(phenylethynyl)phenyl)thiazolidin-3-yl)benzamide

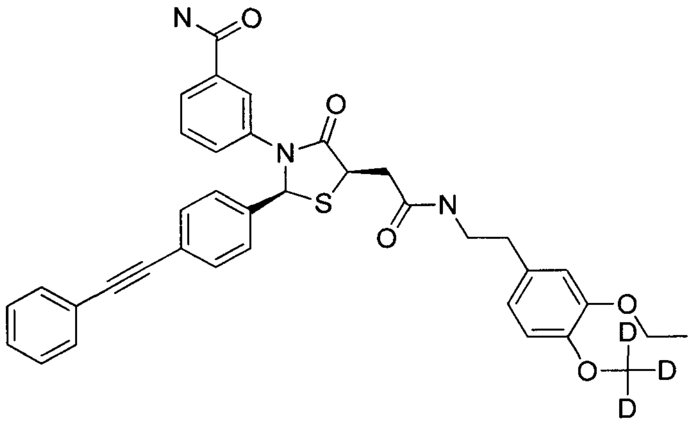

In a similar manner to example 3, product was obtain from 3-[(2S,5R)-5-{[2-(3-Ethoxy-4-hydroxy-phenyl)-ethylcarbamoyl]-methyl}-4-oxo-2-(4-phenylethynyl-phenyl)-thiazolidin-3-yl]-benzamide (50.00 mg; 0.08 mmol; 1.00 eq.) and dimethyl sulfate-$d_6$ (4.10 μl; 0.04 mmol; 0.50 eq.). 28.9mg (56%) of the title compound was obtained as white solid.
$^1$H NMR (500 MHz, $cd_3od$) δ 7.84 - 7.79 (m, 1H), 7.69 - 7.64 (m, 1H), 7.49 - 7.29 (m, 11H), 6.79 (dd, *J* = 7.5, 5.0, 2H), 6.74 (dd, *J* = 8.1, 2.0, 1H), 6.41 (s, 1H), 4.51 - 4.47 (m, 1H), 4.01 - 3.93 (m, 2H), 3.46 - 3.39 (m, 2H), 3.09 (dd, *J* = 15.3, 4.0, 1H), 2.90 (dd, *J* = 15.3, 8.4, 1H), 2.76 - 2.69 (m, 2H), 1.35 - 1.30 (m, 3H).
m/z: 636 ; 637 $[M+H]^+$

Example 7: 3-((2S,5R)-4-oxo-5-(2-oxo-2-((2-(3,4-dimethoxypheny-$d_6$l)ethyl)amino)ethyl)-2-(4-(phenylethynyl)phenyl)thiazolidin-3-yl)benzamide

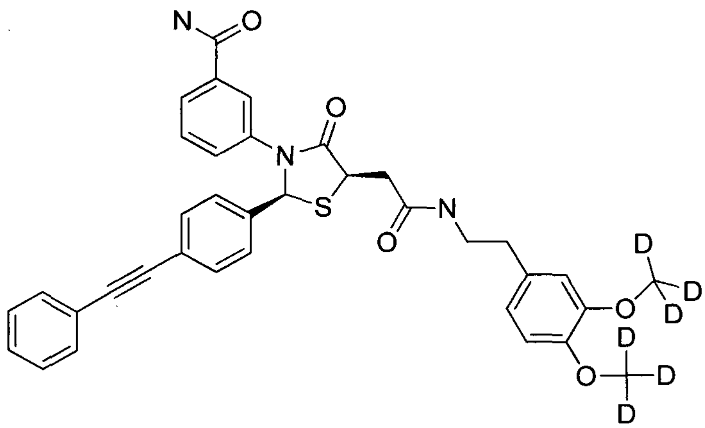

In a similar manner to example 3, product was obtained from 3-[(2S,5R)-5-{[2-(3,4-Dihydroxy-phenyl)-ethylcarbamoyl]-methyl}-4-oxo-2-(4-phenylethynyl-phenyl)-thiazolidin-3-yl]-benzamide (10.00 mg; 0.02 mmol; 1.00 eq.), and dimethyl sulfate-$d_6$ (3.44 μl; 0.03 mmol; 2.00 eq.). 4.3 mg (41%) of the title compound was obtained as white solid.
$^1$H NMR (400 MHz, MeOD) δ 7.83 (s, 1H), 7.70 (d, *J* = *7.5,* 1H), 7.53 - 7.32 (m, 11H), 6.82 (d, *J* = 4.4, 2H), 6.77 (d, *J* = 8.2, 1H), 6.44 (s, 1H), 4.52 (dd, *J* = 8.4, 4.0, 1H), 3.46 (t, *J* = 7.1, 2H), 3.13 (dd, *J* = 15.3, 4.1, 1H), 2.92 (dd, *J* = 15.3, 8.5, 1H), 2.77 (t, *J* = 7.1, 2H).
m/z: 625 ; 626 $[M+H]^+$

Example 8: 3-((2S,5R)-5-(2-((3-ethoxy-4-methoxyphenethyl)amino)-2-oxoethyl)-4-oxo-2-(4-((phenyl-$d_5$)ethynyl)phenyl)thiazolidin-3-yl)benzamide

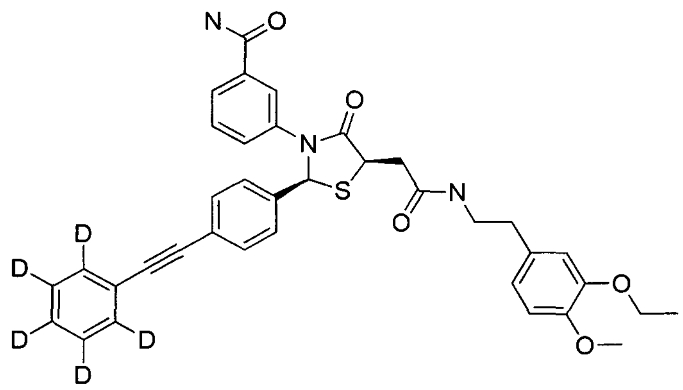

Step 1: To 4-bromobenzaldehyde (750.00 mg; 4.05 mmol; 1.00 eq.), was added zinc chloride (55.25 mg; 0.41 mmol; 0.10 eq.), bis(tri-tert-butylphosphine)palladium(0) (41.43 mg; 0.08 mmol; 0.02 eq.), THF (1.00 ml; 12.34 mmol; 3.04 eq.), diisopropylamine (0.13 ml; 0.92 mmol; 0.23 eq.). Then phenyl-$d_5$-acetylene (477.85 mg; 4.46 mmol; 1.10 eq.) was added. The reaction was stirred at RT overnight. The desired product was isolated by flash chromatography (0 to 20% EtOAC/Hex, over 10 CV). 206 mg (24%) of the title product was obtained as light yellow solid.

Step 2: In a similar manner to example 1 step 1& 2, 2-((2S,5R)-3-(3-carbamoylphenyl)-4-oxo-2-(4-(phenyl-$d_5$-ethynyl)phenyl)thiazolidin-5-yl)acetic acid was obtained from 4-(phenyl-$d_5$-ethynyl)benzaldehyde (206mg, 0.98mmol), (S)-2-Mercapto-succinic acid (439.23 mg; 2.93 mmol; 3.00 eq.) and 3-aminobenzamide (132.76 mg; 0.98 mmol; 1.00 eq.). Final product was isolated by prep HPLC. (131mg, 10% yield over 2 steps).
$^1$H NMR (400 MHz, MeOD) δ 7.83 (s, 1H), 7.70 (d, *J* = 7.3, 1H), 7.43 (dt, *J* = 14.3, 7.8, 6H), 6.90 - 6.70 (m, 3H), 6.44 (s, 1H), 4.52 (dd, *J* = 8.2, 3.8, 1H), 4.02 (dd, *J* = 14.0, 6.9, 2H), 3.78 (s, 3H), 3.46 (t, *J* = 7.1, 2H), 3.12 (dd, *J* = 15.5, 4.4, 1H), 2.92 (dd, *J* = 15.5, 8.3, 1H), 2.76 (t, *J* = 7.1, 2H), 1.35 (dd, *J* = 16.1, 9.1, 3H).
m/z: 638 ; 639 $[M+H]^+$

Example 9: 3-((2S,5R)-4-oxo-5-(2-oxo-2-((1,1,2,2-$d_4$-2-(3-ethoxy-4-methoxyphenyl)ethyl)amino)ethyl)-2-(4-((phenyl-$d_5$)ethynyl)phenyl)thiazolidin-3-yl)benzamide

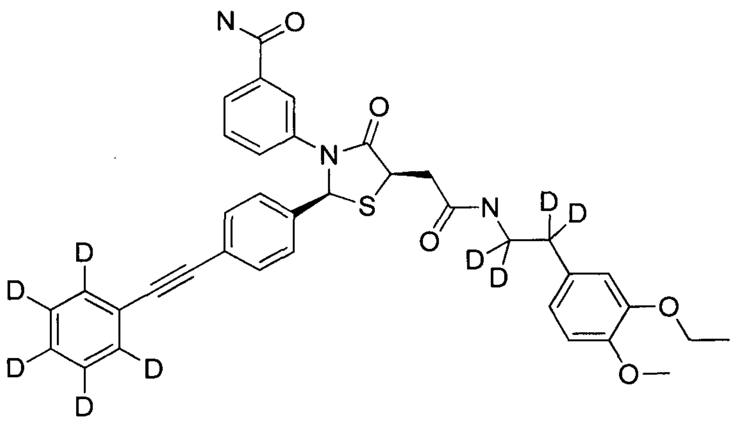

In a similar manner to example 1, product was obtained from 3-[(2S,5R)-5-{[2-(3,4-Dihydroxy-phenyl)-ethylcarbamoyl]-methyl}-4-oxo-2-(4-phenyl-$d_5$-ethynyl-phenyl)-thiazolidin-3-yl]-benzamide (25.00 mg; 0.05 mmol; 1.00 eq.) and 2-(3-ethoxy-4-methoxy-phenyl)ethyl-1,1,2,2-$d_4$-amine HCl (11.87mg, 0.06mmol, 1.1 eq.). The title product (24.5 mg, 70%) was obtained as white solid.
$^1$H NMR (400 MHz, $CDCl_3$) δ 7.72 (s, 1H), 7.59 (d, *J* = 7.3, 1H), 7.38 (ddd, *J* = 15.1, 14.3, 8.4, 5H), 6.91 - 6.60 (m, 3H), 6.21 (s, 1H), 5.83 (s, 1H), 4.48 (dd, *J* = 7.7, 4.6, 1H), 4.08 (dd, *J* = 13.7, 6.7, 2H), 3.87 (s, 3H), 3.17 (dd, *J* = 15.4, 4.5, 1H), 2.81 (dd, *J* = 15.4, 8.3, 1H), 1.45 (t, *J* = 7.0, 3H).
m/z: 642 ; 643 $[M+H]^+$

Example 10: 3-((2S,5R)-4-oxo-5-(2-oxo-2-((1,1,2,2-$d_4$-2-(3-ethoxy-4-methoxy-$d_3$-phenyl)ethyl)amino)ethyl)-2-(4-((phenyl-$d_5$)ethynyl)phenyl)thiazolidin-3-yl)benzamide

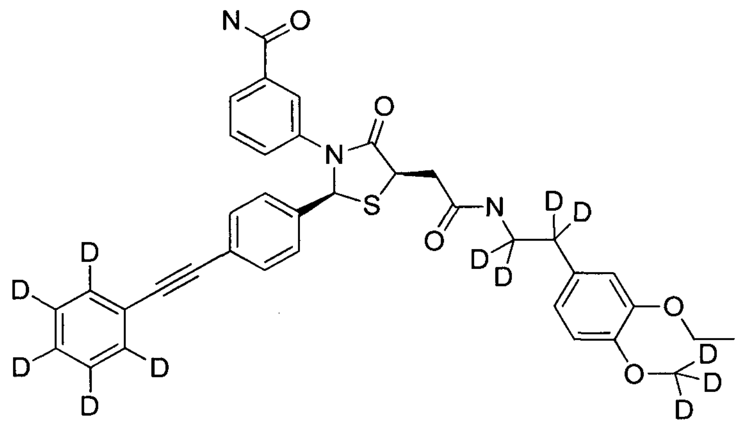

In a similar manner to example 1, product was obtained from 2-((2S,5R)-3-(3-carbamoylphenyl)-4-oxo-2-(4-(phenyl-$d_5$-ethynyl)phenyl)thiazolidin-5-yl)acetic acid (25.00 mg; 0.05 mmol; 1.00 eq.) and 2-(3-ethoxy-4-methoxy-$d_3$-phenyl)ethyl-1,1,2,2-$d_4$-amine HCl (12.05mg, 0.06mmol, 1.1 eq.). A 29.10 mg of the title product (83.2%) was obtained as white solid.
$^1$H NMR (400 MHz, $CDCl_3$ δ 7.72 (s, 1H), 7.59 (d, *J* = 7.3, 1H), 7.45 - 7.30 (m, 5H), 6.81 (d, *J* = 8.6, 1H), 6.74 (dd, *J* = 5.7, 2.0, 2H), 6.21 (s, 1H), 5.83 (s, 1H), 4.48 (dd, *J* = 8.2, 4.4, 1H), 4.08 (dd, *J* = 14.4, 7.3, 2H), 3.17 (dd, *J* = 15.4, 4.5, 1H), 2.81 (dd, *J* = 15.4, 8.3, 1H), 1.45 (t, *J* = 7.0, 3H).
m/z: 645 ; 646 $[M+H]^+$

Example 11: 3-((2S,5R)-4-oxo-5-(2-oxo-2-((1,1,2,2-$d_4$-2-(3-ethoxy-4-hydroxyphenyl)ethyl)amino)ethyl)-2-(4-((phenyl-$d_5$)ethynyl)phenyl)thiazolidin-3-yl)benzamide

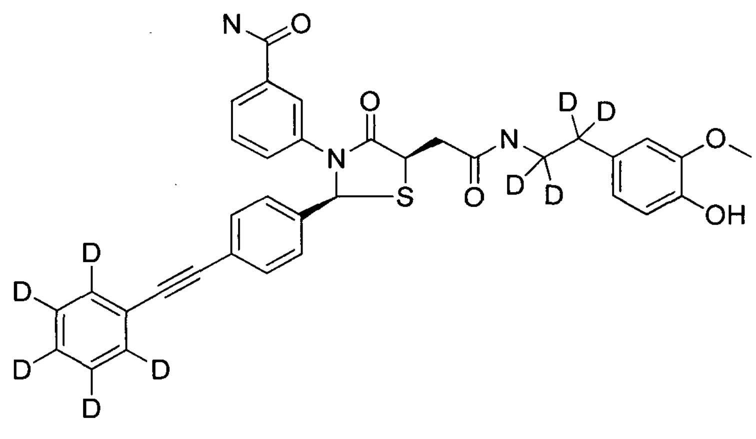

In a similar manner to example 1, product was obtained from 2-((2S,5R)-3-(3-carbamoylphenyl)-4-oxo-2-(4-(phenyl-$d_5$-ethynyl)phenyl)thiazolidin-5-yl)acetic acid (16.6 mg; 0.04 mmol; 1.00 eq.), and 2-(3-ethoxy-4-hydroxyphenyl)ethyl-1,1,2,2- $d_4$-amine HCl (8.22mg, 0.06mmol, 1.1 eq.). 2.5 mg (10%) of the title product was obtained as white solid.
$^1$H NMR (400 MHz, MeOD) δ 7.83 (s, 1H), 7.70 (d, *J* = 7.4, 1H), 7.49 - 7.38 (m, 6H), 7.11 (dd, *J* = 8.1, 1.6, 1H), 6.93 (s, 1H), 6.78 (dd, *J* = 8.2, 2.0, 1H), 6.45 (s, 1H), 4.53 (dd, *J* = 7.9, 4.5, 1H), 3.80 (s, 3H), 3.11 (d, *J* = 4.0, 1H), 2.92 (dd, *J* = 15.3, 8.4, 1H).
m/z: 614; 615 $[M+H]^+$

Example 12: 3-((2S,5R)-5-(2-((3,4-dihydroxyphenethyl)amino)-2-oxoethyl)-4-oxo-2-(4-((2,3,4,5,6-phenyl-$d_5$)ethynyl)phenyl)thiazolidin-3-yl)benzamide

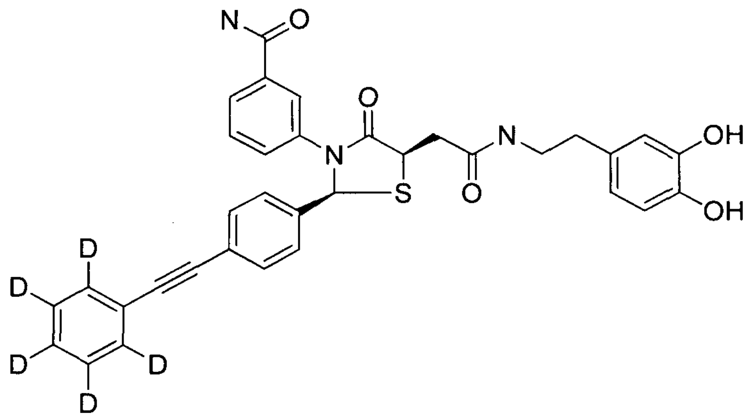

In a similar manner to example 1, product was obtained from 32-((2S,5R)-3-(3-carbamoylphenyl)-4-oxo-2-(4-(phenyl-$d_5$-ethynyl)phenyl)thiazolidin-5-yl)acetic acid (16.6 mg; 0.04 mmol; 1.00 eq.), and 4-(2-Amino-ethyl)-benzene-1,2-diol (6.06mg, 0.04 mmol, 1.1 eq.). The title compound was obtained (2.7 mg, 10%) of as white solid.
$^1$H NMR (400 MHz, MeOD) δ 7.82 (s, 1H), 7.70 (d, *J* = 7.3, 1H), 7.55 - 7.29 (m, 6H), 7.08 (d, *J* = 8.0, 1H), 6.86 (dd, *J* = 25.6, 8.1, 1H), 6.68 (dd, *J* = 8.2, 1.9, 1H), 6.44 (s, 1H), 4.53 (dd, *J* = 8.3, 4.0, 1H), 3.44 (qd, *J* = 13.6, 7.5, 2H), 3.14 (dd, *J* = 15.2, 4.2, 1H), 2.91 (dd, *J* = 15.3, 8.5, 1H), 2.81 - 2.70 (m, 2H).
m/z: 596 ; 597 $[M+H]^+$

Example 13: 3-((2S,5R)-4-oxo-5-(2-oxo-2-((1,1,2,2-$d_4$-2-(3-ethoxy-4-methox-$d_3$-yphenyl)ethyl)amino)ethyl)-2-(4-((phenyl-$d_5$)ethynyl)phenyl)thiazolidin-3-yl)benzamide

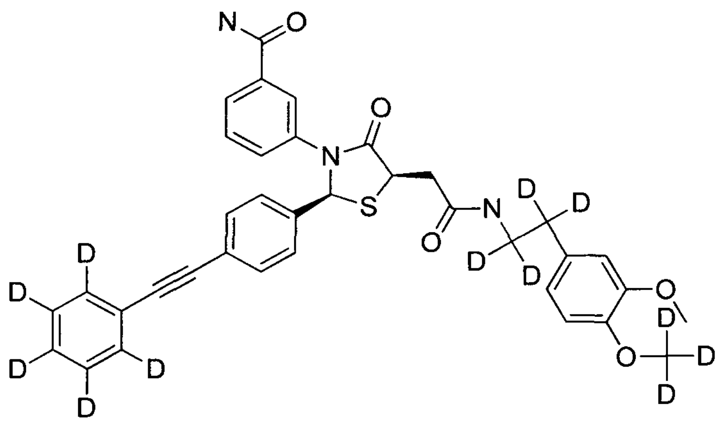

In a similar manner to example 3, product was obtained from product from example 11 (55mg, 0.09 mmol, 1 eq.) and dimethyl sulfate-$d_5$ (4.55 μl; 0.04 mmol; 0.50 eq.). A 26 mg (39%) of the title product was obtained as white solid.
$^1$H NMR (400 MHz, MeOD) δ 7.83 (d, *J* = 1.8, 1H), 7.70 (dt, *J* = 7.4, 1.5, 1H), 7.44 (ddd, *J* = 19.0, 11.4, 6.5, 6H), 6.83 (dd, *J* = 5.0, 3.1, 2H), 6.77 (dd, *J* = 8.2, 1.9, 1H), 6.45 (s, 1H), 4.53 (dd, *J* = 8.2, 3.8, 1H), 3.79 (s, 3H), 3.13 (dd, *J* = 15.3, 4.1, 1H), 2.92 (dd, *J* = 15.3, 8.5, 1H).
m/z: 631 ; 632 $[M+H]^+$

Example 14: 3-((2S,5R)-4-oxo-5-(2-oxo-2-((1,1,2,2-$d_4$-2-(3-ethoxy-4-methoxyphenyl)ethyl)amino)ethyl)-2-(2,3,5,6-$d_4$-4-(phenylethynyl)phenyl)thiazolidin-3-yl)benzamide

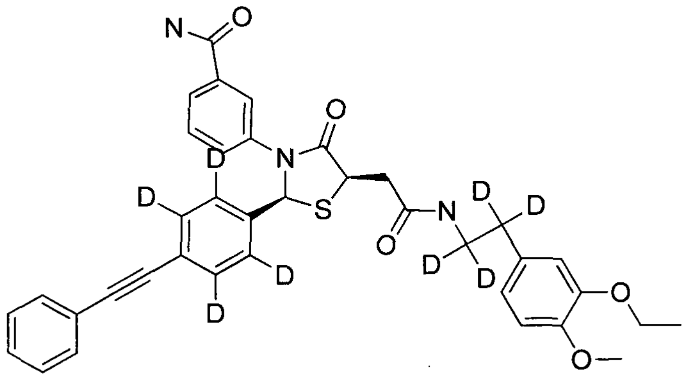

Step 1: To 4-chlorobenzaldehyde-2,3,5,6-$d_4$ (1.00 g; 6.92 mmol; 1.00 eq.) was added caesium carbonate (4506.86 mg; 13.83 mmol; 2.00 eq.), 2-dicyclohexylphosphino-2',4',6'-triisopropylbiphenyl (659.40 mg; 1.38 mmol; 0.20 eq.) and MeCN (60.00 ml; 1148.76 mmol; 166.10 eq.). Then phenylacetylene (1.12 ml; 10.24 mmol; 1.48 eq.) was added and reaction mixture was vacuum/nitrogen flush 3 times. Then dichlorobis(acetonitrile)palladium (II) (179.42 mg; 0.69 mmol; 0.10 eq.) was added and another vacuum/nitrogen flush was done. Reaction was put in a preheated oil bath (83°C) and heated for 5h. Product was isolated by flash chromatography (silica gel, 100g column, DCM/hexanes)

Step 2: In a similar manner to example 1 step 1&2, 2, 2-((2S,5R)-3-(3-carbamoylphenyl)-4-oxo-2-(4-(phenylethynyl)phenyl-$d_4$)thiazolidin-5-yl)acetic acid was obtained from 4-(phenyl-ethynyl)benzaldehyde-2,3,,5,6-$d_4$ (386 mg, 1.84 mmol), S)-2-Mercapto-succinic acid (826.95 mg; 5.51 mmol; 3.00 eq.) and 3-aminobenzamide (249.95 mg; 1.84 mmol; 1.00 eq.). Final product was isolated by prep HPLC. (27 mg, 5% yield over 2 steps).

Step 3: In a similar manner to example 1, product was obtained from 2, 2-((2S,5R)-3-(3-carbamoylphenyl)-4-oxo-2-(4-(phenyl-ethynyl)phenyl-$d_4$)thiazolidin-5-yl)acetic acid (13 mg, 0.02mmol), and 2-(3-ethoxy-4-methoxyphenyl)ethyl-1,1,2,2-$d_4$-amine HCl (5.87mg, 0.02mmol, 1.1 eq.). A 6.3 mg (43%) of the title compound was obtained as white solid.

$^1$H NMR (400 MHz, MeOD) δ 7.83 (d, *J* = 1.7, 1H), 7.73 - 7.63 (m, 1H), 7.54 - 7.27 (m, 7H), 6.88 - 6.72 (m, 3H), 6.44 (d, *J* = 0.8, 1H), 4.52 (dd, *J* = 8.2, 3.9, 1H), 4.07 - 3.95 (m, 2H), 3.78 (s, 3H), 3.12 (dd, *J* = 15.3, 4.1, 1H), 2.92 (dd, *J* = 15.3, 8.4, 1H), 1.35 (t, *J* = 7.0, 3H).

m/z: 641 ; 642 $[M+H]^+$

Example 15: 3-((2S,5R)-4-oxo-5-(2-oxo-2-((1,1,2,2-$d_4$-2-(3-ethoxy-4-methoxy-$d_3$-phenyl)ethyl)amino)ethyl)-2-(2,3,5,6-$d_4$-4-(phenylethynyl)phenyl)thiazolidin-3-yl)benzamide

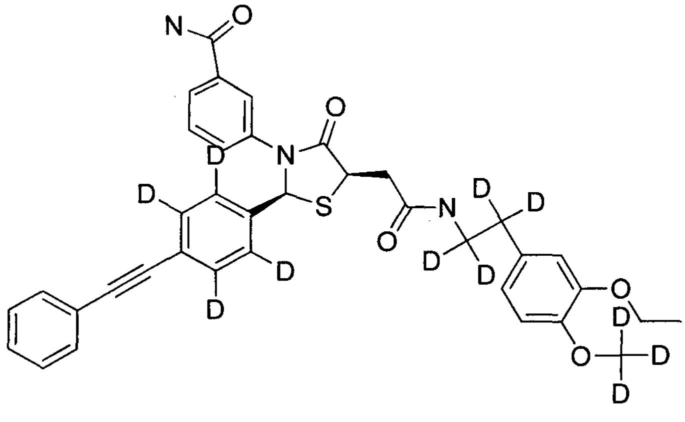

In a similar manner to example 1, product was obtained from 2, 2-((2S,5R)-3-(3-carbamoylphenyl)-4-oxo-2-(4-(phenyl-ethynyl)phenyl-$d_4$)thiazolidin-5-yl)acetic acid (13 mg, 0.02mmol), and 2-(3-ethoxy-4-methoxy-$d_3$-phenyl)ethyl-1,1,2,2-$d_4$-amine HCl (5.87mg, 0.02mmol, 1.1 eq.). The title compound was obtained as white solid (8.5 mg 58%).
$^1$H NMR (400 MHz, MeOD) δ 7.83 (t, *J* = 1.7, 1H), 7.70 (dt, *J* = 7.4, 1.5, 1H), 7.51 - 7.33 (m, 7H), 6.82 (dd, *J* = 7.0, 5.1, 2H), 6.77 (dd, *J* = 8.1, 2.0, 1H), 6.44 (d, *J* = 0.9, 1H), 4.55 - 4.47 (m, 1H), 4.06 - 3.97 (m, 2H), 3.12 (dd, *J* = 15.3, 4.1, 1H), 2.92 (dd, *J* = 15.3, 8.4, 1H), 1.36 (t, *J* = 7.0, 3H).
m/z: 644 ; 645 [M+H]$^+$

Example 16: 3-((2S,5R)-4-oxo-5-(2-oxo-2-((1,1,2,2-$d_4$-2-(3-ethoxy-4-methoxy-$d_3$-phenyl)ethyl)amino)ethyl)-2-(2,3,5,6-$d_4$-4-(phenylethynyl)phenyl-$d_5$)thiazolidin-3-yl)benzamide

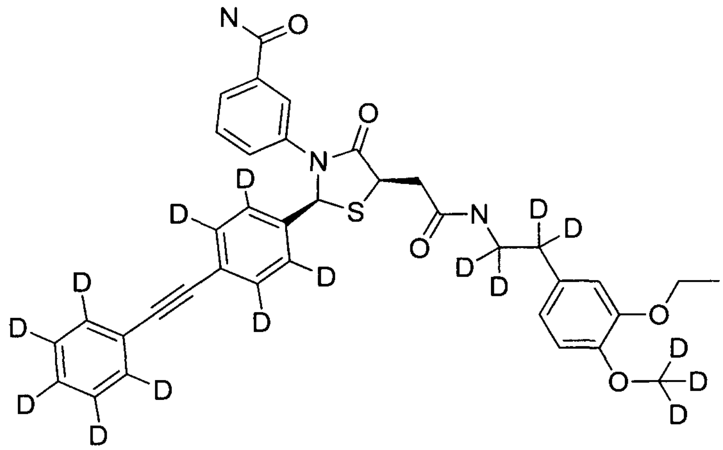

Step 1: In a similar manner to example 14 step 1, 4-(phenyl-$d_5$-ethynyl)benzaldehyde-2,3,4,6-$d_4$ was obtained from 4-chlorobenzaldehyde-2,3,5,6-$d_4$ (2.79 g; 19.30 mmol; 1.00 eq.), and Ethynyl-benzene-$d_5$ (3.10 g; 28.93 mmol; 1.50 eq.). Isolated 3.16 g (76%) of 4-(phenyl-$d_5$-ethynyl)benzaldehyde-2,3,5,6-$d_4$ as an off white solid.

Step 2: In a similar manner to example 1 step 1&2, 2-((2S,5R)-3-(3-carbamoylphenyl)-4-oxo-2-(4-(phenyl-$d_5$-ethynyl)phenyl-$d_4$)thiazolidin-5-yl)acetic acid was obtained from 4-(phenyl-$d_5$-ethynyl)benzaldehyde-2,3,5,6-$d_4$ (2.00 g; 9.29 mmol; 1.00 eq.), (S)-2-Mercapto-succinic acid (4.18 g; 27.87 mmol; 3.00 eq.) and 3-aminobenzamide (1.26 g; 9.29 mmol; 1.00 eq.) Final product was isolated by prep HPLC. (276 mg, 10% yield over 2 steps).

Step 3: In a similar manner to example 1, product was obtained from 2-((2S,5R)-3-(3-carbamoylphenyl)-4-oxo-2-(4-(phenyl-$d_5$-ethynyl)phenyl- $d_4$)thiazolidin-5-yl)acetic acid (60 mg, 0.13 mmol), and 2-(3-ethoxy-4-methoxy-$d_3$-phenyl)ethyl-1,1,2,2-$d_4$-amine HCl (33.85 mg, 0.14 mmol, 1.1 eq.). A 60 mg (72%) of the title compound was obtained as white solid.
$^1$H NMR (400 MHz, MeOD) δ 7.83 (s, 1H), 7.70 (d, *J* = 7.5, 1H), 7.50 - 7.35 (m, 2H), 6.79 (ddd, *J* = 10.1, 9.0, 5.1, 3H), 6.44 (s, 1H), 4.52 (dd, *J* = 8.3, 4.0, 1H), 4.01 (q, *J* = 7.0, 2H), 3.12 (dd, *J* = 15.3, 4.1, 1H), 2.92 (dd, *J* = 15.3, 8.4, 1H), 1.35 (t, *J* = 7.0, 3H).
m/z: 649 ; 650 [M+H]$^+$

Example 17: 3-((2S,5R)-4-oxo-5-(2-oxo-2-((1,1,2,2-$d_4$-2-(3,4-dimethoxy-$d_6$-phenyl)ethyl)amino)ethyl)-2-(2,3,5,6-$d_4$-4-(phenylethynyl)phenyl-$d_5$)thiazolidin-3-yl)benzamide

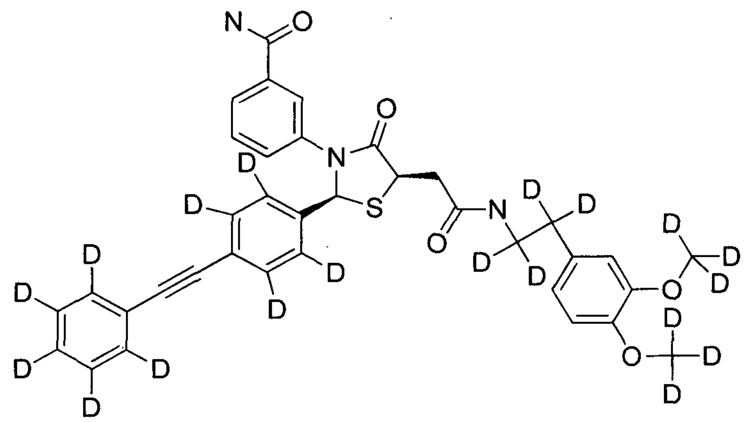

Step 1: In a similar manner to example 1, product was obtained from 2-((2S,5R)-3-(3-carbamoylphenyl)-4-oxo-2-(4-(phenyl-$d_5$-ethynyl)phenyl-$d_4$)thiazolidin-5-yl)acetic acid (50 mg, 0.11 mmol) and 2-(3,4-dihydroxy)ethyl-1,1,2,2-$d_4$-amine HCl (22.8 mg, 0.12 mmol, 1.1 eq.). Isolated 6 mg (9%) of product as white solid.

Step 2: In a similar manner to example 3, product was obtained from product example 17 step 1 (6 mg, 0.01 mmol) and dimethyl sulfate-$d_6$ (0.5 μl; 0.005 mmol; 0.50 eq.). Isolated 3.2 mg (52%) of the title product as white solid.
$^1$H NMR (400 MHz, MeOD) δ 7.83 (d, *J* = 1.7, 1H), 7.70 (d, *J* = 7.5, 1H), 7.43 (dt, *J* = 15.4, 4.8, 2H), 6.83 (dd, *J* = 5.0, 3.1, 2H), 6.77 (dd, *J* = 8.2, 1.9, 1H), 6.45 (s, 1H), 4.53 (dd, *J* = 8.4, 4.0, 1H), 3.12 (dd, *J* = 15.3, 4.1, 1H), 2.92 (dd, *J* = 15.3, 8.5, 1H).
m/z: 638 ; 639 $[M+H]^+$

Example 18:

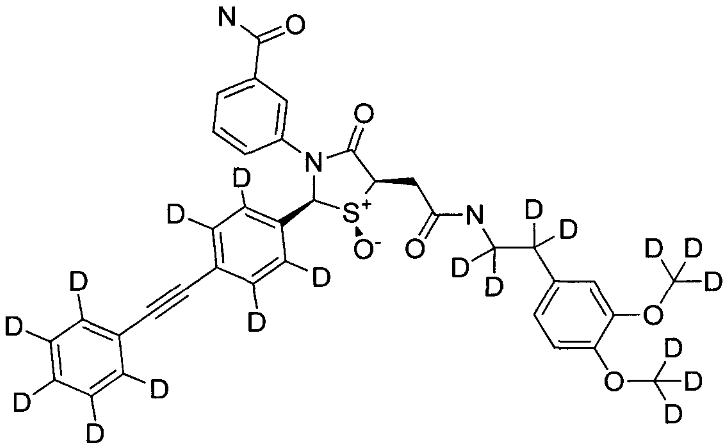

To product from example 17 (100.00 mg; 0.16 mmol; 1.00 eq.) in MeCN (3.80 ml; 72.75 mmol; 464.79 eq.) and water (0.20 ml; 11.10 mmol; 70.92 eq.) (10:1) was added a 1 mL solution of 1-chloromethyl-4-fluoro-1,4-diazoniabicyclo[2.2.2]octane bis(tetrafluoroborate) (66.54 mg; 0.19 mmol; 1.20 eq.) in MeCN (2mL) and water (0.2mL). The reaction was stirred at RT for 30 min. Product was isolated by prep HPLC (40% ACN/60% water for 5min, then up to 60% ACN/40% water for 20min, 254nm, 0.1 %TFA). A 36 mg (30%) of the title product was obtained as white solid. Stereochemistry at sulfoxide was arbitrary assigned.
$^1$H NMR (400 MHz, MeOD) δ 7.85 (s, 1H), 7.68 (d, *J* = 7.5, 1H), 7.48 - 7.35 (m, 2H), 6.93 - 6.84 (m, 2H), 6.79 (dd, *J* = 8.1, 1.9, 1H), 6.55 (s, 1H), 4.55 (dd, *J* = 10.6, 3.8, 1H), 3.23 (dd, *J* = 16.8, 3.8, 1H), 3.02 (dd, *J* = 16.7, 10.7, 1H).
m/z: 654 ; 655 $[M+H]^+$

Example 19:

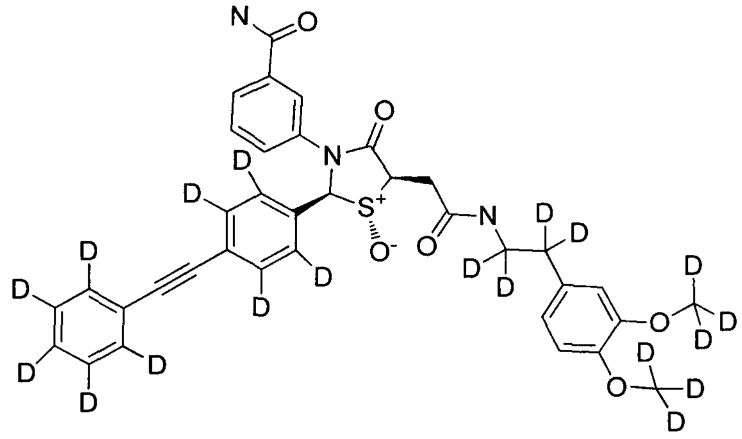

To product from example 17 (80.00 mg; 0.13 mmol; 1.00 eq.) in dichloromethane (2mL) was added 3-chloroperbenzoic acid (28.06 mg; 0.13 mmol; 1.00 eq.). The reaction was stirred at RT for 1h. Product was isolated by prep HPLC (40 to 65% ACN/water over 20 min, 0.1% TFA). A 46.2 mg (48%) of the title product was obtained as white solid. Stereochemistry at sulfoxide was arbitrary assigned.
$^1$H NMR (400 MHz, MeOD) δ 8.07 (s, 1H), 7.79 (d, *J* = 7.9, 1H), 7.71 (d, *J* = 9.4, 1H), 7.53 (t, *J* = 8.0, 1H), 6.86 (dd, *J* = 7.0, 5.1, 2H), 6.77 (dd, *J* = 8.1, 2.0, 1H), 6.40 (s, 1H), 4.41 (dd, *J* = 10.5, 4.0, 1H), 3.17 - 3.10 (m, 1H), 2.90 (dd, *J* = 16.8, 10.5, 1H).
m/z: 654 ; 655 $[M+H]^+$

Examples 20 and 21:

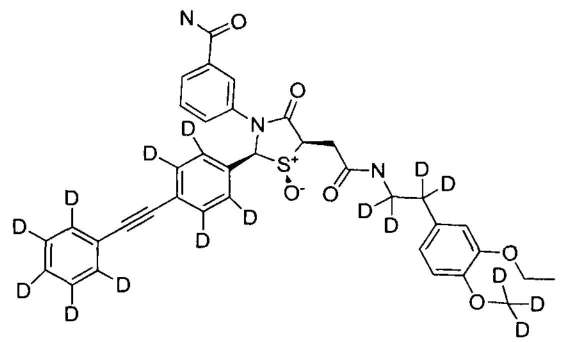

Example 20

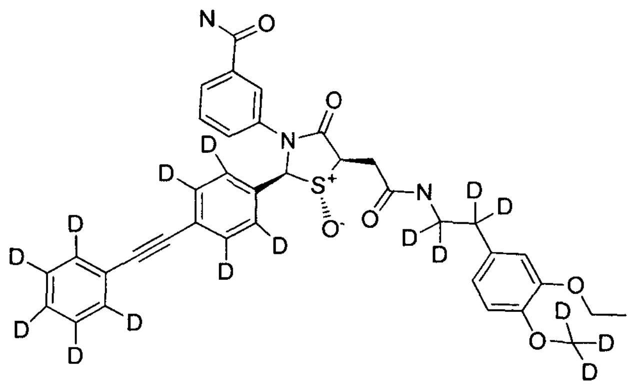

Example 21

To product from example 16 (45.00 mg; 0.07 mmol; 1.00 eq.) in dichloromethane was added 3chloroperbenzoic acid (15.52 mg; 0.07 mmol; 1.00 eq.). The reaction was stirred at RT for 4h. Product was purified by prep HPLC (40 to 65% ACN/water over 20 min, 0.1% TFA). Product example 20 eluted at 53%. Isolated 3.7 mg of example 20 (7%) as white solid. Product example 21, eluted at 55%. A 10.6 mg (20%) of example 21 as white solid was obtained. Stereochemistry at sulfoxide arbitrary assigned.
Example 20: $^1$H NMR (500 MHz, $cd_3od$) δ 7.86 (d, *J* = 14.5, 1H), 7.67 (d, *J* = 8.0, 1H), 7.58 - 7.23 (m, 2H), 7.07 - 6.68 (m, 3H), 6.54 (s, 1H), 4.54 (d, *J* = 7.0, 1H), 4.06 (q, *J* = 6.8, 2H), 3.24 - 3.08 (m, 1H), 3.00 (dd, *J* = 16.8, 10.9, 1H), 1.38 (t, *J* = 7.0, 3H).
m/z: 654 ; 655 $[M+H]^+$

Example 21:

$^1$H NMR (500 MHz, $cd_3od$) δ 8.06 (s, 1H), 7.74 (dd, *J* = 34.9, 8.1, 2H), 7.51 (t, *J* = 7.9, 1H), 6.97-6.68 (m, 3H), 6.40 (s, 1H), 4.39 (dd, *J* = 10.5, 3.9, 1H), 4.05 (q, *J* = 7.0, 2H), 3.18 - 3.02 (m, 1H), 2.89 (dd, *J* = 16.8, 10.6, 1H), 1.43 - 1.23 (m, 3H).

m/z: 654 ; 655 [M+H]+

Example 22: 3-((2S,5R)-4-oxo-5-(2-oxo-2-((1,1,2,2-$d_4$-2-(3-ethoxy-4-methoxyphenyl)ethyl)amino)ethyl)-2-(4-((phenyl)ethynyl)phenyl)thiazolidin-3-yl)benzamide

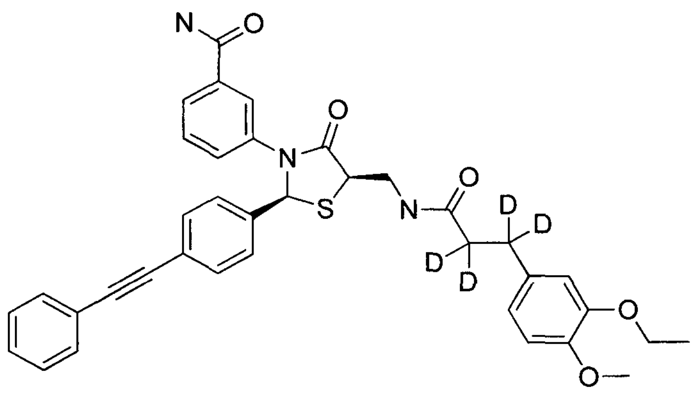

Step 1: To [(2S,5R)-3-(3-Carbamoyl-phenyl)-4-oxo-2-(4-phenylethynyl-phenyl)-thiazolidin-5-yl]-acetic acid (150.00 mg; 0.33 mmol; 1.00 eq.) , in toluene (1.00 ml), was added N-ethyl-N-isopropylpropan-2-amine (0.06 ml; 0.33 mmol; 1.00 eq.), diphenyl azidophosphate (0.08 ml; 0.38 mmol; 1.15 eq.) and 2-methylpropan-2-ol (1.00 ml). Mixture was heated to 100C for 5h. Reaction was cooled, concentrated and purified by flash chromatography (silica gel 10g, 0 to 5% MeOH/EtOAc). Isolated 91 mg of [(2S,5R)-3-(3-Carbamoyl-phenyl)-4-oxo-2-(4-phenylethynyl-phenyl)-thiazolidin-5-ylmethyl]-carbamic acid tert-butyl ester (53%) as an off white solid.

Step 2: To [(2S,5R)-3-(3-Carbamoyl-phenyl)-4-oxo-2-(4-phenylethynyl-phenyl)-thiazolidin-5-ylmethyl]-carbamic acid tert-butyl ester (90.00 mg; 0.17 mmol; 1.00 eq.) in dichloromethane (1.00 ml) was added trifluoroacetic acid (50.00 μl; 0.67 mmol; 3.93 eq.). The reaction was stirred at room temperature overnight. Product was purified by flash chromatography (KPNH, 10g, 0 to 20% MeOH/DCM). Isolated 53 mg of 3-[(2S,5R)-5-aminomethyl-4-oxo-2-(4-phenylethynyl-phenyl)-thiazolidin-3-yl]-benzamide (77%) as white solid.

Step 3: To 3-[(2S,5R)-5-Aminomethyl-4-oxo-2-(4-phenylethynyl-phenyl)-thiazolidin-3-yl]-benzamide (30.00 mg; 0.07 mmol; 1.00 eq.) in DCM (2.00 ml; 31.20 mmol; 444.63 eq.) was added 3-(3-ethoxy-4-methoxyphenyl)propanoic-2,2,3,3-$d_4$ acid (19.22 mg, 0.08 mmol, 1.1 eq.), N,N-Diisopropylethylamine (0.01 ml; 0.08 mmol; 1.20 eq.) and 2,4,6-tripropyl-1,3,5,2,4,6-trioxatriphosphinane 2,4,6-trioxide (0.03 ml; 0.11 mmol; 1.50 eq.). The reaction was stirred at RT 1h. Product was purified by flash chromatography (KPNH, 0 to 20% MeOH/DCM). The title product was obtained as white solid (30.1 mg 68%).
$^1$H NMR (400 MHz, $CDCl_3$) δ 7.73 (s, 1H), 7.66 (d, *J* = 7.9, 1H), 7.52 (dd, *J* = 12.3, 5.4, 2H), 7.44 (d, *J* = 8.3, 2H), 7.36 (dd, *J* = 5.8, 2.6, 3H), 7.27 - 7.19 (m, 3H), 6.83 (d, *J* = 8.8, 1H), 6.80 - 6.74 (m, 2H), 6.45 (s, 1H), 6.11 (s, 1H), 4.14 - 4.03 (m, 3H), 4.02 - 3.95 (m, 1H), 3.85 (s, 3H), 3.53 (d, *J* = 12.7, 1H), 1.42 (t, *J* = 7.0, 3H).
m/z: 637 ; 638 [M+H]+

**$EC_{50}$ of cyclic AMP production in CHO FSHR cells + $EC_{20}$ FSH**

2500 Cho-FSHR-LUC-1-1-43 cells were plated per well in 5 μL of phenol red free DMEM/F12 + 1% FBS. Cells were plated in 384 well, solid white low volume plates (Greiner 784075) by Multidrop. Cells were assayed by adding 100 μL of 2X $EC_{20}$ FSH/IBMX in DMEM/F12 + 0.1 % BSA) by Multidrop to 2 μL of test compound stamped in 384 well plates (compounds are diluted 1:50). The final FSH concentration was 0.265 pM, and the final IBMX concentration was 200 μM. The compound plate map was as follows: Column 1: 2 μL of DMSO; Column 2: 2 μL of DMSO; Columns 3-12 and 13-24: 2 μL of test compound, diluted 1:4 in 100% DMSO, or 2 μL of FSH, diluted 1:4 in DMEM/F12+0.1% BSA. The starting concentration for FSH was 50 nM (final concentration was 0.5 nM). Furthermore, Column 23 contained 2 μL of $EC_{100}$ FSH reference (100X) (diluted in DMEM/F12 + 0.1% BSA) at a final concentration of 0.5 nM, and Column 24 contained 2 μL of 1 mM AS707664/2 reference compound 2. 5 μL of compound + $EC_{20}$ FSH mixture were transferred to cell plates (1:2 dilution into 5 μL of cell media) The plates were incubated at 37 °C for 1 h. 10 μL of mixed HTRF (CisBio # 62AM4PEC) reagents were added per well and incubated at room temperature for 1 h. The plates were read on Envision using the cAMP HTRF - low volume 384 well protocol. The readout was the calculated fluorescence ratio (665 nm / 620 nm).

The results are depicted in table 1.

**Intrinsic clearance (Clint) assay protocol:**

Instrumentation

A Tecan Genesis workstation (RSP 150/8) was used for to perform the microsomal incubations. Analysis was carried out using a Waters ACQUITY UPLC system coupled to an ABSciex API3000 mass spectrometer. Data analysis was performed using Assay Explorer (Symyx).

| UPLC conditions | | | |
|---|---|---|---|
| Column : Acquity UPLC BEH C18, 2.1 x 50mm, 1.7 um (Waters) | | | |
| Mobile phases : | A = 0.1 % formic acid in water | | |
| | B = acetonitrile | | |
| Gradient: | Time | %A | %B |
| | initial | 90 | 10 |
| | 0.47 | 5 | 95 |
| | 0.65 | 5 | 95 |
| | 0.66 | 90 | 10 |
| Flow rate : | 0.750 mL/min | | |
| Detection : | ESI, MRM | | |
| Injection: | 10 uL | | |
| Column temperature: | 50°C | | |

Chemicals

0.1 M potassium phosphate buffer pH 7.4 containing 1 mM $MgCl_2$

15 mM NADPH in phosphate buffer

4.4 mg protein/mL liver microsomes in phosphate buffer

acetonitrile

20% DMSO in water

Microsomal Incubation

Each experiment consists of 12 test and 2 reference compounds. The reference compounds are incubated as a cocktail.

Dilution of test compounds was done in 2 steps from a 10 mM DMSO stock solution. First 4 μL stock solution was added to 196 μL of 20% DMSO in potassium phosphate buffer pH 7.4. In a second step 10 μL of the first dilution were added to 1890 μL potassium phosphate buffer and 100 μL internal standard solution to a final concentration of 0.8 μM.

100 μL of the final compound dilution were aliquoted into a 96 deep well plate. 12.5 μL liver microsomes were added to each well (0.5 mg/mL final protein concentration) and the samples preincubated for 5 min at 37 °C and 800 rpm agitation.

After the preincubation, 250 μL cold acetonitrile were added to the 0 min samples to prevent a reaction. Following this, 12.5 μL NADPH solution were added to all wells to start the incubation, with the exception of the 0 min and 30 min controls without cofactor, where the NADPH was substituted for phosphate buffer.

The incubations were stopped after 5, 10, 20 and 30 min by adding 250 μL cold acetonitrile to the individual wells.

The quenched samples were then centrifuged at 4000g for 1 h at 4 °C. 100 μL of the supernatant were transferred into 96 well plates for analysis.

Data Analysis

The metabolic stability of each compound was determined by measurement of the change in LC-MS/MS peak area over time. Assay Explorer software was used to automatically calculate the slope k of the decline. The intrinsic clearance (Clint) of each compound was then calculated according to the formula:

$$\text{Clint } (\mu L/min/mg \text{ protein}) = k \text{ } 1000 \text{ /protein concentration.}$$

Classification of results

| | Human | Rat |
|---|---|---|
| low clearance | <10 | <17 |
| medium clearance | 10 - 55 | 17 - 100 |
| high clearance | >55 | >100 |

The results are depicted in table 1.

**Table 1**

| Compound | $EC_{50}$ of cAMP production [nM (% effect)] | Clint (human/rat) [μL/min/mg protein] |
|---|---|---|
| Reference compound | 0.3 (98) | 182/120 |
| 1 | 0.87 (99) | 67/31 |
| 2 | 1.9 (97) | 106/24 |
| 3 | 1.7 (97) | 111/29 |
| 4 | 0.7 (99) | 191/51 |
| 5 | 0.8 (99) | 168/45 |
| 6 | 0.7 (96) | 208/56 |
| 7 | 0.5 (99) | 184/44 |
| 8 | 1.7 (99) | 188/54 |
| 9 | 0.3 (97) | 181/31 |
| 10 | 0.3 (98) | 161/23 |
| 11 | 335 (99) | |
| 12 | 220 (99) | |
| 13 | 1.3 (97) | |
| 14 | 2 (99) | 492/95 |
| 15 | 0.6 (99) | |
| 16 | 0.7 (100) | |
| 17 | 0.9 (100) | |
| 18 | 0.7 (101) | |
| 19 | 101 (100) | 127/61 |
| 20 | 25 (103) | 35/18 |
| 21 | 113 (103) | 351/241 |

(continued)

| | | |
|---|---|---|
| 22 | 349 (101) | 46/11 |

## Claims

1. **1.** Deuterated thiazolidinone derivative according to formula (I)

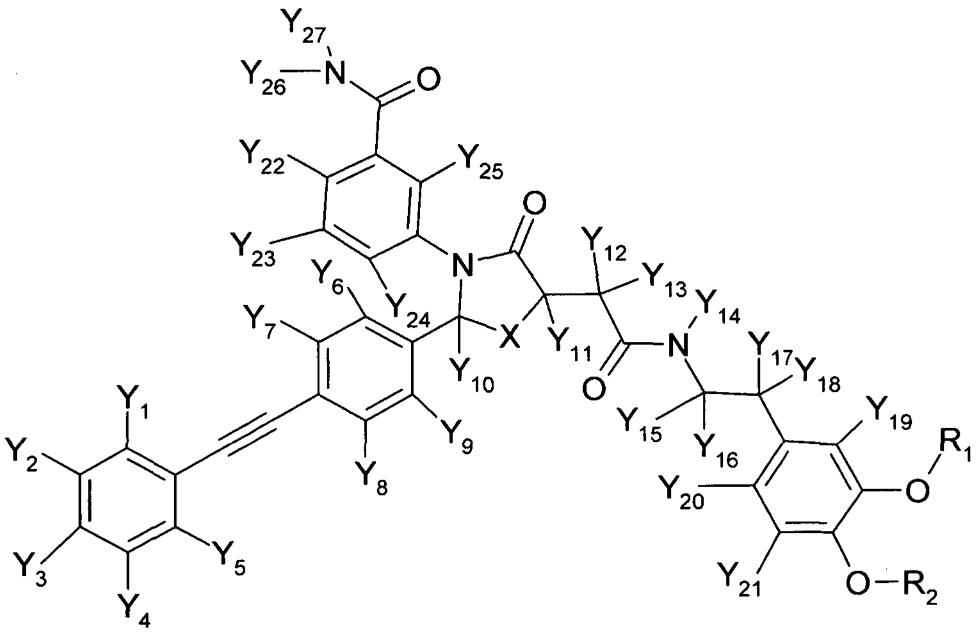

(I)

wherein:

each Y is independently from each other selected from the group consisting of: "hydrogen (H), deuterium (D)";
X is selected from the group consisting of: "S, sulfoxide";
R1, R2 are independently from each other selected from the group consisting of: "hydrogen (H), deuterium (D), methyl, $CH_2D$, $CHD_2$, $CD_3$, ethyl, $CHDCH_3$, $CHDCH_2D$, $CHDCHD_2$, $CHDCD_3$, $CD_2CH_3$, $CD_2CH_2D$, $CD_2CHD_2$, $CD_2CD_3$";
with the proviso that

(i) $Y_{15}$, $Y_{16}$, $Y_{17}$, $Y_{18}$ are deuterium (D), or
(ii) $Y_1$, $Y_2$, $Y_3$, $Y_4$, $Y_5$ are deuterium (D), or
(iii) one of R1, R2 comprises at least one deuterium (D);

optionally, at least one carbon atom is independently from each other replaced by $^{13}C$;
and the physiologically acceptable salts, solvates, tautomers and stereoisomers thereof, including mixtures thereof in all ratios.

**2.** Deuterated thiazolidinone derivative as claimed in claim 1, wherein
$R_1$ and/or $R_2$ are $CD_3$;
and the physiologically acceptable salts, solvates, tautomers and stereoisomers thereof, including mixtures thereof in all ratios.

**3.** Deuterated thiazolidinone derivative as claimed in any of claims 1 or 2, wherein

(a) $Y_{15}$, $Y_{16}$, $Y_{17}$, $Y_{18}$ are deuterium (D) and the other Y are hydrogen (H) and none of $R_1$, $R_2$ comprises one or more deuterium (D); or
(b) $Y_{15}$, $Y_{16}$, $Y_{17}$, $Y_{18}$ are deuterium (D) and the other Y are hydrogen (H) and one of $R_1$, $R_2$ is $CD_3$ and the

other one of $R_1$, $R_2$ does not comprise one or more deuterium (D); or

(c) all Y are hydrogen (H) and one of $R_1$, $R_2$ is $CD_3$ and the other one of $R_1$, $R_2$ does not comprise one or more deuterium (D); or

(d) $Y_1$, $Y_2$, $Y_3$, $Y_4$, $Y_5$ are deuterium (D) and the other Y are hydrogen (H) and none of $R_1$, $R_2$ comprises one or more deuterium (D); or

(e) all Y are hydrogen (H) and both of $R_1$, $R_2$ are $CD_3$; or

(f) $Y_1$, $Y_2$, $Y_3$, $Y_4$, $Y_5$, $Y_{15}$, $Y_{16}$, $Y_{17}$, $Y_{18}$ are deuterium (D) and the other Y are hydrogen (H) and none of $R_1$, $R_2$ comprises one or more deuterium (D); or

(g) $Y_1$, $Y_2$, $Y_3$, $Y_4$, $Y_5$, $Y_{15}$, $Y_{16}$, $Y_{17}$, $Y_{18}$ are deuterium (D) and the other Y are hydrogen (H) and one of $R_1$, $R_2$ is $CD_3$ and the other one of $R_1$, $R_2$ does not comprise one or more deuterium (D); or

(h) $Y_6$, $Y_7$, $Y_8$, $Y_9$, $Y_{15}$, $Y_{16}$, $Y_{17}$, $Y_{18}$ are deuterium (D) and the other Y are hydrogen (H) and one of $R_1$, $R_2$ is $CD_3$ and the other one of $R_1$, $R_2$ does not comprise one or more deuterium (D);

and the physiologically acceptable salts, solvates, tautomers and stereoisomers thereof, including mixtures thereof in all ratios.

**4.** Deuterated thiazolidinone derivative selected from the group consisting of:

| Compound | Structure |
|---|---|
| 1 | 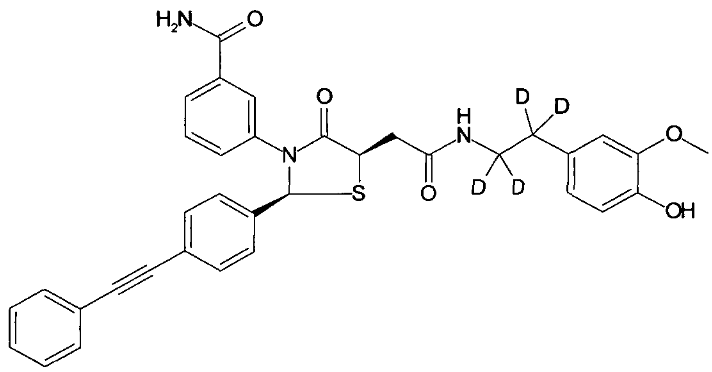 |
| 2 | 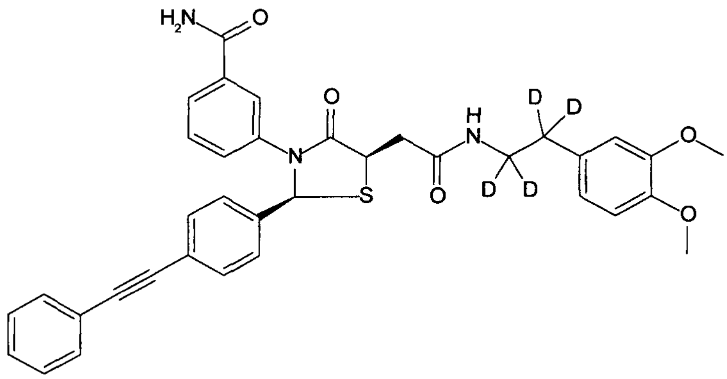 |
| 3 | 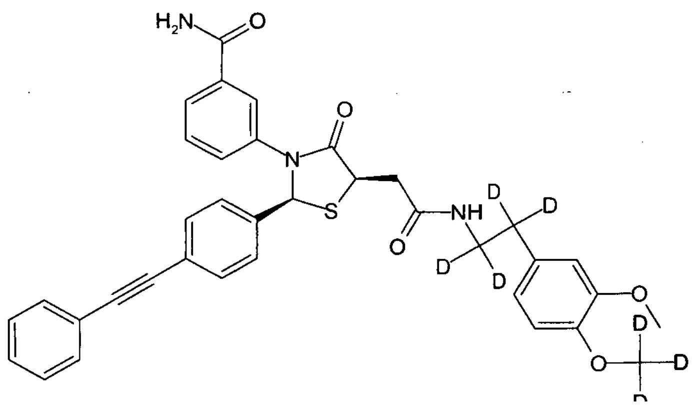 |

(continued)

| Compound | Structure |
|---|---|
| 4 | 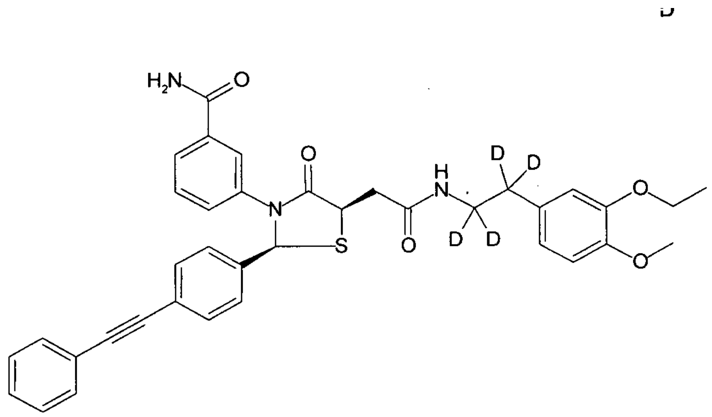 |
| 5 | 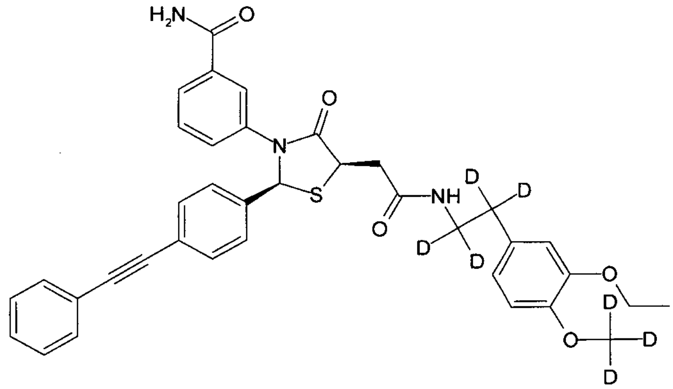 |
| 6 | 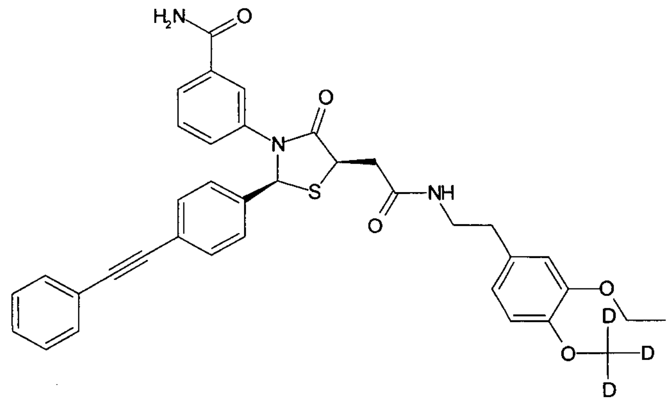 |
| 7 | 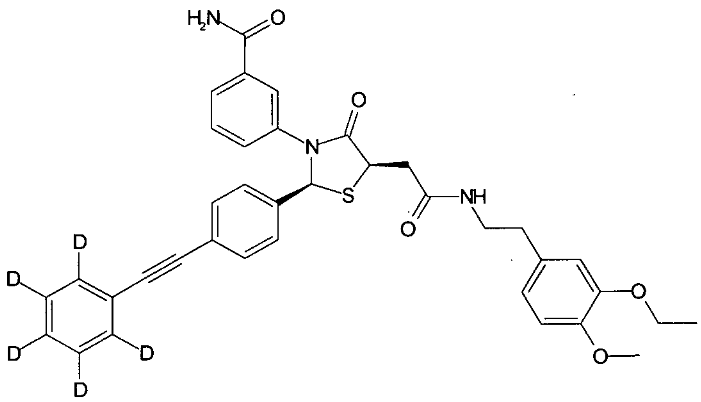 |

(continued)

| Compound | Structure |
|---|---|
| **8** | 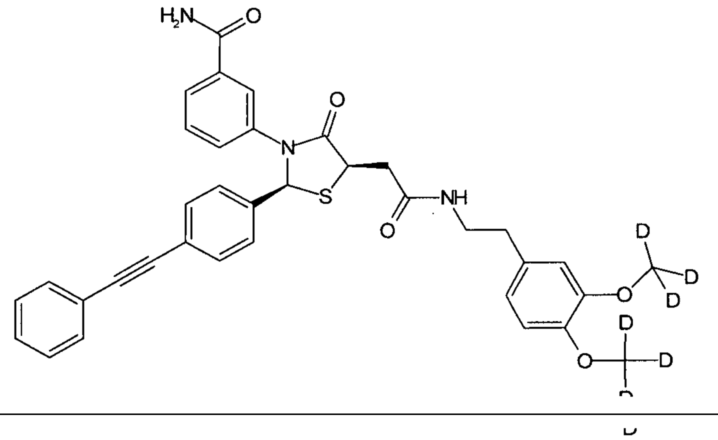 |
| **9** | 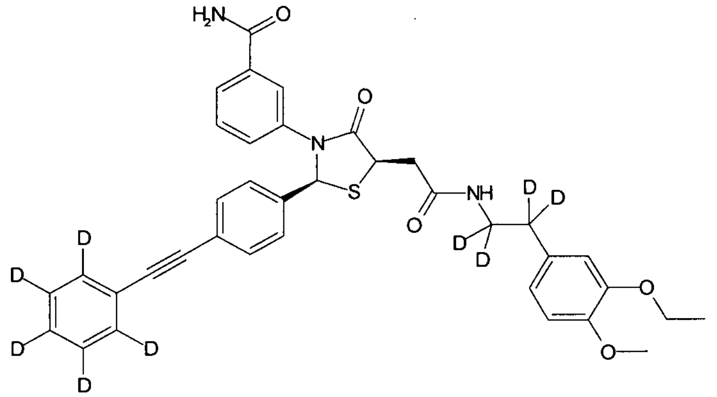 |
| **10** | 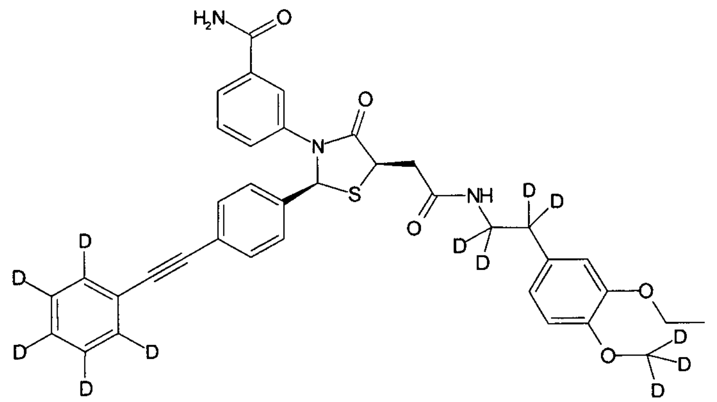 |
| **11** | 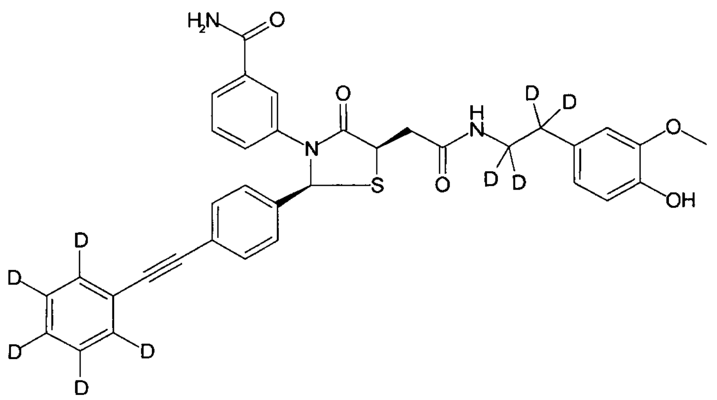 |

(continued)

| Compound | Structure |
|---|---|
| **12** | 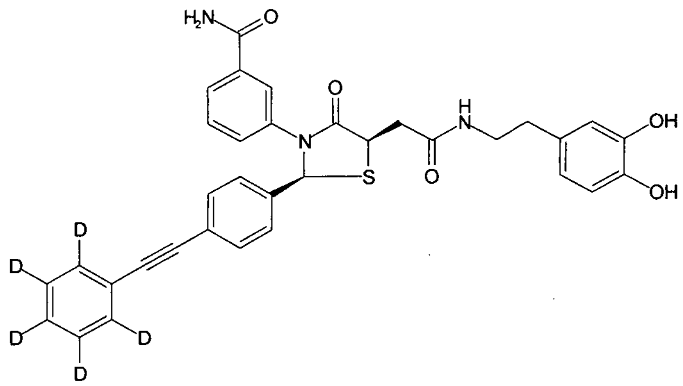 |
| **13** | 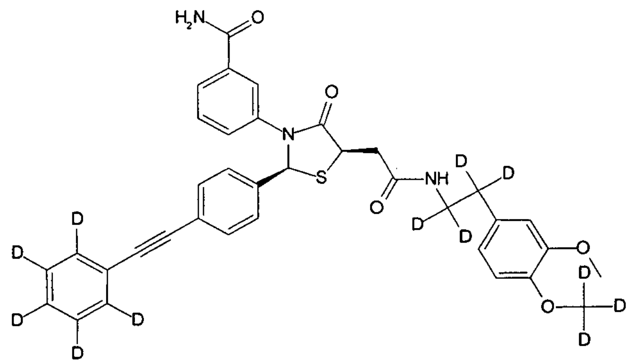 |
| **14** | 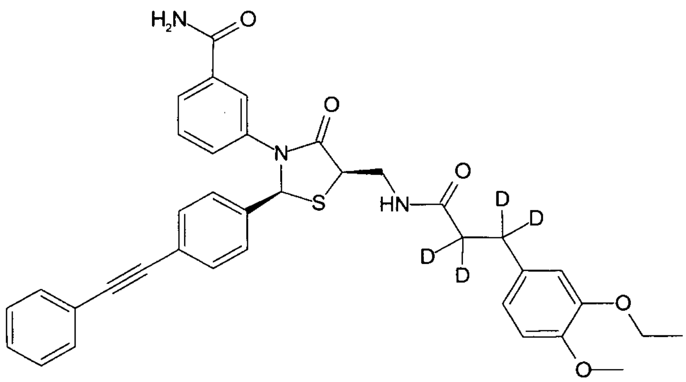 |
| **15** | 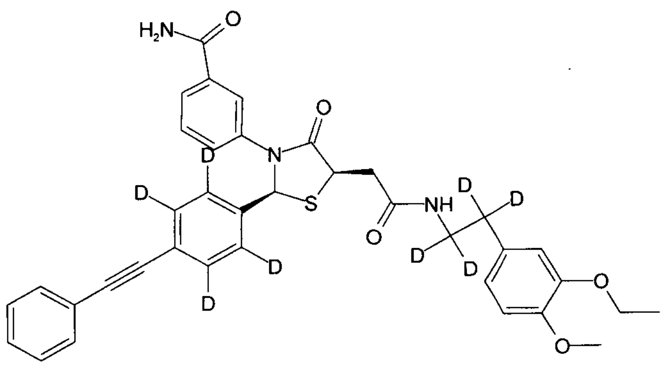 |

(continued)

| Compound | Structure |
|---|---|
| 16 | 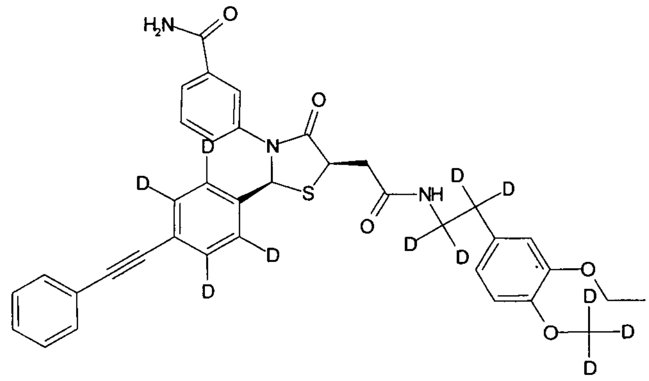 |
| 17 | 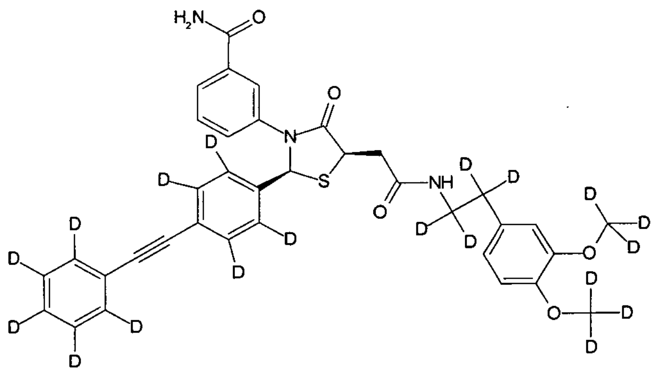 |
| 18 | 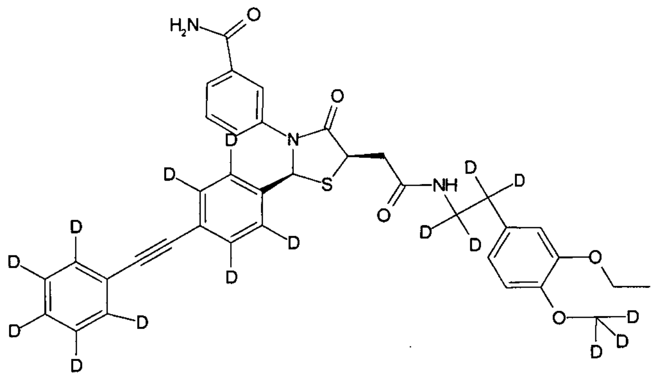 |
| 19 | 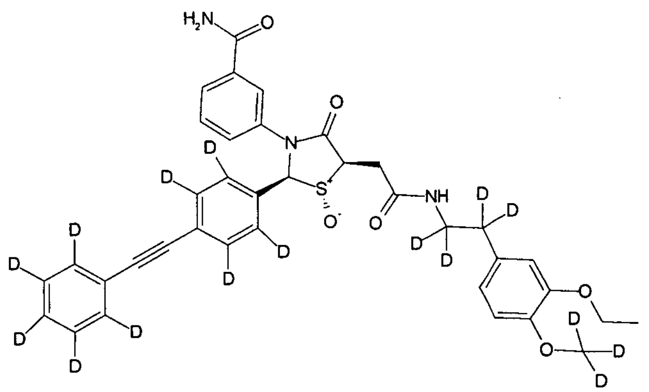 |

(continued)

| Compound | Structure |
|---|---|
| 20 | 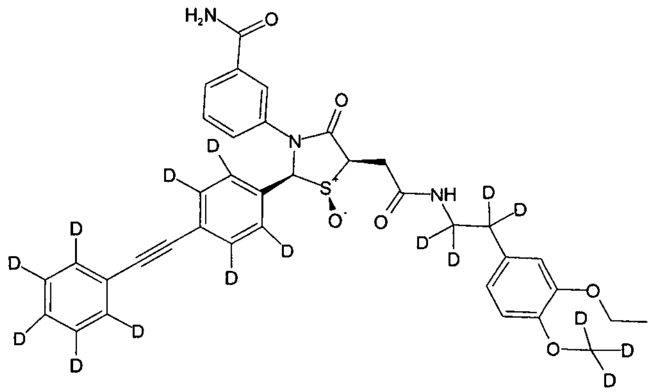 |
| 21 | 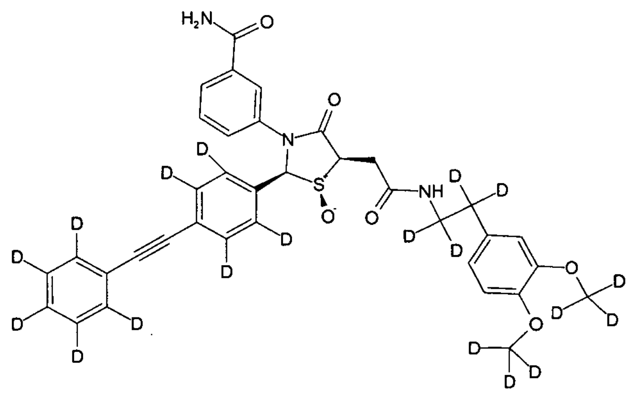 |
| 22 | 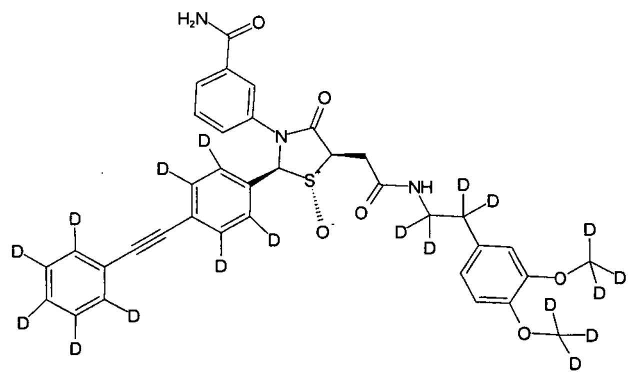 |

and the physiologically acceptable salts, solvates, tautomers and stereoisomers thereof, including mixtures thereof in all ratios.

**5.** Medicament comprising at least one compound according to any of claims 1 to 4.

**6.** Medicament comprising at least one compound according to any of claims 1 to 4 for use in the prophylactic or therapeutic treatment and/or monitoring of fertility disorders.

**7.** Pharmaceutical composition comprising a therapeutically effective amount of at least one compound according to any of claims 1 to 4, optionally further comprising at least one additional compound selected from the group consisting of physiologically acceptable excipients, auxiliaries, adjuvants, diluents, carriers and/or additional pharmaceutically active substance other than the compound according to any of claims 1 to 4.

**8.** Kit comprising a therapeutically effective amount of at least one compound according to any of claims 1 to 4 and/or

at least one pharmaceutical composition according to claim 7 and a therapeutically effective amount of at least one further pharmacologically active substance other than the compound according to any of claims 1 to 4.

**9.** A compound according to any of claims 1 to 4 or at least one pharmaceutical composition according to claim 7 for use in the modulation of a FSH receptor in a positive allosteric manner, wherein a cell capable of expressing the FSH receptor is contacted in the presence of FSH with the said compound.

**10.** A compound according to any of claims 1 to 4 or at least one pharmaceutical composition according to claim 7 for use in the treatment of fertility disorders, wherein a therapeutically effective amount of the said compound is administered to a mammal in need of such treatment.

**Patentansprüche**

**1.** Deuteriertes Thiazolidinonderivat nach Formel (I)

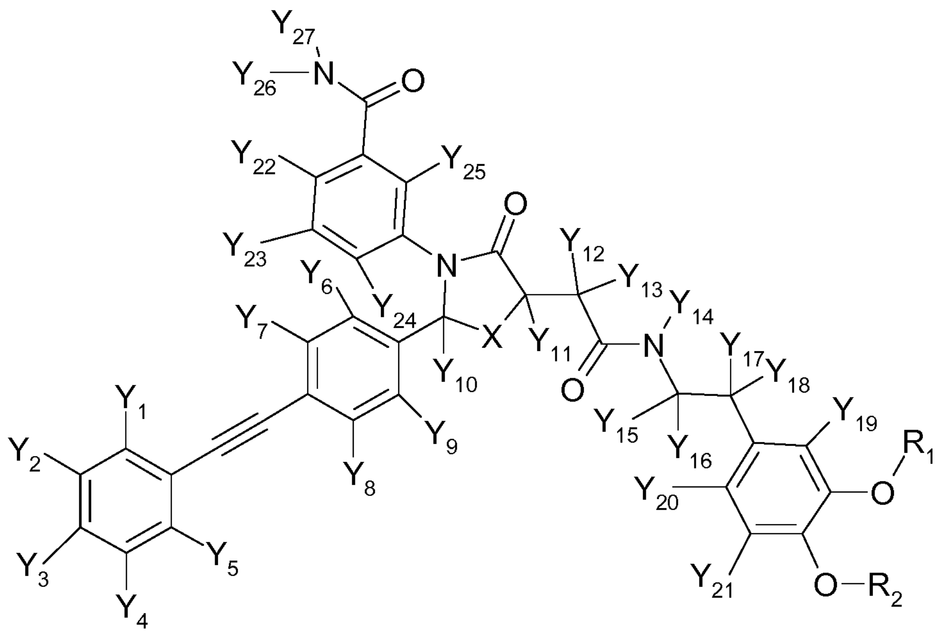

**(I)**

bei der:

jedes Y unabhängig voneinander aus der Gruppe ausgewählt ist, die besteht aus: "Wasserstoff (H), Deuterium (D)";
X aus der Gruppe ausgewählt ist, die besteht aus: "S, Sulfoxid";
R1, R2 unabhängig voneinander aus der Gruppe ausgewählt sind, die besteht aus: "Wasserstoff (H), Deuterium (D), Methyl, $CH_2D$, $CHD_2$, $CD_3$, Ethyl, $CHDCH_3$, $CHDCH_2D$, $CHDCHD_2$, $CHDCD_3$, $CD_2CH_3$, $CD_2CH_2D$, $CD_2CHD_2$, $CD_2CD_3$";
mit der Maßgabe, dass

(i) $Y_{15}$, $Y_{16}$, $Y_{17}$, $Y_{18}$ Deuterium (D) sind oder
(ii) $Y_1$, $Y_2$, $Y_3$, $Y_4$, $Y_5$ Deuterium (D) sind oder
(iii) einer von R1, R2 mindestens ein Deuterium (D) enthält;

gegebenenfalls mindestens ein Kohlenstoffatom unabhängig voneinander durch $^{13}C$ ersetzt ist;
und die physiologisch unbedenklichen Salze, Solvate, Tautomere und Stereoisomere davon, einschließlich deren Mischungen in allen Verhältnissen.

**2.** Deuteriertes Thiazolidinonderivat nach Anspruch 1, bei dem
$R_1$ und/oder $R_2$ $CD_3$ sind;

und die physiologisch unbedenklichen Salze, Solvate, Tautomere und Stereoisomere davon, einschließlich deren Mischungen in allen Verhältnissen.

**3.** Deuteriertes Thiazolidinonderivat nach beliebigen der Ansprüche 1 oder 2, bei dem

(a) $Y_{15}$, $Y_{16}$, $Y_{17}$, $Y_{18}$ Deuterium (D) sind und die anderen Y Wasserstoff (H) sind und keiner von $R_1$, $R_2$ ein oder mehrere Deuterium (D) enthält; oder
(b) $Y_{15}$, $Y_{16}$, $Y_{17}$, $Y_{18}$ Deuterium (D) sind und die anderen Y Wasserstoff (H) sind und einer von $R_1$, $R_2$ $CD_3$ ist und der andere von $R_1$, $R_2$ nicht ein oder mehrere Deuterium (D) enthält; oder
(c) alle Y Wasserstoff (H) sind und einer von $R_1$, $R_2$ $CD_3$ ist und der andere von $R_1$, $R_2$ nicht ein oder mehrere Deuterium (D) enthält; oder
(d) $Y_1$, $Y_2$, $Y_3$, $Y_4$, $Y_5$ Deuterium (D) sind und die anderen Y Wasserstoff (H) sind und keiner von $R_1$, $R_2$ ein oder mehrere Deuterium (D) enthält; oder
(e) alle Y Wasserstoff (H) sind und beide von $R_1$, $R_2$ $CD_3$ sind; oder
(f) $Y_1$, $Y_2$, $Y_3$, $Y_4$, $Y_5$, $Y_{15}$, $Y_{16}$, $Y_{17}$, $Y_{18}$ Deuterium (D) sind und die anderen Y Wasserstoff (H) sind und keiner von $R_1$, $R_2$ ein oder mehrere Deuterium (D) enthält; oder
(g) $Y_1$, $Y_2$, $Y_3$, $Y_4$, Y5, $Y_{15}$, $Y_{16}$, $Y_{17}$, $Y_{18}$ Deuterium (D) sind und die anderen Y Wasserstoff (H) sind und einer von $R_1$, $R_2$ $CD_3$ ist und der andere von $R_1$, $R_2$ nicht ein oder mehrere Deuterium (D) enthält; oder
(h) $Y_6$, $Y_7$, $Y_8$, $Y_9$, $Y_{15}$, $Y_{16}$, $Y_{17}$, $Y_{18}$ Deuterium (D) sind und die anderen Y Wasserstoff (H) sind und einer von $R_1$, $R_2$ $CD_3$ ist und der andere von $R_1$, $R_2$ nicht ein oder mehrere Deuterium (D) enthält;

und die physiologisch unbedenklichen Salze, Solvate, Tautomere und Stereoisomere davon, einschließlich deren Mischungen in allen Verhältnissen.

**4.** Deuteriertes Thiazolidinonderivat, das aus der Gruppe ausgewählt ist, die besteht aus:

| Verbindung | Struktur |
|---|---|
| 1 | 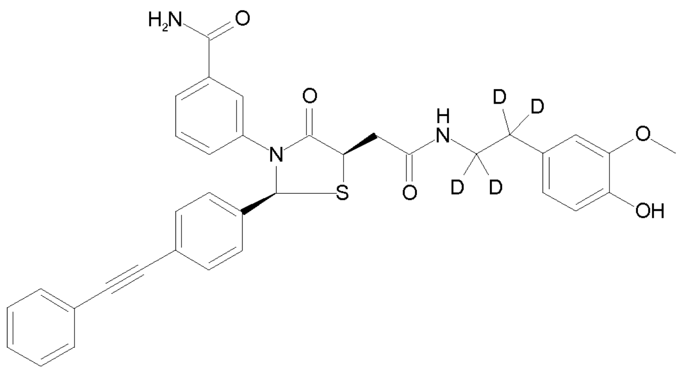 |
| 2 | 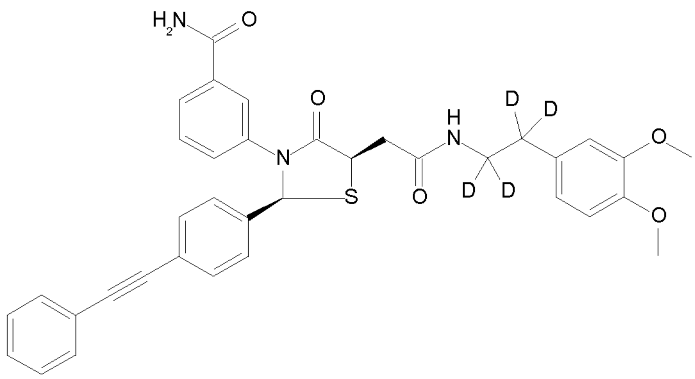 |

(fortgesetzt)

| Verbindung | Struktur |
|---|---|
| 3 | 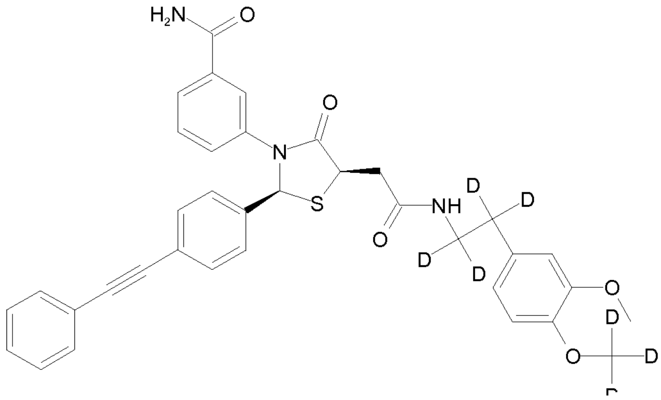 |
| 4 | 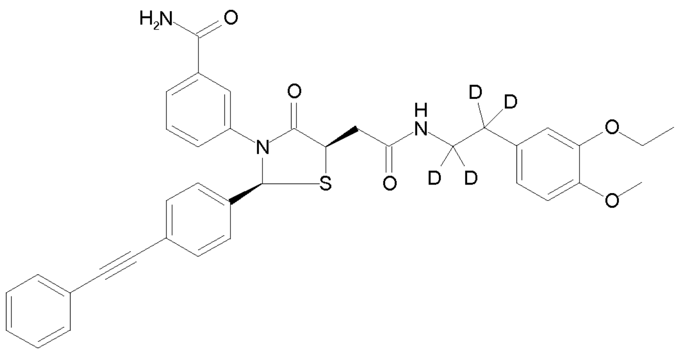 |
| 5 | 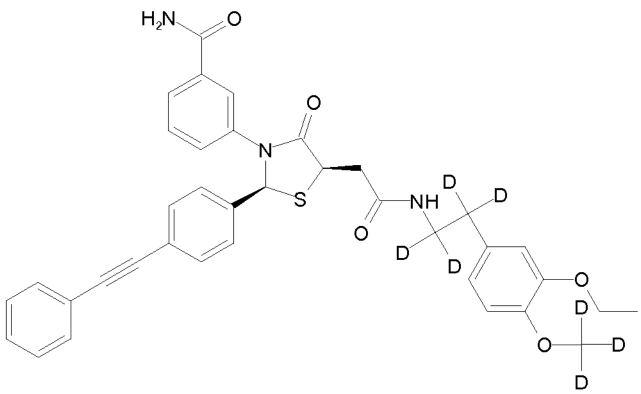 |
| 6 | 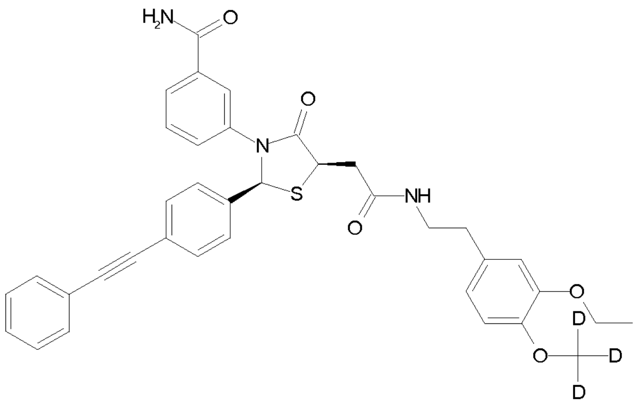 |

(fortgesetzt)

| Verbindung | Struktur |
|---|---|
| **7** | 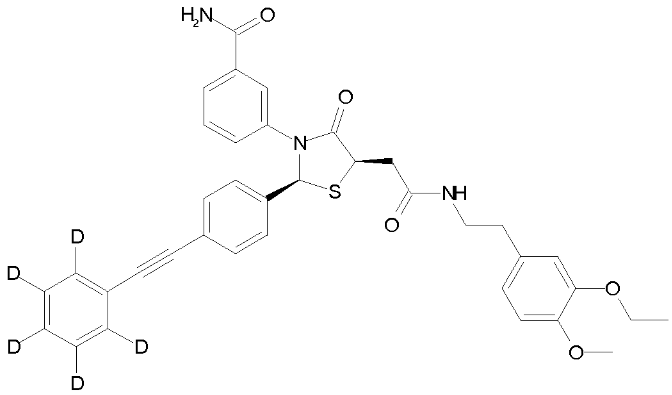 |
| **8** | 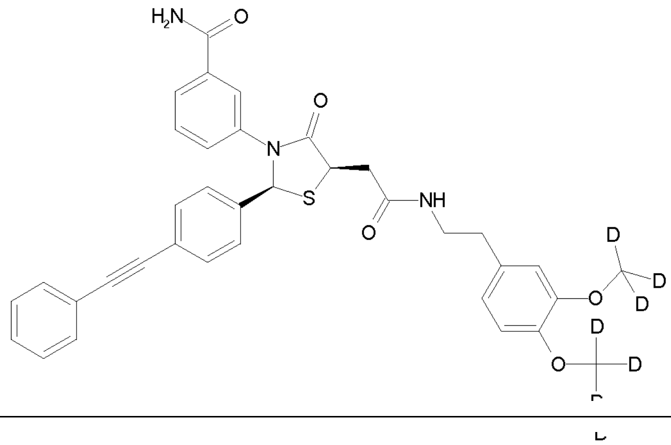 |
| **9** | 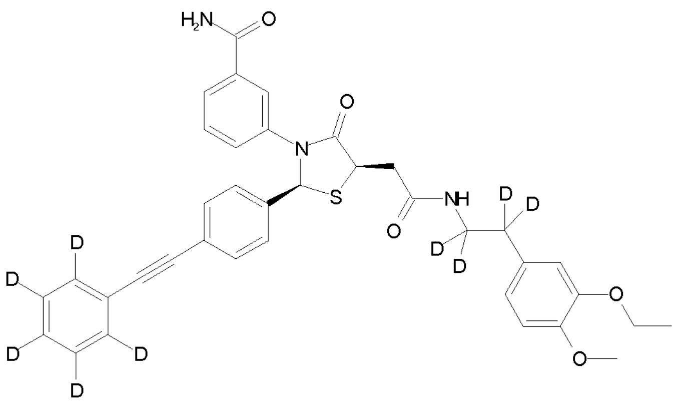 |
| **10** | 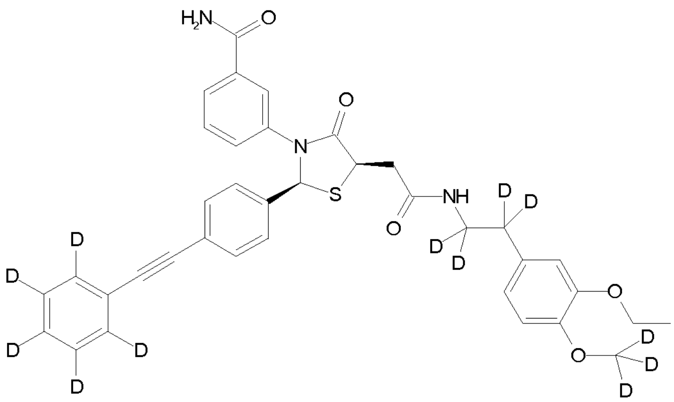 |

(fortgesetzt)

| Verbindung | Struktur |
|---|---|
| 11 | 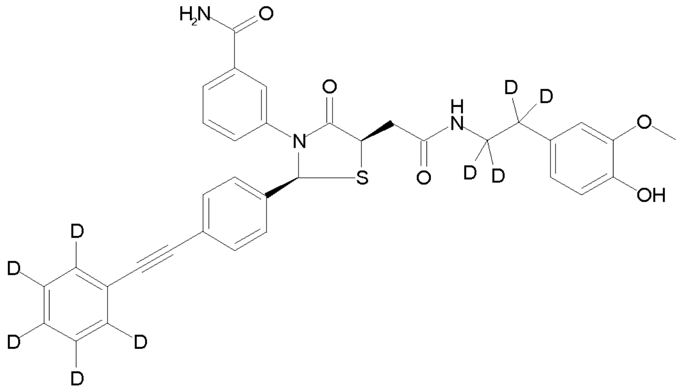 |
| 12 | 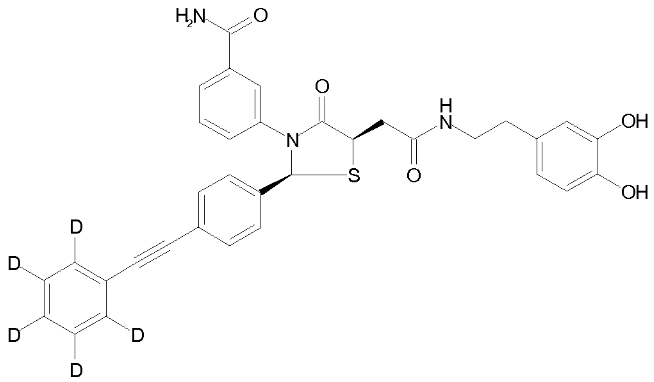 |
| 13 | 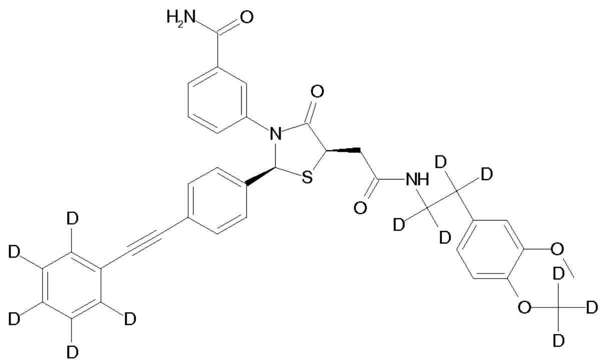 |
| 14 | 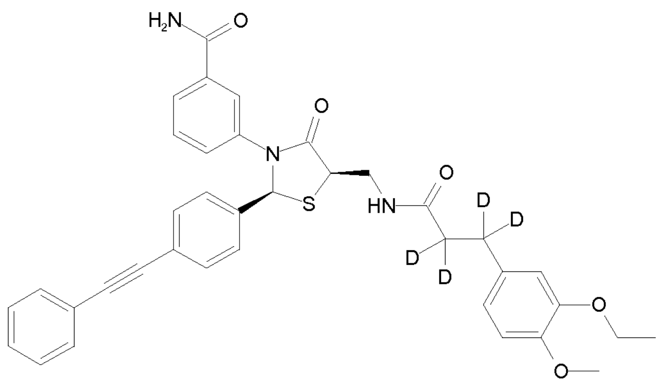 |

(fortgesetzt)

| Verbindung | Struktur |
|---|---|
| **15** | 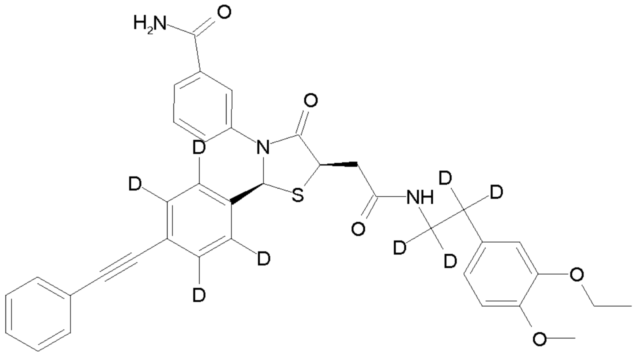 |
| **16** | 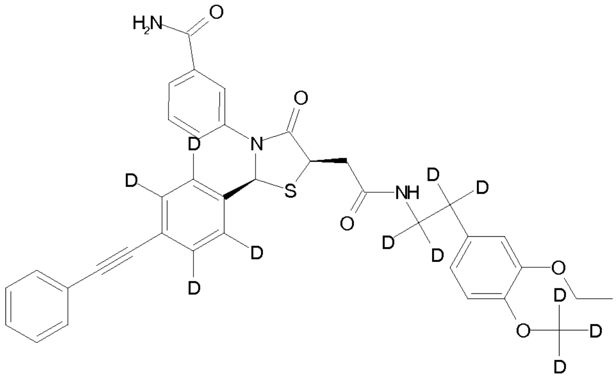 |
| **17** | 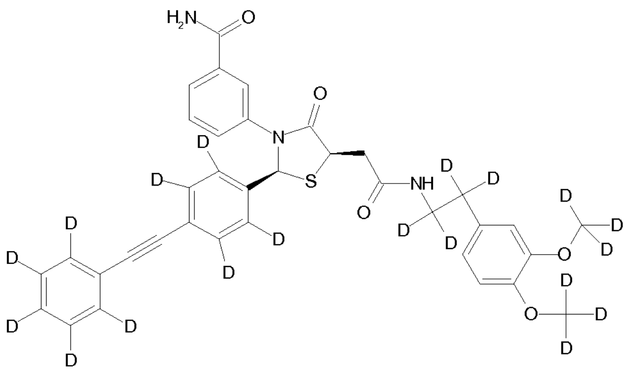 |
| **18** | 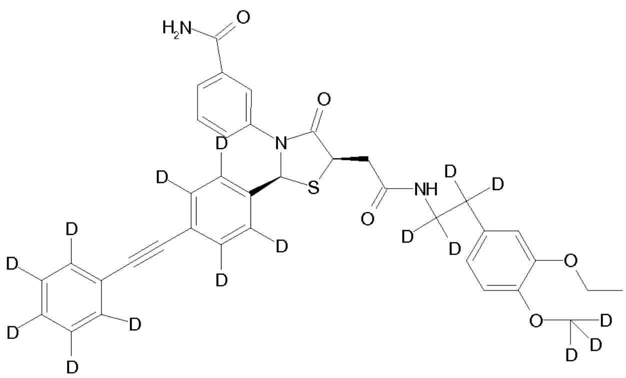 |

(fortgesetzt)

| Verbindung | Struktur |
|---|---|
| **19** | 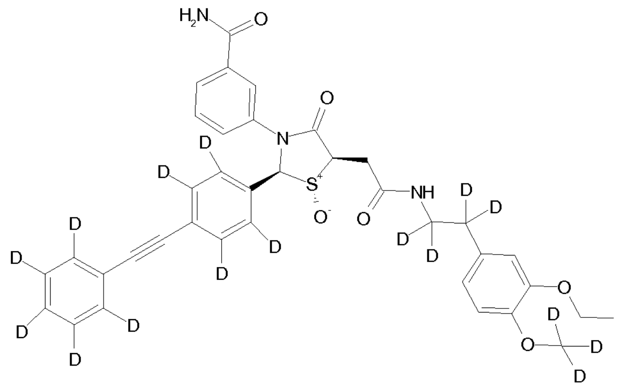 |
| **20** | 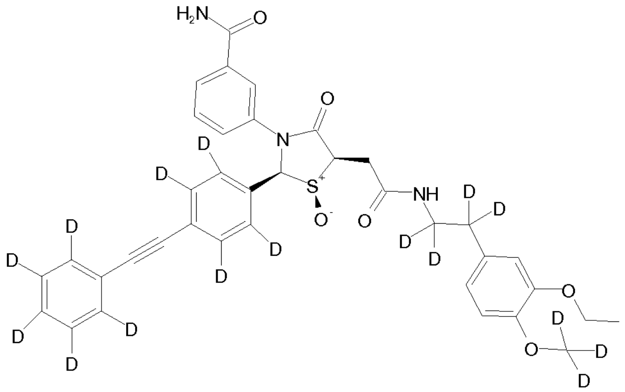 |
| **21** | 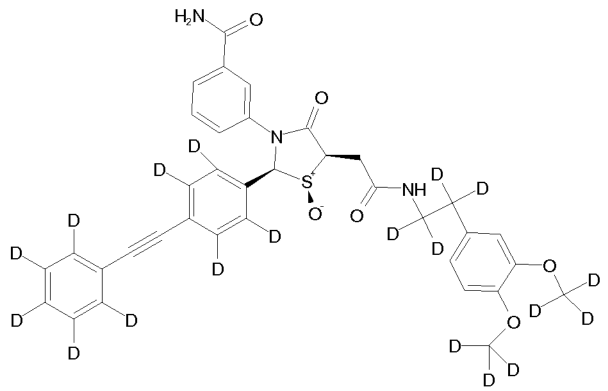 |
| **22** | 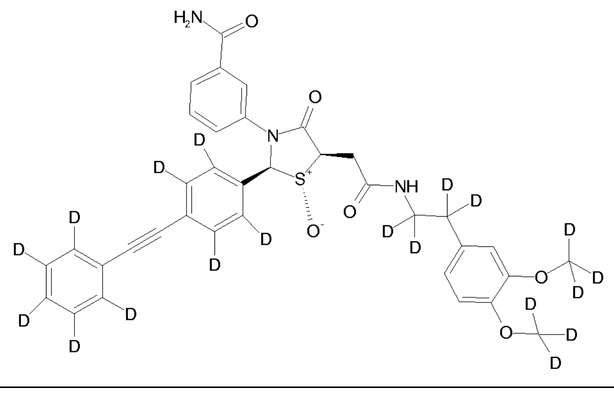 |

und die physiologisch unbedenklichen Salze, Solvate, Tautomere und Stereoisomere davon, einschließlich deren

Mischungen in allen Verhältnissen.

5. Arzneimittel enthaltend mindestens eine Verbindung nach beliebigen der Ansprüche 1 bis 4.

6. Arzneimittel enthaltend mindestens eine Verbindung nach beliebigen der Ansprüche 1 bis 4 zur Verwendung bei der prophylaktischen oder therapeutischen Behandlung und/oder Überwachung von Fruchtbarkeitsstörungen.

7. Pharmazeutische Zusammensetzung enthaltend eine therapeutisch wirksame Menge mindestens einer Verbindung nach beliebigen der Ansprüche 1 bis 4, gegebenenfalls außerdem enthaltend mindestens eine zusätzliche Verbindung ausgewählt aus der Gruppe bestehend aus physiologisch unbedenklichen Streckmitteln, Hilfsstoffen, Adjuvanzien, Verdünnungsmitteln, Trägerstoffen und/oder zusätzlicher pharmazeutisch aktiver Substanz, bei der es sich nicht um die Verbindung nach beliebigen der Ansprüche 1 bis 4 handelt.

8. Kit enthaltend eine therapeutisch wirksame Menge mindestens einer Verbindung nach beliebigen der Ansprüche 1 bis 4 und/oder mindestens eine pharmazeutische Zusammensetzung nach Anspruch 7 und eine therapeutisch wirksame Menge mindestens einer weiteren pharmakologisch aktiven Substanz, bei der es sich nicht um die Verbindung nach beliebigen der Ansprüche 1 bis 4 handelt.

9. Verbindung nach beliebigen der Ansprüche 1 bis 4 oder mindestens eine pharmazeutische Zusammensetzung nach Anspruch 7 zur Verwendung bei der Modulierung eines FSH-Rezeptors auf positiv allosterische Weise, wobei eine Zelle, die zur Expression des FSH-Rezeptors befähigt ist, in Gegenwart von FSH mit der Verbindung in Kontakt gebracht wird.

10. Verbindung nach beliebigen der Ansprüche 1 bis 4 oder mindestens eine pharmazeutische Zusammensetzung nach Anspruch 7 zur Verwendung bei der Behandlung von Fruchtbarkeitsstörungen, wobei eine therapeutisch wirksame Menge der Verbindung einem Säuger verabreicht wird, der einer solchen Behandlung bedarf.

**Revendications**

1. Dérivé de thiazolidinone deutérié répondant à la formule (I)

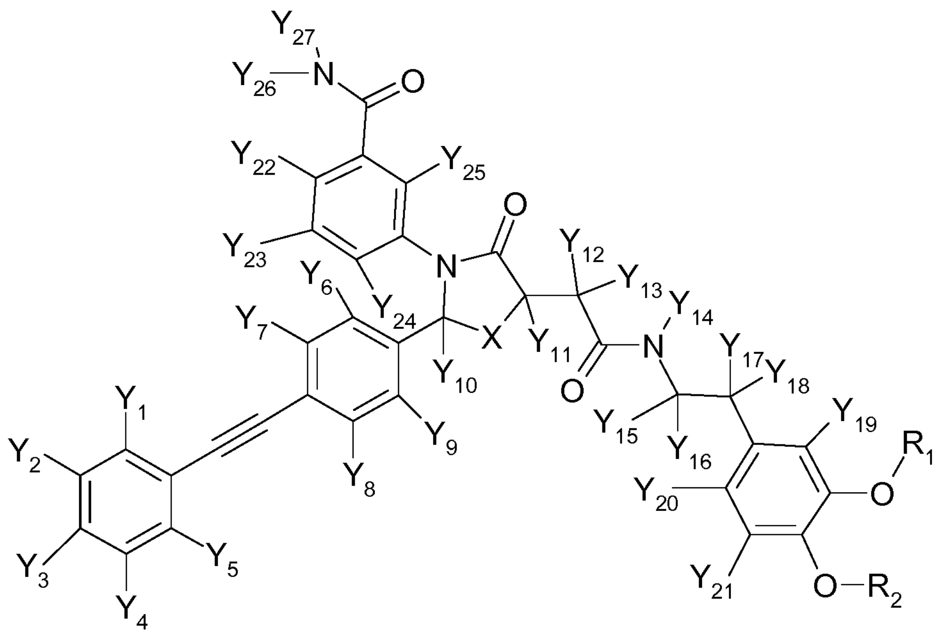

**(I)**

dans lequel :

chaque Y est choisi indépendamment les uns des autres dans le groupe constitué par : « hydrogène (H), deutérium (D) » ;

X est choisi dans le groupe constitué par : « S, sulfoxyde » ;
R1, R2 sont choisis indépendamment l'un de l'autre dans le groupe constitué par : « hydrogène (H), deutérium (D), méthyle, $CH_2D$, $CHD_2$, $CD_3$, éthyle, $CHDCH_3$, $CHDCH_2D$, $CHDCHD_2$, $CHDCD_3$, $CD_2CH_3$, $CD_2CH_2D$, $CD_2CHD_2$, $CD_2CD_3$ » ;
à condition que

(i) $Y_{15}$, $Y_{16}$, $Y_{17}$, $Y_{18}$ soient deutérium (D), ou
(ii) $Y_1$, $Y_2$, $Y_3$, $Y_4$, $Y_5$ soient deutérium (D), ou
(iii) l'un parmi R1, R2 comprenne au moins un deutérium (D) ;

éventuellement, au moins un atome de carbone est, indépendamment les uns des autres, remplacé par $^{13}C$ ; ainsi que les sels, solvates, tautomères et stéréoisomères physiologiquement acceptables de celui-ci, y compris des mélanges de ceux-ci en toutes proportions.

**2.** Dérivé de thiazolidinone deutérié selon la revendication 1, dans lequel
$R_1$ et/ou $R_2$ sont $CD_3$ ;
ainsi que les sels, solvates, tautomères et stéréoisomères physiologiquement acceptables de celui-ci, y compris des mélanges de ceux-ci en toutes proportions.

**3.** Dérivé de thiazolidinone deutérié selon l'une quelconque des revendications 1 ou 2, dans lequel

(a) $Y_{15}$, $Y_{16}$, $Y_{17}$, $Y_{18}$ sont deutérium (D) et les autres Y sont hydrogène (H) et aucun parmi $R_1$, $R_2$ ne comprend un ou plusieurs deutérium (D) ; ou
(b) $Y_{15}$, $Y_{16}$, $Y_{17}$, $Y_{18}$ sont deutérium (D) et les autres Y sont hydrogène (H) et l'un parmi $R_1$, $R_2$ est $CD_3$ et l'autre parmi $R_1$, $R_2$ ne comprend pas un ou plusieurs deutérium (D) ; ou
(c) tous les Y sont hydrogène (H) et l'un parmi $R_1$, $R_2$ est $CD_3$ et l'autre parmi $R_1$, $R_2$ ne comprend pas un ou plusieurs deutérium (D) ; ou
(d) $Y_1$, $Y_2$, $Y_3$, $Y_4$, $Y_5$ sont deutérium (D) et les autres Y sont hydrogène (H) et aucun parmi $R_1$, $R_2$ ne comprend un ou plusieurs deutérium (D) ; ou
(e) tous les Y sont hydrogène (H) et les deux parmi $R_1$, $R_2$ sont $CD_3$ ; ou
(f) $Y_1$, $Y_2$, $Y_3$, $Y_4$, $Y_5$, $Y_{15}$, $Y_{16}$, $Y_{17}$, $Y_{18}$ sont deutérium (D) et les autres Y sont hydrogène (H) et aucun parmi $R_1$, $R_2$ ne comprend un ou plusieurs deutérium (D) ; ou
(g) $Y_1$, $Y_2$, $Y_3$, $Y_4$, $Y_5$, $Y_{15}$, $Y_{16}$, $Y_{17}$, $Y_{18}$ sont deutérium (D) et les autres Y sont hydrogène (H) et l'un parmi $R_1$, $R_2$ est $CD_3$ et l'autre parmi $R_1$, $R_2$ ne comprend pas un ou plusieurs deutérium (D) ; ou
(h) $Y_6$, $Y_7$, $Y_8$, $Y_9$, $Y_{15}$, $Y_{16}$, $Y_{17}$, $Y_{18}$ sont deutérium (D) et les autres Y sont hydrogène (H) et l'un parmi $R_1$, $R_2$ est $CD_3$ et l'autre parmi $R_1$, $R_2$ ne comprend pas un ou plusieurs deutérium (D) ;

ainsi que les sels, solvates, tautomères et stéréoisomères physiologiquement acceptables de celui-ci, y compris des mélanges de ceux-ci en toutes proportions.

**4.** Dérivé de thiazolidinone deutérié, choisi dans le groupe constitué par :

| Composé | Structure |
|---|---|
| 1 | 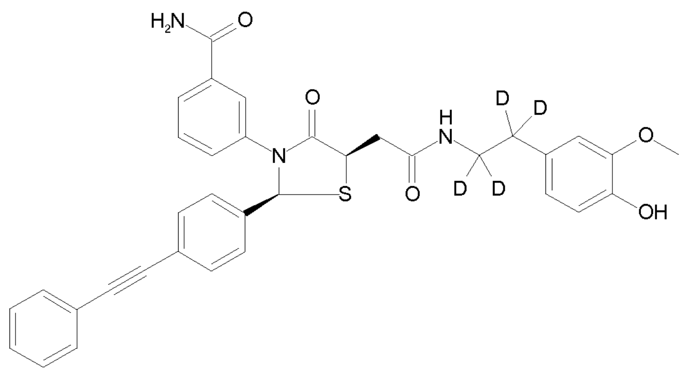 |

(suite)

| Composé | Structure |
|---|---|
| **2** | 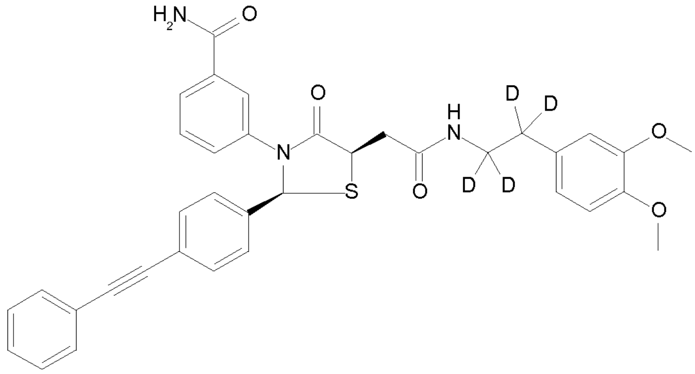 |
| **3** | 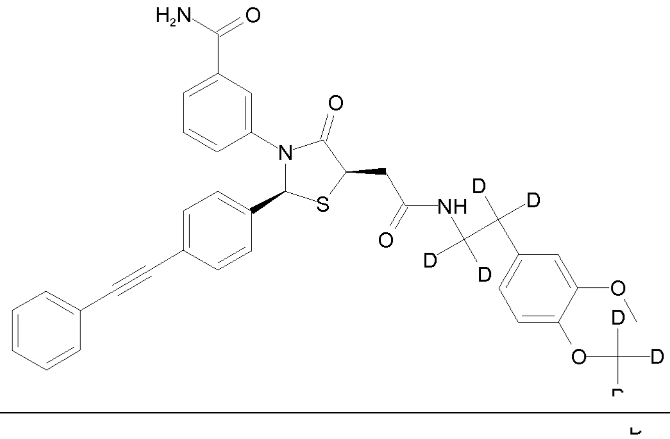 |
| **4** | 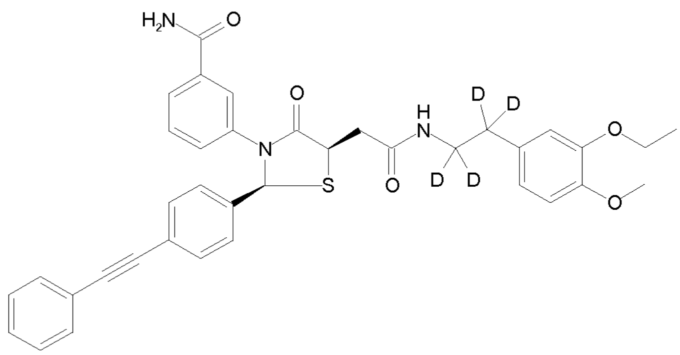 |
| **5** | 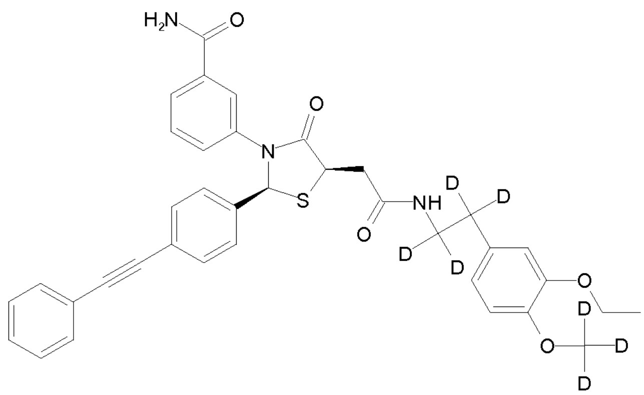 |

(suite)

| Composé | Structure |
|---|---|
| **6** | 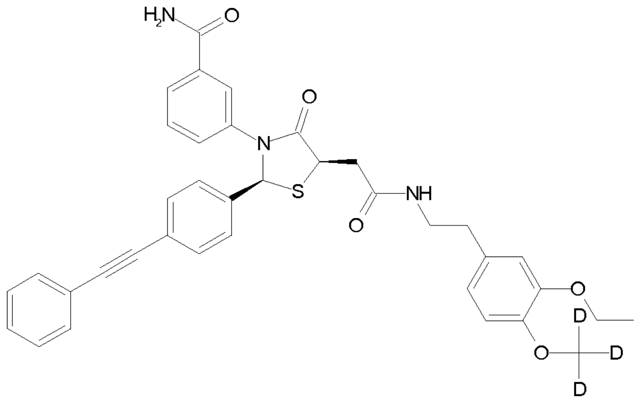 |
| **7** | 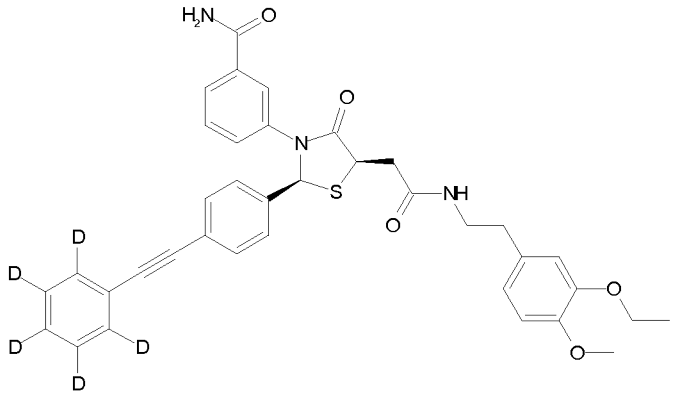 |
| **8** | 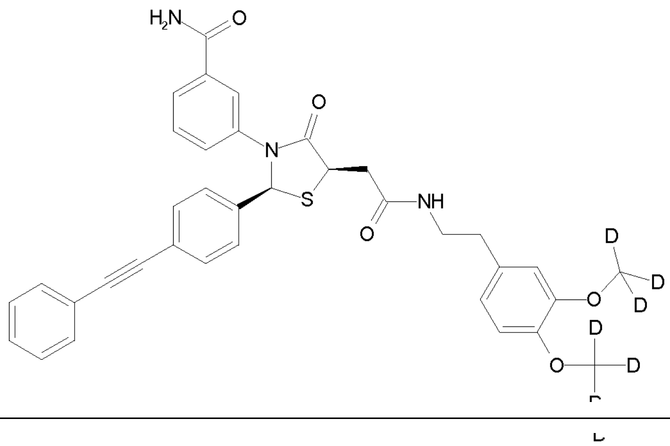 |
| **9** | 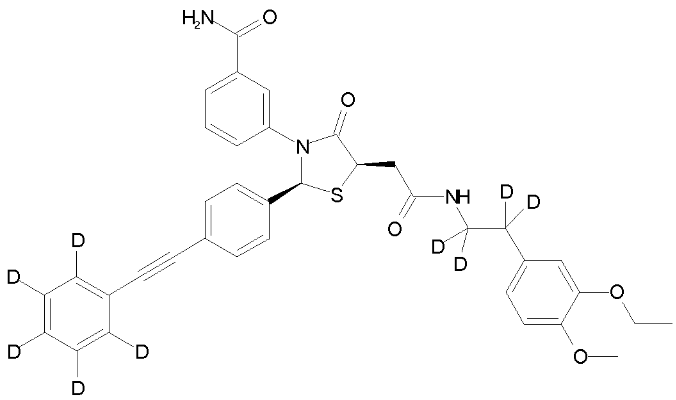 |

(suite)

| Composé | Structure |
|---|---|
| **10** | 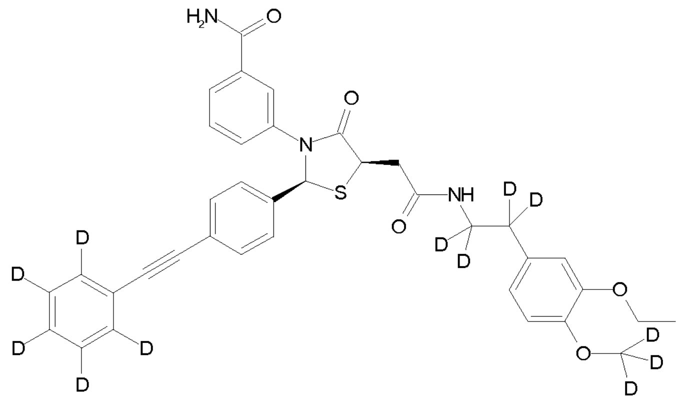 |
| **11** | 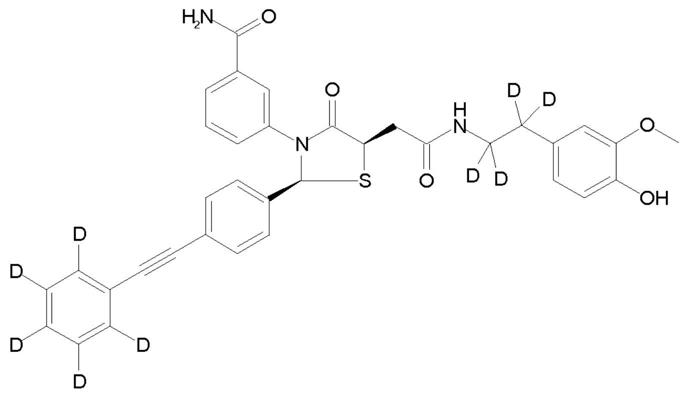 |
| **12** | 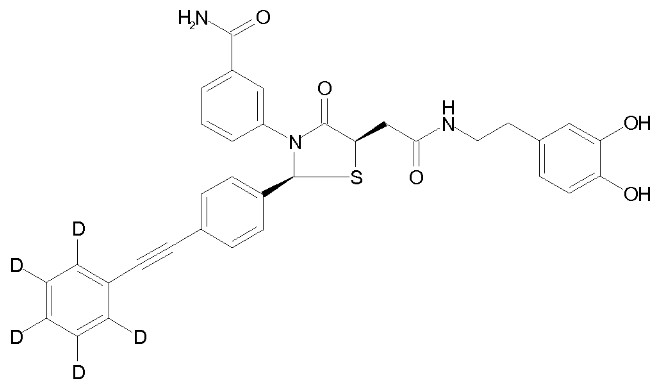 |
| **13** | 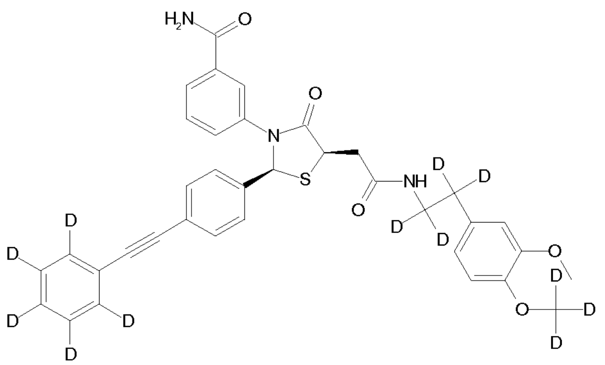 |

(suite)

| Composé | Structure |
|---|---|
| **14** | 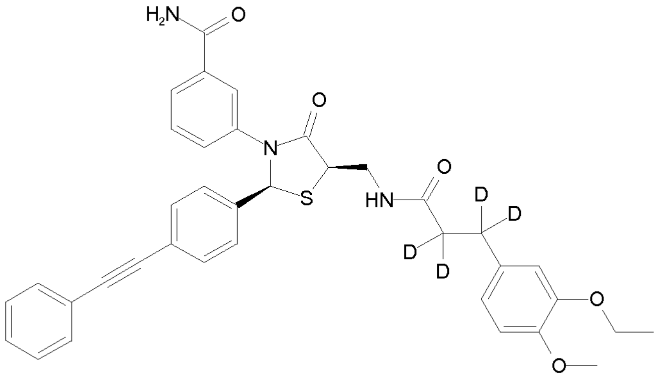 |
| **15** | 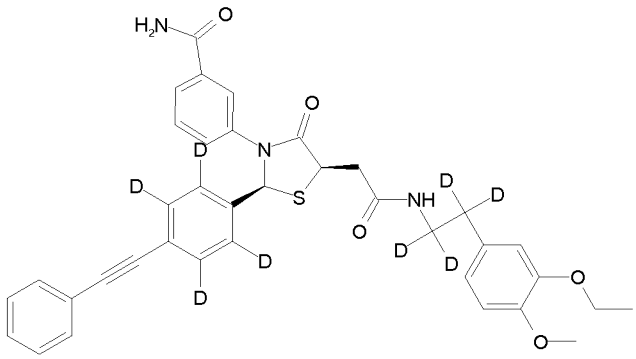 |
| **16** | 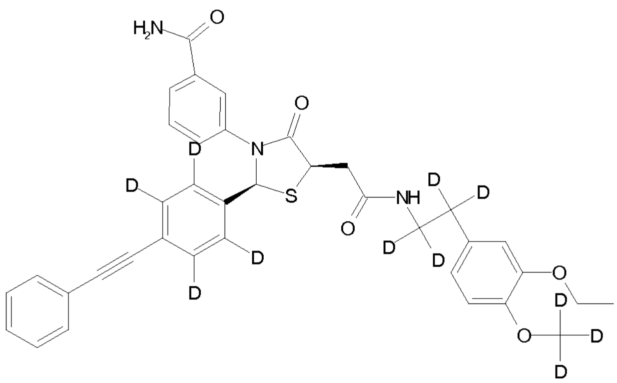 |
| **17** | 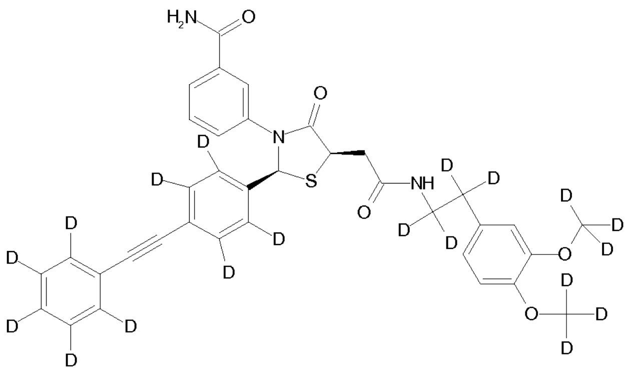 |

(suite)

| Composé | Structure |
|---|---|
| **18** | 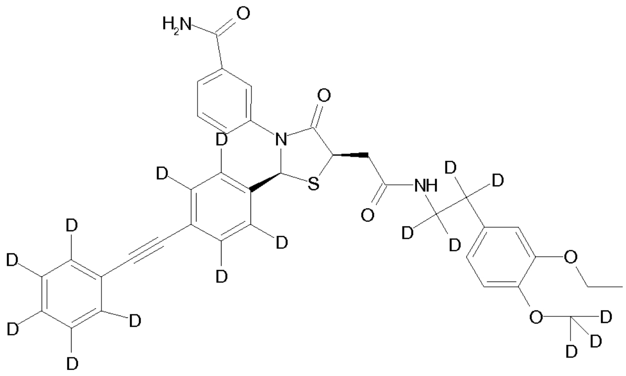 |
| **19** | 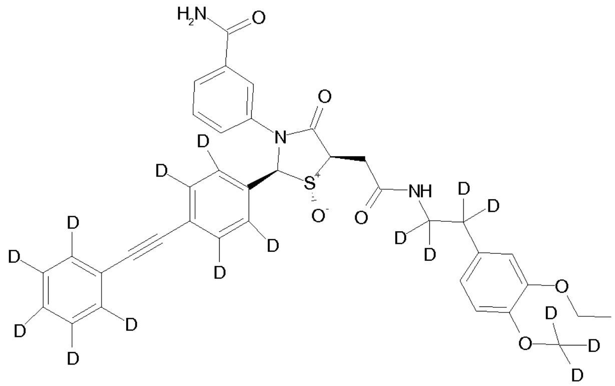 |
| **20** | 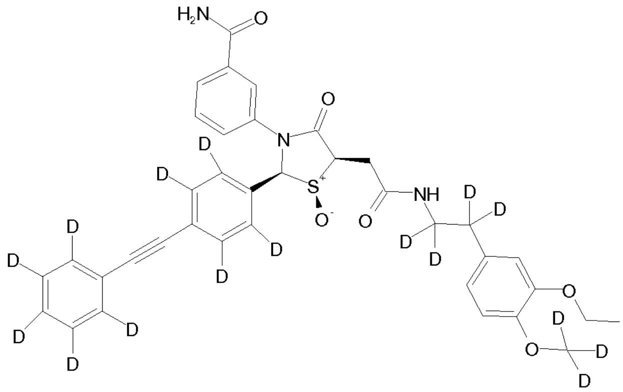 |
| **21** | 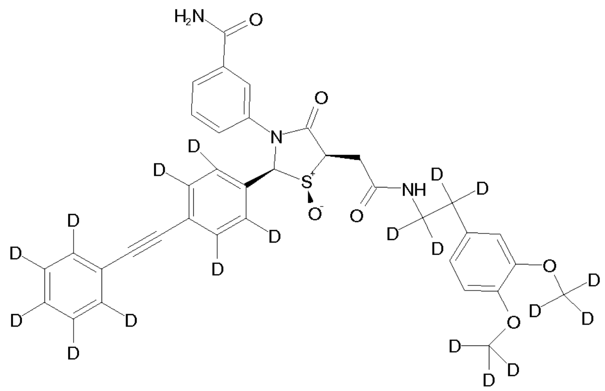 |

(suite)

| Composé | Structure |
|---|---|
| 22 | 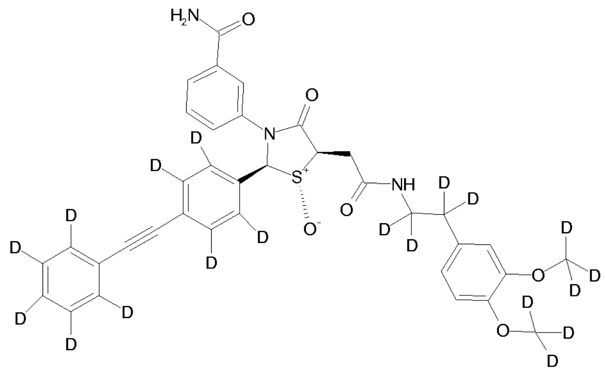 |

ainsi que les sels, solvates, tautomères et stéréoisomères physiologiquement acceptables de celui-ci, y compris des mélanges de ceux-ci en toutes proportions.

**5.** Médicament comprenant au moins un composé selon l'une quelconque des revendications 1 à 4.

**6.** Médicament comprenant au moins un composé selon l'une quelconque des revendications 1 à 4, pour une utilisation dans le traitement prophylactique ou thérapeutique et/ou le suivi de troubles de la fertilité.

**7.** Composition pharmaceutique comprenant une quantité thérapeutiquement efficace d'au moins un composé selon l'une quelconque des revendications 1 à 4, comprenant éventuellement en outre au moins un composé supplémentaire choisi dans le groupe constitué par les excipients, auxiliaires, adjuvants, diluants, véhicules physiologiquement acceptables et/ou une substance pharmaceutiquement active supplémentaire autre que le composé selon l'une quelconque des revendications 1 à 4.

**8.** Kit comprenant une quantité thérapeutiquement efficace d'au moins un composé selon l'une quelconque des revendications 1 à 4 et/ou d'au moins une composition pharmaceutique selon la revendication 7 et une quantité thérapeutiquement efficace d'au moins une substance pharmacologiquement active supplémentaire autre que le composé selon l'une quelconque des revendications 1 à 4.

**9.** Composé selon l'une quelconque des revendications 1 à 4 ou au moins une composition pharmaceutique selon la revendication 7, pour une utilisation dans la modulation d'un récepteur de FSH d'une manière allostérique positive, où une cellule capable d'exprimer le récepteur de FSH est mise en contact en présence de FSH avec ledit composé.

**10.** Composé selon l'une quelconque des revendications 1 à 4 ou au moins une composition pharmaceutique selon la revendication 7, pour une utilisation dans le traitement de troubles de la fertilité, où une quantité thérapeutiquement efficace dudit composé est administrée à un mammifère ayant besoin d'un tel traitement.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 200209706 A
- WO 2010136438 A
- US 6653338 B
- WO 0209705 A
- WO 0209706 A
- WO 2009045476 A
- WO 2002009706 A


### Non-patent literature cited in the description


- **PELLETIER JC et al.** disclose the preparation of highly substituted gamma-lactam follicle stimulating hormone receptor agonists. *Bioorg. Med. Chem.,* 2005, vol. 13, 5986-5995
- **WROBEL J et al.** describe 5-alkylated thiazolidinones as follicle-stimulating hormone receptor agonists. *Bioorg. Med. Chem.,* 2006, vol. 14, 5729-5741
- **FOSTER, A.B.** *Advances in Drug Research,* 1985, vol. 14, 1-40
- **KUSHNER et al.** *Canadian Journal of Physiology and Pharmacology,* 1999, vol. 77 (2), 79-88
- **HOUBEN-WEYL.** Methods of Organic Chemistry. Georg Thieme Verlag
- Organic Reactions. John Wiley & Sons, Inc,